# EUROPEAN PATENT APPLICATION

(11) **EP 3 892 298 A1**
(43) Date of publication of application: **13.10.2021**
(21) Application number: 20169101.1
(22) Date of filing: 09.04.2020
(51) Int. Cl.: A61K 39/215, G16B 10/00, C07K 14/165, A61P 43/00

(54) **EPITOPES HAVING SEQUENCE HOMOLOGY TO CORONAVIRUS SPIKE PROTEIN SUBUNIT AND USES THEREOF**

(71) Applicant: PreviPharma Consulting GmbH, 68167 Mannheim (DE)
(72) Inventor: Kießig, Stephan T., 69168 Wiesloch (DE); Mandago, Maurice, 69250 Schönau (DE); Varga, Andras, 13355 Berlin (DE)
(74) Representative: Jacobi, Markus Alexander

(57) **Abstract**

The present invention relates to polypeptides comprising epitopes having a sequence homology to a sequence section of subunit S1 and/or subunit S2 of coronavirus spike protein from severe acute respiratory syndrome coronavirus 2 (SARS-CoV-2). Moreover, the present invention refers to a vaccine comprising such polypeptide. The invention further relates to an antibody binding to subunit S1 and/or subunit S2 and to methods for isolating and detecting such antibody and to uses of the polypeptides and antibodies.

SEQUENCE LISTING

<110>  PreviPharma Consulting GmbH

<120>  Epitopes having sequence homology to coronavirus spike protein
       subunit and uses thereof

<130>  PREV72920EP

<160>  62

<170>  PatentIn version 3.5

<210>  1
<211>  1250
<212>  PRT
<213>  Severe acute respiratory syndrome coronavirus 1

<400>  1

Met Phe Ile Phe Leu Leu Phe Leu Thr Leu Thr Ser Gly Ser Asp Leu
1               5                   10                  15

Asp Arg Cys Thr Thr Phe Asp Asp Val Gln Ala Pro Asn Tyr Thr Gln
            20                  25                  30

His Thr Ser Ser Met Arg Gly Val Tyr Tyr Pro Asp Glu Ile Phe Arg
        35                  40                  45

Ser Asp Thr Leu Tyr Leu Thr Gln Asp Leu Phe Leu Pro Phe Tyr Ser
    50                  55                  60

Asn Val Thr Gly Phe His Thr Ile Asn His Thr Phe Gly Asn Pro Val
65                  70                  75                  80

Ile Pro Phe Lys Asp Gly Ile Tyr Phe Ala Ala Thr Glu Lys Ser Asn
                85                  90                  95

Val Val Arg Gly Trp Val Phe Gly Ser Thr Met Asn Asn Lys Ser Gln
            100                 105                 110

Ser Val Ile Ile Ile Asn Asn Ser Thr Asn Val Val Ile Arg Ala Cys
        115                 120                 125

Asn Phe Glu Leu Cys Asp Asn Pro Phe Phe Ala Val Ser Lys Pro Met
    130                 135                 140

Gly Thr Gln Thr His Thr Met Ile Phe Asp Asn Ala Phe Asn Cys Thr
145                 150                 155                 160

Phe Glu Tyr Ile Ser Asp Ala Phe Ser Leu Asp Val Ser Glu Lys Ser
                165                 170                 175

Gly Asn Phe Lys His Leu Arg Glu Phe Val Phe Lys Asn Lys Asp Gly
            180                 185                 190

Phe Leu Tyr Val Tyr Lys Gly Tyr Gln Pro Ile Asp Val Val Arg Asp
        195                 200                 205

Leu Pro Ser Gly Phe Asn Thr Leu Lys Pro Ile Phe Lys Leu Pro Leu
    210                 215                 220

Gly Ile Asn Ile Thr Asn Phe Arg Ala Ile Leu Thr Ala Phe Ser Pro
225                 230                 235                 240

Ala Gln Asp Ile Trp Gly Thr Ser Ala Ala Ala Tyr Phe Val Gly Tyr
                245                 250                 255

Leu Lys Pro Thr Thr Phe Met Leu Lys Tyr Asp Glu Asn Gly Thr Ile
            260                 265                 270

Thr Asp Ala Val Asp Cys Ser Gln Asn Pro Leu Ala Glu Leu Lys Cys
        275                 280                 285

Ser Val Lys Ser Phe Glu Ile Asp Lys Gly Ile Tyr Gln Thr Ser Asn
    290                 295                 300

Phe Arg Val Val Pro Ser Gly Asp Val Val Arg Phe Pro Asn Ile Thr
305                 310                 315                 320

Asn Leu Cys Pro Phe Gly Glu Val Phe Asn Ala Thr Lys Phe Pro Ser
                325                 330                 335

Val Tyr Ala Trp Glu Arg Lys Lys Ile Ser Asn Cys Val Ala Asp Tyr
            340                 345                 350

Ser Val Leu Tyr Asn Ser Thr Phe Phe Ser Thr Phe Lys Cys Tyr Gly
        355                 360                 365

Val Ser Ala Thr Lys Leu Asn Asp Leu Cys Phe Ser Asn Val Tyr Ala
    370                 375                 380

Asp Ser Phe Val Val Lys Gly Asp Asp Val Arg Gln Ile Ala Pro Gly
385                 390                 395                 400

Gln Thr Gly Val Ile Ala Asp Tyr Asn Tyr Lys Leu Pro Asp Asp Phe
                405                 410                 415

Met Gly Cys Val Leu Ala Trp Asn Thr Arg Asn Ile Asp Ala Thr Ser
            420                 425                 430

Thr Gly Asn Tyr Asn Tyr Lys Tyr Arg Tyr Leu Arg His Gly Lys Leu
        435                 440                 445

Arg Pro Phe Glu Arg Asp Ile Ser Asn Val Pro Phe Ser Pro Asp Gly
    450                 455                 460

Lys Pro Cys Thr Pro Pro Ala Leu Asn Cys Tyr Trp Pro Leu Asn Asp
465                 470                 475                 480

Tyr Gly Phe Tyr Thr Thr Thr Gly Ile Gly Tyr Gln Pro Tyr Arg Val
                485                 490                 495

Val Val Leu Ser Phe Glu Leu Leu Asn Ala Pro Ala Thr Val Cys Gly
            500                 505                 510

Pro Lys Leu Ser Thr Asp Leu Ile Lys Asn Gln Cys Val Asn Phe Asn
        515                 520                 525

Phe Asn Gly Leu Thr Gly Thr Gly Val Leu Thr Pro Ser Ser Lys Arg
    530                 535                 540

Phe Gln Pro Phe Gln Gln Phe Gly Arg Asp Val Ser Asp Phe Thr Asp
545                 550                 555                 560

Ser Val Arg Asp Pro Lys Thr Ser Glu Ile Leu Asp Ile Ser Pro Cys
                565                 570                 575

Ser Phe Gly Gly Val Ser Val Ile Thr Pro Gly Thr Asn Ala Ser Ser
            580                 585                 590

Glu Val Ala Val Leu Tyr Gln Asp Val Asn Cys Thr Asp Val Ser Thr
        595                 600                 605

Ala Ile His Ala Asp Gln Leu Thr Pro Ala Trp Arg Ile Tyr Ser Thr
    610                 615                 620

Gly Asn Asn Val Phe Gln Thr Gln Ala Gly Cys Leu Ile Gly Ala Glu
625                 630                 635                 640

His Val Asp Thr Ser Tyr Glu Cys Asp Ile Pro Ile Gly Ala Gly Ile
                645                 650                 655

Cys Ala Ser Tyr His Thr Val Ser Leu Leu Arg Ser Thr Ser Gln Lys
            660                 665                 670

Ser Ile Val Ala Tyr Thr Met Ser Leu Gly Ala Asp Ser Ser Ile Ala

        675                 680                 685

Tyr Ser Asn Asn Thr Ile Ala Ile Pro Thr Asn Phe Ser Ile Ser Ile
    690                 695                 700

Thr Thr Glu Val Met Pro Val Ser Met Ala Lys Thr Ser Val Asp Cys
705                 710                 715                 720

Asn Met Tyr Ile Cys Gly Asp Ser Thr Glu Cys Ala Asn Leu Leu Leu
                725                 730                 735

Gln Tyr Gly Ser Phe Cys Thr Gln Leu Asn Arg Ala Leu Ser Gly Ile
            740                 745                 750

Ala Ala Glu Gln Asp Arg Asn Thr Arg Glu Val Phe Ala Gln Val Lys
        755                 760                 765

Gln Met Tyr Lys Thr Pro Thr Leu Lys Tyr Phe Gly Gly Phe Asn Phe
    770                 775                 780

Ser Gln Ile Leu Pro Asp Pro Leu Lys Pro Thr Lys Arg Ser Phe Ile
785                 790                 795                 800

Glu Asp Leu Leu Phe Asn Lys Val Thr Leu Ala Asp Ala Gly Phe Met
                805                 810                 815

Lys Gln Tyr Gly Glu Cys Leu Gly Asp Ile Asn Ala Arg Asp Leu Ile
            820                 825                 830

Cys Ala Gln Lys Phe Asn Gly Leu Thr Val Leu Pro Pro Leu Leu Thr
        835                 840                 845

Asp Asp Met Ile Ala Ala Tyr Thr Ala Ala Leu Val Ser Gly Thr Ala
    850                 855                 860

Thr Ala Gly Trp Thr Phe Gly Ala Gly Ala Ala Leu Gln Ile Pro Phe
865                 870                 875                 880

Ala Met Gln Met Ala Tyr Arg Phe Asn Gly Ile Gly Val Thr Gln Asn
                885                 890                 895

Val Leu Tyr Glu Asn Gln Lys Gln Ile Ala Asn Gln Phe Asn Lys Ala
            900                 905                 910

Ile Ser Gln Ile Gln Glu Ser Leu Thr Thr Thr Ser Thr Ala Leu Gly
        915                 920                 925

Lys Leu Gln Asp Val Val Asn Gln Asn Ala Gln Ala Leu Asn Thr Leu
    930                 935                 940

Val Lys Gln Leu Ser Ser Asn Phe Gly Ala Ile Ser Ser Val Leu Asn
945                 950                 955                 960

Asp Ile Leu Ser Arg Leu Asp Lys Val Glu Ala Glu Val Gln Ile Asp
                965                 970                 975

Arg Leu Ile Thr Gly Arg Leu Gln Ser Leu Gln Thr Tyr Val Thr Gln
            980                 985                 990

Gln Leu Ile Arg Ala Ala Glu Ile Arg Ala Ser Ala Asn Leu Ala Ala
        995                 1000                1005

Thr Lys Met Ser Glu Cys Val Leu Gly Gln Ser Lys Arg Val Asp
    1010                1015                1020

Phe Cys Gly Lys Gly Tyr His Leu Met Ser Phe Pro Gln Ala Ala
    1025                1030                1035

Pro His Gly Val Val Phe Leu His Val Thr Tyr Val Pro Ser Gln
    1040                1045                1050

Glu Arg Asn Phe Thr Thr Ala Pro Ala Ile Cys His Glu Gly Lys
    1055                1060                1065

Ala Tyr Phe Pro Arg Glu Gly Val Phe Val Phe Asn Gly Thr Ser
    1070                1075                1080

Trp Phe Ile Thr Gln Arg Asn Phe Phe Ser Pro Gln Ile Ile Thr
    1085                1090                1095

Thr Asp Asn Thr Phe Val Ser Gly Asn Cys Asp Val Val Ile Gly
    1100                1105                1110

Ile Ile Asn Asn Thr Val Tyr Asp Pro Leu Gln Pro Glu Leu Asp
    1115                1120                1125

Ser Phe Lys Glu Glu Leu Asp Lys Tyr Phe Lys Asn His Thr Ser
    1130                1135                1140

Pro Asp Val Asp Leu Gly Asp Ile Ser Gly Ile Asn Ala Ser Val
    1145                1150                1155

Val Asn Ile Gln Lys Glu Ile Asp Arg Leu Asn Glu Val Ala Lys
    1160                1165                1170

Asn Leu Asn Glu Ser Leu Ile Asp Leu Gln Glu Leu Gly Lys Tyr
    1175                1180                1185

Glu Gln Tyr Ile Lys Trp Pro Trp Tyr Val Trp Leu Gly Phe Ile
    1190                1195                1200

Ala Gly Leu Ile Ala Ile Val Met Val Thr Ile Leu Leu Cys Cys
    1205                1210                1215

Met Thr Ser Cys Cys Ser Cys Leu Lys Gly Ala Cys Ser Cys Gly
    1220                1225                1230

Ser Cys Cys Lys Phe Asp Glu Asp Asp Ser Glu Pro Val Leu Lys
    1235                1240                1245

Gly Val
    1250

<210> 2
<211> 1273
<212> PRT
<213> Severe acute respiratory syndrome coronavirus 2

<400> 2

<210>  3
<211>  1350
<212>  PRT
<213>  Middle East respiratory syndrome coronavirus

<400>  3

Met Ile His Ser Val Phe Leu Leu Met Phe Leu Leu Thr Pro Thr Glu
1               5                   10                  15

Ser Tyr Val Asp Val Gly Pro Asp Ser Ala Lys Ser Ala Cys Ile Glu
            20                  25                  30

Val Asp Ile Gln Gln Thr Phe Phe Asp Lys Thr Trp Pro Arg Pro Ile
        35                  40                  45

Asp Val Ser Lys Ala Asp Gly Ile Ile Tyr Pro Gln Gly Arg Thr Tyr
    50                  55                  60

Ser Asn Ile Thr Ile Thr Tyr Gln Gly Leu Phe Pro Tyr Gln Gly Asp
65                  70                  75                  80

His Gly Asp Met Tyr Val Tyr Ser Ala Gly His Ala Thr Gly Thr Thr
                85                  90                  95

Pro Gln Lys Leu Phe Val Ala Asn Tyr Ser Gln Asp Val Lys Gln Phe
            100                 105                 110

Ala Asn Gly Phe Val Val Arg Ile Gly Ala Ala Ala Asn Ser Thr Gly
        115                 120                 125

Thr Val Ile Ile Ser Pro Ser Thr Ser Ala Thr Ile Arg Lys Ile Tyr
    130                 135                 140

Pro Ala Phe Met Leu Gly Ser Ser Val Gly Asn Phe Ser Asp Gly Lys
145                 150                 155                 160

Met Gly Arg Phe Phe Asn Tyr Thr Leu Val Leu Leu Pro Asp Gly Cys
                165                 170                 175

Gly Thr Leu Leu Arg Ala Phe Tyr Cys Ile Leu Glu Pro Arg Ser Gly
            180                 185                 190

Asn Tyr Cys Pro Ala Gly Asn Ser Tyr Thr Ser Phe Ala Thr Tyr His
        195                 200                 205

Thr Pro Ala Thr Asp Cys Ser Asp Gly Asn Tyr Asn Arg Asn Ala Ser
    210                 215                 220

Leu Asn Ser Phe Lys Glu Tyr Phe Asn Leu Arg Asn Cys Thr Phe Met
225                 230                 235                 240

Tyr Thr Tyr Asn Ile Thr Glu Asp Glu Ile Leu Glu Trp Phe Gly Ile
                245                 250                 255

Thr Gln Thr Ala Gln Gly Val His Leu Phe Ser Ser Arg Tyr Val Asp
            260                 265                 270

Leu Tyr Gly Gly Asn Met Phe Gln Phe Ala Thr Leu Pro Val Tyr Asp
        275                 280                 285

Thr Ile Lys Tyr Tyr Ser Ile Ile Pro His Ser Ile Arg Ser Ile Gln
    290                 295                 300

Ser Asp Arg Lys Ala Trp Ala Ala Phe Tyr Val Tyr Lys Leu Gln Pro
305                 310                 315                 320

Leu Thr Phe Leu Leu Asp Phe Ser Val Asp Gly Tyr Ile Arg Arg Ala
                325                 330                 335

Ile Asp Cys Gly Phe Asn Asp Leu Ser Gln Leu His Cys Ser Tyr Glu
            340                 345                 350

Ser Phe Asp Val Glu Ser Gly Val Tyr Ser Val Ser Ser Phe Glu Ala
        355                 360                 365

Lys Pro Ser Gly Ser Val Val Glu Gln Ala Glu Gly Val Glu Cys Asp
    370                 375                 380

Phe Ser Pro Leu Leu Ser Gly Thr Pro Pro Gln Val Tyr Asn Phe Lys
385                 390                 395                 400

Arg Leu Val Phe Thr Asn Cys Asn Tyr Asn Leu Thr Lys Leu Leu Ser
                405                 410                 415

Leu Phe Ser Val Asn Asp Phe Thr Cys Ser Gln Ile Ser Pro Ala Ala
            420                 425                 430

Ile Ala Ser Asn Cys Tyr Ser Ser Leu Ile Leu Asp Tyr Phe Ser Tyr
        435                 440                 445

Pro Leu Ser Met Lys Ser Asp Leu Ser Val Ser Ser Ala Gly Pro Ile
    450                 455                 460

Ser Gln Phe Asn Tyr Lys Gln Ser Phe Ser Asn Pro Thr Cys Leu Ile
465                 470                 475                 480

Leu Ala Thr Val Pro His Asn Leu Thr Thr Ile Thr Lys Pro Phe Lys
                485                 490                 495

Tyr Ser Tyr Ile Asn Lys Cys Ser Arg Leu Leu Ser Asp Asp Arg Thr
            500                 505                 510

Glu Val Pro Gln Leu Val Asn Ala Asn Gln Tyr Ser Pro Cys Val Ser
        515                 520                 525

Ile Val Pro Ser Thr Val Trp Glu Asp Gly Asp Tyr Tyr Arg Lys Gln
    530                 535                 540

Leu Ser Pro Leu Glu Gly Gly Gly Trp Leu Val Ala Ser Gly Ser Thr
545                 550                 555                 560

Val Ala Met Thr Glu Gln Leu Gln Met Gly Phe Gly Ile Thr Val Gln

                565                 570                 575

Tyr Gly Thr Asp Thr Asn Ser Val Cys Pro Lys Phe Glu Phe Ala Asn
            580                 585                 590

Asp Thr Lys Ile Ala Ser Gln Leu Gly Asn Cys Val Glu Tyr Ser Leu
        595                 600                 605

Tyr Gly Val Ser Gly Arg Gly Val Phe Gln Asn Cys Thr Ala Val Gly
    610                 615                 620

Val Arg Gln Gln Arg Phe Val Tyr Asp Ala Tyr Gln Asn Leu Val Gly
625                 630                 635                 640

Tyr Tyr Ser Asp Asp Gly Asn Tyr Tyr Cys Leu Arg Ala Cys Val Ser
                645                 650                 655

Val Pro Val Ser Val Ile Tyr Asp Lys Glu Thr Lys Thr His Ala Thr
            660                 665                 670

Leu Phe Gly Ser Val Ala Cys Glu His Ile Ser Ser Thr Met Ser Gln
        675                 680                 685

Tyr Ser Arg Ser Thr Arg Ser Met Leu Lys Arg Arg Asp Ser Thr Tyr
    690                 695                 700

Gly Pro Leu Gln Thr Pro Val Gly Cys Val Leu Gly Leu Val Asn Ser
705                 710                 715                 720

Ser Leu Phe Val Glu Asp Cys Lys Leu Pro Leu Gly Gln Ser Leu Cys
                725                 730                 735

Ala Leu Pro Asp Thr Pro Ser Thr Leu Thr Pro Arg Ser Val Arg Ser
            740                 745                 750

Val Pro Gly Glu Met Arg Leu Ala Ser Ile Ala Phe Asn His Pro Ile
        755                 760                 765

Gln Val Asp Gln Leu Asn Ser Ser Tyr Phe Lys Leu Ser Ile Pro Thr
    770                 775                 780

Asn Phe Ser Phe Gly Val Thr Gln Glu Tyr Ile Gln Thr Thr Ile Gln
785                 790                 795                 800

Lys Val Thr Val Asp Cys Lys Gln Tyr Val Cys Asn Gly Phe Gln Lys
                805                 810                 815

Cys Glu Gln Leu Leu Arg Glu Tyr Gly Gln Phe Cys Ser Lys Ile Asn
                820                 825                 830

Gln Ala Leu His Gly Ala Asn Leu Arg Gln Asp Asp Ser Val Arg Asn
            835                 840                 845

Leu Phe Ala Ser Val Lys Ser Tyr Gln Ser Ser Pro Ile Ile Pro Gly
    850                 855                 860

Phe Gly Gly Asp Phe Asn Leu Thr Leu Leu Glu Pro Val Ser Ile Ser
865                 870                 875                 880

Thr Gly Ser Arg Ser Ala Arg Ser Ala Ile Glu Asp Leu Leu Phe Asp
                885                 890                 895

Lys Val Thr Ile Ala Asp Pro Gly Tyr Met Gln Gly Tyr Asp Asp Cys
            900                 905                 910

Met Gln Gln Gly Pro Ala Ser Ala Arg Asp Leu Ile Cys Ala Gln Tyr
        915                 920                 925

Val Ala Gly Tyr Lys Val Leu Pro Pro Leu Met Asp Val Asn Met Glu
    930                 935                 940

Ala Ala Tyr Thr Ser Ser Leu Leu Gly Ser Ile Ala Gly Val Gly Trp
945                 950                 955                 960

Thr Ala Gly Leu Ser Ser Phe Ala Ala Ile Pro Phe Ala Gln Ser Ile
                965                 970                 975

Phe Tyr Arg Leu Asn Gly Val Gly Ile Thr Gln Gln Val Leu Ser Glu
            980                 985                 990

Asn Gln Lys Leu Ile Ala Asn Lys  Phe Asn Gln Ala Leu  Gly Ala Met
        995                 1000                1005

Gln Thr  Gly Phe Thr Thr Thr  Asn Glu Ala Phe Gln  Lys Val Gln
    1010                1015                1020

Asp Ala  Val Asn Asn Asn Ala  Gln Ala Leu Ser Lys  Leu Ala Ser
    1025                1030                1035

Glu Leu  Ser Asn Thr Phe Gly  Ala Ile Ser Ala Ser  Ile Gly Asp
    1040                1045                1050

Ile Ile  Gln Arg Leu Asp Val  Leu Glu Gln Asp Ala  Gln Ile Asp
    1055                1060                1065

Arg Leu Ile Asn Gly Arg Leu Thr Thr Leu Asn Ala Phe Val Ala
    1070                1075                1080

Gln Gln Leu Val Arg Ser Glu Ser Ala Ala Leu Ser Ala Gln Leu
    1085                1090                1095

Ala Lys Asp Lys Val Asn Glu Cys Val Lys Ala Gln Ser Lys Arg
    1100                1105                1110

Ser Gly Phe Cys Gly Gln Gly Thr His Ile Val Ser Phe Val Val
    1115                1120                1125

Asn Ala Pro Asn Gly Leu Tyr Phe Met His Val Gly Tyr Tyr Pro
    1130                1135                1140

Ser Asn His Ile Glu Val Val Ser Ala Tyr Gly Leu Cys Asp Leu
    1145                1150                1155

Ala Asn Pro Thr Asn Cys Ile Ala Pro Val Asn Gly Tyr Phe Ile
    1160                1165                1170

Lys Thr Asn Asn Thr Arg Ile Val Asp Glu Trp Ser Tyr Thr Gly
    1175                1180                1185

Ser Ser Phe Tyr Ala Pro Glu Pro Ile Thr Ser Phe Asn Thr Lys
    1190                1195                1200

Tyr Val Ala Pro Gln Val Thr Tyr Gln Asn Ile Ser Thr Asn Leu
    1205                1210                1215

Pro Pro Pro Leu Leu Gly Asn Ser Thr Gly Ile Asp Phe Gln Asp
    1220                1225                1230

Glu Leu Asp Glu Phe Phe Lys Asn Val Ser Thr Ser Ile Pro Asn
    1235                1240                1245

Phe Gly Ser Leu Thr Gln Ile Asn Thr Thr Leu Leu Asp Leu Thr
    1250                1255                1260

Tyr Glu Met Leu Ser Leu Gln Gln Val Val Lys Ala Leu Asn Glu
    1265                1270                1275

Ser Tyr Ile Asp Leu Lys Glu Leu Gly Asn Tyr Thr Tyr Tyr Asn
    1280                1285                1290

Lys Trp Pro Trp Tyr Ile Trp Leu Gly Phe Ile Ala Gly Leu Val
    1295                1300                1305

Ala Leu Ala Leu Cys Val Phe Phe Ile Leu Cys Cys Thr Gly Cys
    1310                1315                1320

Gly Thr Asn Cys Met Gly Lys Leu Lys Cys Asn Arg Cys Cys Asp
    1325                1330                1335

Arg Tyr Glu Glu Tyr Asp Leu Glu Pro His Lys Val
    1340                1345                1350

<210> 4
<211> 1350
<212> PRT
<213> Human coronavirus NL63

<400> 4

Met Lys Leu Phe Leu Ile Leu Leu Val Leu Pro Leu Ala Ser Cys Phe
1               5                   10                  15

Phe Thr Cys Asn Ser Asn Ala Asn Leu Ser Met Leu Gln Leu Gly Val
            20                  25                  30

Pro Asp Asn Ser Ser Thr Ile Val Thr Gly Leu Leu Pro Thr His Trp
        35                  40                  45

Phe Cys Ala Asn Gln Ser Thr Ser Val Tyr Ser Ala Asn Gly Phe Phe
    50                  55                  60

Tyr Ile Asp Val Gly Asn His Arg Ser Ala Phe Ala Leu His Thr Gly
65                  70                  75                  80

Tyr Tyr Asp Val Asn Gln Tyr Tyr Ile Tyr Val Thr Asn Glu Ile Gly
                85                  90                  95

Leu Asn Ala Ser Val Thr Leu Lys Ile Cys Lys Phe Gly Ile Asn Thr
            100                 105                 110

Thr Phe Asp Phe Leu Ser Asn Ser Ser Ser Ser Phe Asp Cys Ile Val
        115                 120                 125

Asn Leu Leu Phe Thr Glu Gln Leu Gly Ala Pro Leu Gly Ile Thr Ile
    130                 135                 140

Ser Gly Glu Thr Val Arg Leu His Leu Tyr Asn Val Thr Arg Thr Phe
145                 150                 155                 160

Tyr Val Pro Ala Ala Tyr Lys Leu Thr Lys Leu Ser Val Lys Cys Tyr
                165                 170                 175

Phe Asn Tyr Ser Cys Val Phe Ser Val Val Asn Ala Thr Val Thr Val
        180                 185                 190

Asn Val Thr Thr His Asn Gly Arg Val Val Asn Tyr Thr Val Cys Asp
        195                 200                 205

Asp Cys Asn Gly Tyr Thr Asp Asn Ile Phe Ser Val Gln Gln Asp Gly
    210                 215                 220

Arg Ile Pro Asn Gly Phe Pro Phe Asn Asn Trp Phe Leu Leu Thr Asn
225                 230                 235                 240

Gly Ser Thr Leu Val Asp Gly Val Ser Arg Leu Tyr Gln Pro Leu Arg
                245                 250                 255

Leu Thr Cys Leu Trp Pro Val Pro Gly Leu Lys Ser Ser Thr Gly Phe
            260                 265                 270

Val Tyr Phe Asn Ala Thr Gly Ser Asp Val Asn Cys Asn Gly Tyr Gln
        275                 280                 285

His Asn Ser Val Ala Asp Val Met Arg Tyr Asn Leu Asn Phe Ser Ala
    290                 295                 300

Asn Ser Val Asp Asn Leu Lys Ser Gly Val Ile Val Phe Lys Thr Leu
305                 310                 315                 320

Gln Tyr Asp Val Leu Phe Tyr Cys Ser Asn Ser Ser Ser Gly Val Leu
                325                 330                 335

Asp Thr Thr Ile Pro Phe Gly Pro Ser Ser Gln Pro Tyr Tyr Cys Phe
            340                 345                 350

Ile Asn Ser Thr Ile Asn Thr Thr His Val Ser Thr Phe Val Gly Ile
        355                 360                 365

Leu Pro Pro Thr Val Arg Glu Ile Val Val Ala Arg Thr Gly Gln Phe
    370                 375                 380

Tyr Ile Asn Gly Phe Lys Tyr Phe Asp Leu Gly Phe Ile Glu Ala Val
385                 390                 395                 400

Asn Phe Asn Val Thr Thr Ala Ser Ala Thr Asp Phe Trp Thr Val Ala
                405                 410                 415

Phe Ala Thr Phe Val Asp Val Leu Val Asn Val Ser Ala Thr Lys Ile

420                 425                 430

Gln Asn Leu Leu Tyr Cys Asp Ser Pro Phe Glu Lys Leu Gln Cys Glu
        435                 440                 445

His Leu Gln Phe Gly Leu Gln Asp Gly Phe Tyr Ser Ala Asn Phe Leu
    450                 455                 460

Asp Asp Asn Val Leu Pro Glu Thr Tyr Val Ala Leu Pro Ile Tyr Tyr
465                 470                 475                 480

Gln His Thr Asp Ile Asn Phe Thr Ala Thr Ala Ser Phe Gly Gly Ser
                485                 490                 495

Cys Tyr Val Cys Lys Pro His Gln Val Asn Ile Ser Leu Asn Gly Asn
            500                 505                 510

Thr Ser Val Cys Val Arg Thr Ser His Phe Ser Ile Arg Tyr Ile Tyr
        515                 520                 525

Asn Arg Val Lys Ser Gly Ser Pro Gly Asp Ser Ser Trp His Ile Tyr
    530                 535                 540

Leu Lys Ser Gly Thr Cys Pro Phe Ser Phe Ser Lys Leu Asn Asn Phe
545                 550                 555                 560

Gln Lys Phe Lys Thr Ile Cys Phe Ser Thr Val Glu Val Pro Gly Ser
                565                 570                 575

Cys Asn Phe Pro Leu Glu Ala Thr Trp His Tyr Thr Ser Tyr Thr Ile
            580                 585                 590

Val Gly Ala Leu Tyr Val Thr Trp Ser Glu Gly Asn Ser Ile Thr Gly
        595                 600                 605

Val Pro Tyr Pro Val Ser Gly Ile Arg Glu Phe Ser Asn Leu Val Leu
    610                 615                 620

Asn Asn Cys Thr Lys Tyr Asn Ile Tyr Asp Tyr Val Gly Thr Gly Ile
625                 630                 635                 640

Ile Arg Ser Ser Asn Gln Ser Leu Ala Gly Gly Ile Thr Tyr Val Ser
                645                 650                 655

Asn Ser Gly Asn Leu Leu Gly Phe Lys Asn Val Ser Thr Gly Asn Ile
            660                 665                 670

Phe Ile Val Thr Pro Cys Asn Gln Pro Asp Gln Val Ala Val Tyr Gln
        675                 680                 685

Gln Ser Ile Ile Gly Ala Met Thr Ala Val Asn Glu Ser Arg Tyr Gly
    690                 695                 700

Leu Gln Asn Leu Leu Gln Leu Pro Asn Phe Tyr Tyr Val Ser Asn Gly
705                 710                 715                 720

Gly Asn Asn Cys Thr Thr Ala Val Met Thr Tyr Ser Asn Phe Gly Ile
                725                 730                 735

Cys Ala Asp Gly Ser Leu Ile Pro Val Arg Pro Arg Asn Ser Ser Asp
            740                 745                 750

Asn Gly Ile Ser Ala Ile Ile Thr Ala Asn Leu Ser Ile Pro Ser Asn
        755                 760                 765

Trp Thr Thr Ser Val Gln Val Glu Tyr Leu Gln Ile Thr Ser Thr Pro
    770                 775                 780

Ile Val Val Asp Cys Ala Thr Tyr Val Cys Asn Gly Asn Pro Arg Cys
785                 790                 795                 800

Lys Asn Leu Leu Lys Gln Tyr Thr Ser Ala Cys Lys Thr Ile Glu Asp
                805                 810                 815

Ala Leu Arg Leu Ser Ala His Leu Glu Thr Asn Asp Val Ser Ser Met
            820                 825                 830

Leu Thr Phe Asp Ser Asn Ala Phe Ser Leu Ala Asn Val Thr Ser Phe
        835                 840                 845

Gly Asp Tyr Asn Leu Ser Ser Val Leu Pro Gln Arg Asn Ile Arg Ser
    850                 855                 860

Ser Arg Ile Ala Gly Arg Ser Ala Leu Glu Asp Leu Leu Phe Ser Lys
865                 870                 875                 880

Val Val Thr Ser Gly Leu Gly Thr Val Asp Val Asp Tyr Lys Ser Cys
                885                 890                 895

Thr Lys Gly Leu Ser Ile Ala Asp Leu Ala Cys Ala Gln Tyr Tyr Asn
            900                 905                 910

Gly Ile Met Val Leu Pro Gly Val Ala Asp Ala Glu Arg Met Ala Met
        915                 920                 925

Tyr Thr Gly Ser Leu Ile Gly Gly Met Val Leu Gly Gly Leu Thr Ser
    930                 935                 940

Ala Ala Ala Ile Pro Phe Ser Leu Ala Leu Gln Ala Arg Leu Asn Tyr
945                 950                 955                 960

Val Ala Leu Gln Thr Asp Val Leu Gln Glu Asn Gln Lys Ile Leu Ala
                965                 970                 975

Ala Ser Phe Asn Lys Ala Ile Asn Asn Ile Val Ala Ser Phe Ser Ser
            980                 985                 990

Val Asn Asp Ala Ile Thr Gln Thr  Ala Glu Ala Ile His  Thr Val Thr
        995                 1000                 1005

Ile Ala  Leu Asn Lys Ile Gln  Asp Val Val Asn Gln  Gln Gly Ser
    1010                 1015                 1020

Ala Leu  Asn His Leu Thr Ser  Gln Leu Arg His Asn  Phe Gln Ala
    1025                 1030                 1035

Ile Ser  Asn Ser Ile Gln Ala  Ile Tyr Asp Arg Leu  Asp Ser Ile
    1040                 1045                 1050

Gln Ala  Asp Gln Gln Val Asp  Arg Leu Ile Thr Gly  Arg Leu Ala
    1055                 1060                 1065

Ala Leu  Asn Ala Phe Val Ser  Gln Val Leu Asn Lys  Tyr Thr Glu
    1070                 1075                 1080

Val Arg  Ser Ser Arg Arg Leu  Ala Gln Gln Lys Val  Asn Glu Cys
    1085                 1090                 1095

Val Lys  Ser Gln Ser Asn Arg  Tyr Gly Phe Cys Gly  Asn Gly Thr
    1100                 1105                 1110

His Ile  Phe Ser Ile Val Asn  Ser Ala Pro Asp Gly  Leu Leu Phe
    1115                 1120                 1125

Leu His  Thr Val Leu Leu Pro  Thr Asp Tyr Lys Asn  Val Lys Ala
    1130                 1135                 1140

Trp Ser  Gly Ile Cys Val Asp  Gly Ile Tyr Gly Tyr  Val Leu Arg
    1145                 1150                 1155

Gln Pro  Asn Leu Val Leu Tyr  Ser Asp Asn Gly Val  Phe Arg Val
    1160                 1165                 1170

Thr Ser Arg Ile Met Phe Gln Pro Arg Leu Pro Val Leu Ser Asp
    1175                1180                1185

Phe Val Gln Ile Tyr Asn Cys Asn Val Thr Phe Val Asn Ile Ser
    1190                1195                1200

Arg Val Glu Leu His Thr Val Ile Pro Asp Tyr Val Asp Val Asn
    1205                1210                1215

Lys Thr Leu Gln Glu Phe Ala Gln Asn Leu Pro Lys Tyr Val Lys
    1220                1225                1230

Pro Asn Phe Asp Leu Thr Pro Phe Asn Leu Thr Tyr Leu Asn Leu
    1235                1240                1245

Ser Ser Glu Leu Lys Gln Leu Glu Ala Lys Thr Ala Ser Leu Phe
    1250                1255                1260

Gln Thr Thr Val Glu Leu Gln Gly Leu Ile Asp Gln Ile Asn Ser
    1265                1270                1275

Thr Tyr Val Asp Leu Lys Leu Leu Asn Arg Phe Glu Asn Tyr Ile
    1280                1285                1290

Lys Trp Pro Trp Trp Val Trp Leu Ile Ile Ser Val Val Phe Val
    1295                1300                1305

Val Leu Leu Ser Leu Leu Val Phe Cys Cys Leu Ser Thr Gly Cys
    1310                1315                1320

Cys Gly Cys Cys Asn Cys Leu Thr Ser Ser Met Arg Gly Cys Cys
    1325                1330                1335

Asp Cys Gly Ser Thr Lys Leu Pro Tyr Tyr Glu Phe
    1340                1345                1350

<210>  5
<211>  1350
<212>  PRT
<213>  Human coronavirus OC43

<400>  5

Met Phe Leu Ile Leu Leu Ile Ser Leu Pro Thr Ala Phe Ala Val Ile
1               5                   10                  15

Gly Asp Leu Asn Cys Pro Leu Asp Pro Lys Leu Lys Gly Ser Phe Asn
            20                  25                  30

Asn Arg Asp Thr Gly Ser Pro Ser Ile Ser Thr Asp Thr Val Asp Val
        35                  40                  45

Thr Asn Gly Leu Gly Thr Tyr Tyr Val Leu Asp Arg Val Tyr Leu Asn
    50                  55                  60

Thr Thr Leu Phe Leu Asn Gly Tyr Tyr Pro Thr Ser Gly Ser Thr Tyr
65                  70                  75                  80

Arg Asn Met Ala Leu Lys Gly Thr Asp Leu Leu Ser Thr Leu Trp Phe
                85                  90                  95

Lys Pro Pro Phe Leu Ser Asp Phe Ile Asn Gly Ile Phe Ala Lys Val
            100                 105                 110

Lys Asn Thr Lys Val Phe Lys Asp Gly Val Met Tyr Ser Glu Phe Pro
        115                 120                 125

Ala Ile Thr Ile Gly Ser Thr Phe Val Asn Thr Ser Tyr Ser Val Val
    130                 135                 140

Val Gln Pro Arg Thr Ile Asn Ser Thr Gln Asp Gly Val Asn Lys Leu
145                 150                 155                 160

Gln Gly Leu Leu Glu Val Ser Val Cys Gln Tyr Asn Met Cys Glu His
                165                 170                 175

Pro His Thr Ile Cys His Pro Asn Leu Gly Asn His Phe Lys Glu Leu
            180                 185                 190

Trp His Leu Asp Thr Gly Val Val Ser Cys Leu Tyr Lys Arg Asn Phe
        195                 200                 205

Thr Tyr Asp Val Asn Ala Thr Tyr Leu Tyr Phe His Phe Tyr Gln Glu
    210                 215                 220

Gly Gly Thr Phe Tyr Ala Tyr Phe Thr Asp Thr Gly Phe Val Thr Lys
225                 230                 235                 240

Phe Leu Phe Asn Val Tyr Leu Gly Met Ala Leu Ser His Tyr Tyr Val
                245                 250                 255

Met Pro Leu Thr Cys Ile Ser Arg Arg Asp Ile Gly Phe Thr Leu Glu
            260                 265                 270

Tyr Trp Val Thr Pro Leu Thr Pro Arg Gln Tyr Leu Leu Ala Phe Asn

275                 280                 285

Gln Asp Gly Ile Ile Phe Asn Ala Val Asp Cys Met Ser Asp Phe Met
    290                 295                 300

Ser Glu Ile Lys Cys Lys Thr Gln Ser Ile Ala Pro Pro Thr Gly Val
305                 310                 315                 320

Tyr Glu Leu Asn Gly Tyr Thr Val Gln Pro Ile Ala Asp Val Tyr Arg
                325                 330                 335

Arg Lys Pro Asp Leu Pro Asn Cys Asn Ile Glu Ala Trp Leu Asn Asp
            340                 345                 350

Lys Ser Val Pro Ser Pro Leu Asn Trp Glu Arg Lys Thr Phe Ser Asn
        355                 360                 365

Cys Asn Phe Asn Met Ser Ser Leu Met Ser Phe Ile Gln Ala Asp Ser
    370                 375                 380

Phe Thr Cys Asn Asn Ile Asp Ala Ala Lys Ile Tyr Gly Met Cys Phe
385                 390                 395                 400

Ser Ser Ile Thr Ile Asp Lys Phe Ala Ile Pro Asn Arg Arg Lys Val
                405                 410                 415

Asp Leu Gln Leu Gly Asn Leu Gly Tyr Leu Gln Ser Ser Asn Tyr Arg
            420                 425                 430

Ile Asp Thr Thr Ala Thr Ser Cys Gln Leu Tyr Tyr Asn Leu Pro Ala
        435                 440                 445

Ala Asn Val Ser Val Ser Arg Phe Asn Pro Ser Thr Trp Asn Lys Arg
    450                 455                 460

Phe Gly Phe Ile Glu Asp Ser Val Phe Val Pro Gln Pro Thr Gly Val
465                 470                 475                 480

Phe Thr Asn His Ser Val Val Tyr Ala Gln His Cys Phe Lys Ala Pro
                485                 490                 495

Lys Asn Phe Cys Pro Cys Ser Ser Cys Pro Gly Lys Asn Asn Gly Ile
            500                 505                 510

Gly Thr Cys Pro Ala Gly Thr Asn Ser Leu Thr Cys Asp Asn Leu Cys
        515                 520                 525

Thr Leu Asp Pro Ile Thr Phe Lys Ala Pro Asp Thr Tyr Lys Cys Pro
    530                 535                 540

Gln Thr Lys Ser Leu Val Gly Ile Gly Glu His Cys Ser Gly Leu Ala
545                 550                 555                 560

Val Lys Ser Asp Tyr Cys Gly Asn Asn Ser Cys Thr Cys Gln Pro Gln
                565                 570                 575

Ala Phe Leu Gly Trp Ser Ala Asp Ser Cys Leu Gln Gly Asp Lys Cys
            580                 585                 590

Asn Ile Phe Ala Asn Phe Ile Leu His Asp Val Asn Asn Gly Leu Thr
        595                 600                 605

Cys Ser Thr Asp Leu Gln Lys Ala Asn Thr Glu Ile Glu Leu Gly Val
    610                 615                 620

Cys Val Asn Tyr Asp Leu Tyr Gly Ile Ser Gly Gln Gly Ile Phe Val
625                 630                 635                 640

Glu Val Asn Ala Thr Tyr Tyr Asn Ser Trp Gln Asn Leu Leu Tyr Asp
                645                 650                 655

Ser Asn Gly Asn Leu Tyr Gly Phe Arg Asp Tyr Ile Thr Asn Arg Thr
            660                 665                 670

Phe Met Ile His Ser Cys Tyr Ser Gly Arg Val Ser Ala Ala Tyr His
        675                 680                 685

Ala Asn Ser Ser Glu Pro Ala Leu Leu Phe Arg Asn Ile Lys Cys Asn
    690                 695                 700

Tyr Val Phe Asn Asn Ser Leu Thr Arg Gln Leu Gln Pro Ile Asn Tyr
705                 710                 715                 720

Ser Phe Asp Ser Tyr Leu Gly Cys Val Val Asn Ala Tyr Asn Ser Thr
                725                 730                 735

Ala Ile Ser Val Gln Thr Cys Asp Leu Thr Val Gly Ser Gly Tyr Cys
            740                 745                 750

Val Asp Tyr Ser Lys Asn Arg Arg Ser Arg Arg Ala Ile Thr Thr Gly
        755                 760                 765

Tyr Arg Phe Thr Asn Phe Glu Pro Phe Thr Val Asn Ser Val Asn Asp
    770                 775                 780

Ser Leu Glu Pro Val Gly Gly Leu Tyr Glu Ile Gln Ile Pro Ser Glu
785                 790                 795                 800

Phe Thr Ile Gly Asn Met Glu Glu Phe Ile Gln Thr Ser Ser Pro Lys
                805                 810                 815

Val Thr Ile Asp Cys Ala Ala Phe Val Cys Gly Asp Tyr Ala Ala Cys
            820                 825                 830

Lys Leu Gln Leu Val Glu Tyr Gly Ser Phe Cys Asp Asn Ile Asn Ala
        835                 840                 845

Ile Leu Thr Glu Val Asn Glu Leu Leu Asp Thr Thr Gln Leu Gln Val
    850                 855                 860

Ala Asn Ser Leu Met Asn Gly Val Thr Leu Ser Thr Lys Leu Lys Asp
865                 870                 875                 880

Gly Val Asn Phe Asn Val Asp Asp Ile Asn Phe Ser Pro Val Leu Gly
                885                 890                 895

Cys Leu Gly Ser Glu Cys Ser Lys Ala Ser Ser Arg Ser Ala Ile Glu
            900                 905                 910

Asp Leu Leu Phe Asp Lys Val Lys Leu Ser Asp Val Gly Phe Val Glu
        915                 920                 925

Ala Tyr Asn Asn Cys Thr Gly Gly Ala Glu Ile Arg Asp Leu Ile Cys
    930                 935                 940

Val Gln Ser Tyr Lys Gly Ile Lys Val Leu Pro Pro Leu Leu Ser Glu
945                 950                 955                 960

Asn Gln Ile Ser Gly Tyr Thr Leu Ala Ala Thr Ser Ala Ser Leu Phe
                965                 970                 975

Pro Pro Trp Thr Ala Ala Ala Gly Val Pro Phe Tyr Leu Asn Val Gln
            980                 985                 990

Tyr Arg Ile Asn Gly Leu Gly Val  Thr Met Asp Val Leu  Ser Gln Asn
        995                 1000                 1005

Gln Lys  Leu Ile Ala Asn Ala  Phe Asn Asn Ala Leu  His Ala Ile
    1010                 1015                 1020

Gln Gln  Gly Phe Asp Ala Thr  Asn Ser Ala Leu Val  Lys Ile Gln
    1025                 1030                 1035

Ala Val Val Asn Ala Asn Ser Glu Ala Leu Asn Asn Leu Leu Gln
    1040                1045                1050

Gln Leu Ser Asn Arg Phe Gly Ala Ile Ser Ala Ser Leu Gln Glu
    1055                1060                1065

Ile Leu Ser Arg Leu Asp Ala Leu Glu Ala Glu Ala Gln Ile Asp
    1070                1075                1080

Arg Leu Ile Asn Gly Arg Leu Thr Ala Leu Asn Ala Tyr Val Ser
    1085                1090                1095

Gln Gln Leu Ser Asp Ser Thr Leu Val Lys Phe Ser Ala Ala Gln
    1100                1105                1110

Ala Met Glu Lys Val Asn Glu Cys Val Lys Ser Gln Ser Ser Arg
    1115                1120                1125

Ile Asn Phe Cys Gly Asn Gly Asn His Ile Ile Ser Leu Val Gln
    1130                1135                1140

Asn Ala Pro Tyr Gly Leu Tyr Phe Ile His Phe Asn Tyr Val Pro
    1145                1150                1155

Thr Lys Tyr Val Thr Ala Lys Val Ser Pro Gly Leu Cys Ile Ala
    1160                1165                1170

Gly Asn Arg Gly Ile Ala Pro Lys Ser Gly Tyr Phe Val Asn Val
    1175                1180                1185

Asn Asn Thr Trp Met Tyr Thr Gly Ser Gly Tyr Tyr Tyr Pro Glu
    1190                1195                1200

Pro Ile Thr Glu Asn Asn Val Val Val Met Ser Thr Cys Ala Val
    1205                1210                1215

Asn Tyr Thr Lys Ala Pro Tyr Val Met Leu Asn Thr Ser Ile Pro
    1220                1225                1230

Asn Leu Pro Asp Phe Lys Glu Glu Leu Gly Gln Trp Phe Lys Asn
    1235                1240                1245

Gln Thr Ser Val Ala Pro Asp Leu Ser Leu Asp Tyr Ile Asn Val
    1250                1255                1260

Thr Phe Leu Asp Leu Gln Val Glu Met Asn Arg Leu Gln Glu Ala

1265                    1270                    1275

Ile Lys  Val Leu Asn His Ser  Tyr Ile Asn Leu Lys  Asp Ile Gly
    1280                    1285                    1290

Thr Tyr  Glu Tyr Tyr Val Lys  Trp Pro Trp Tyr Val  Trp Leu Leu
    1295                    1300                    1305

Ile Cys  Leu Ala Gly Val Ala  Met Leu Val Leu Leu  Phe Phe Ile
    1310                    1315                    1320

Cys Cys  Cys Thr Gly Cys Gly  Thr Ser Cys Phe Lys  Lys Cys Gly
    1325                    1330                    1335

Gly Cys  Cys Asp Asp Tyr Thr  Gly Tyr Gln Glu Leu
    1340                    1345                    1350

<210>  6
<211>  1350
<212>  PRT
<213>  Human coronavirus HKU1

<400>  6

Met Phe Leu Ile Ile Phe Ile Leu Pro Thr Thr Leu Ala Val Ile Gly
1               5                   10                  15

Asp Phe Asn Cys Thr Asn Ser Phe Ile Asn Asp Tyr Asn Lys Thr Ile
            20                  25                  30

Pro Arg Ile Ser Glu Asp Val Val Asp Val Ser Leu Gly Leu Gly Thr
        35                  40                  45

Tyr Tyr Val Leu Asn Arg Val Tyr Leu Asn Thr Thr Leu Leu Phe Thr
    50                  55                  60

Gly Tyr Phe Pro Lys Ser Gly Ala Asn Phe Arg Asp Leu Ala Leu Lys
65                  70                  75                  80

Gly Ser Lys Tyr Leu Ser Thr Leu Trp Tyr Lys Pro Pro Phe Leu Ser
                85                  90                  95

Asp Phe Asn Asn Gly Ile Phe Ser Lys Val Lys Asn Thr Lys Leu Tyr
            100                 105                 110

Val Asn Asn Thr Leu Tyr Ser Glu Phe Ser Thr Ile Val Ile Gly Ser
        115                 120                 125

Val Phe Val Asn Thr Ser Tyr Thr Ile Val Val Gln Pro His Asn Gly

    130                 135                 140

Ile Leu Glu Ile Thr Ala Cys Gln Tyr Thr Met Cys Glu Tyr Pro His
145                 150                 155                 160

Thr Val Cys Lys Ser Lys Gly Ser Ile Arg Asn Glu Ser Trp His Ile
                165                 170                 175

Asp Ser Ser Glu Pro Leu Cys Leu Phe Lys Lys Asn Phe Thr Tyr Asn
            180                 185                 190

Val Ser Ala Asp Trp Leu Tyr Phe His Phe Tyr Gln Glu Arg Gly Val
        195                 200                 205

Phe Tyr Ala Tyr Tyr Ala Asp Val Gly Met Pro Thr Thr Phe Leu Phe
    210                 215                 220

Ser Leu Tyr Leu Gly Thr Ile Leu Ser His Tyr Tyr Val Met Pro Leu
225                 230                 235                 240

Thr Cys Lys Ala Ile Ser Ser Asn Thr Asp Asn Glu Thr Leu Glu Tyr
                245                 250                 255

Trp Val Thr Pro Leu Ser Arg Arg Gln Tyr Leu Leu Asn Phe Asp Glu
            260                 265                 270

His Gly Val Ile Thr Asn Ala Val Asp Cys Ser Ser Ser Phe Leu Ser
        275                 280                 285

Glu Ile Gln Cys Lys Thr Gln Ser Phe Ala Pro Asn Thr Gly Val Tyr
    290                 295                 300

Asp Leu Ser Gly Phe Thr Val Lys Pro Val Ala Thr Val Tyr Arg Arg
305                 310                 315                 320

Ile Pro Asn Leu Pro Asp Cys Asp Ile Asp Asn Trp Leu Asn Asn Val
                325                 330                 335

Ser Val Pro Ser Pro Leu Asn Trp Glu Arg Arg Ile Phe Ser Asn Cys
            340                 345                 350

Asn Phe Asn Leu Ser Thr Leu Leu Arg Leu Val His Val Asp Ser Phe
        355                 360                 365

Ser Cys Asn Asn Leu Asp Lys Ser Lys Ile Phe Gly Ser Cys Phe Asn
    370                 375                 380

Ser Ile Thr Val Asp Lys Phe Ala Ile Pro Asn Arg Arg Arg Asp Asp
385                 390                 395                 400

Leu Gln Leu Gly Ser Ser Gly Phe Leu Gln Ser Ser Asn Tyr Lys Ile
                405                 410                 415

Asp Ile Ser Ser Ser Ser Cys Gln Leu Tyr Tyr Ser Leu Pro Leu Val
            420                 425                 430

Asn Val Thr Ile Asn Asn Phe Asn Pro Ser Ser Trp Asn Arg Arg Tyr
        435                 440                 445

Gly Phe Gly Ser Phe Asn Val Ser Ser Tyr Asp Val Val Tyr Ser Asp
    450                 455                 460

His Cys Phe Ser Val Asn Ser Asp Phe Cys Pro Cys Ala Asp Pro Ser
465                 470                 475                 480

Val Val Asn Ser Cys Val Lys Ser Lys Pro Leu Ser Ala Ile Cys Pro
                485                 490                 495

Ala Gly Thr Lys Tyr Arg His Cys Asp Leu Asp Thr Thr Leu Tyr Val
            500                 505                 510

Asn Asn Trp Cys Arg Cys Ser Cys Leu Pro Asp Pro Ile Ser Thr Tyr
        515                 520                 525

Ser Pro Asn Thr Cys Pro Gln Lys Lys Val Val Val Gly Ile Gly Glu
    530                 535                 540

His Cys Pro Gly Leu Gly Ile Asn Glu Glu Lys Cys Gly Thr Gln Leu
545                 550                 555                 560

Asn His Ser Ser Cys Ser Cys Ser Pro Asp Ala Phe Leu Gly Trp Ser
                565                 570                 575

Phe Asp Ser Cys Ile Ser Asn Asn Arg Cys Asn Ile Phe Ser Asn Phe
            580                 585                 590

Ile Phe Asn Gly Ile Asn Ser Gly Thr Thr Cys Ser Asn Asp Leu Leu
        595                 600                 605

Tyr Ser Asn Thr Glu Val Ser Thr Gly Val Cys Val Asn Tyr Asp Leu
    610                 615                 620

Tyr Gly Ile Thr Gly Gln Gly Ile Phe Lys Glu Val Ser Ala Ala Tyr
625                 630                 635                 640

Tyr Asn Asn Trp Gln Asn Leu Leu Tyr Asp Ser Asn Gly Asn Ile Ile
                645                 650                 655

Gly Phe Lys Asp Phe Leu Thr Asn Lys Thr Tyr Thr Ile Leu Pro Cys
            660                 665                 670

Tyr Ser Gly Arg Val Ser Ala Ala Phe Tyr Gln Asn Ser Ser Ser Pro
        675                 680                 685

Ala Leu Leu Tyr Arg Asn Leu Lys Cys Ser Tyr Val Leu Asn Asn Ile
    690                 695                 700

Ser Phe Ile Ser Gln Pro Phe Tyr Phe Asp Ser Tyr Leu Gly Cys Val
705                 710                 715                 720

Leu Asn Ala Val Asn Leu Thr Ser Tyr Ser Val Ser Ser Cys Asp Leu
                725                 730                 735

Arg Met Gly Ser Gly Phe Cys Ile Asp Tyr Ala Leu Pro Ser Ser Arg
            740                 745                 750

Arg Lys Arg Arg Gly Ile Ser Ser Pro Tyr Arg Phe Val Thr Phe Glu
        755                 760                 765

Pro Phe Asn Val Ser Phe Val Asn Asp Ser Val Glu Thr Val Gly Gly
    770                 775                 780

Leu Phe Glu Ile Gln Ile Pro Thr Asn Phe Thr Ile Ala Gly His Glu
785                 790                 795                 800

Glu Phe Ile Gln Thr Ser Ser Pro Lys Val Thr Ile Asp Cys Ser Ala
                805                 810                 815

Phe Val Cys Ser Asn Tyr Ala Ala Cys His Asp Leu Leu Ser Glu Tyr
            820                 825                 830

Gly Thr Phe Cys Asp Asn Ile Asn Ser Ile Leu Asn Glu Val Asn Asp
        835                 840                 845

Leu Leu Asp Ile Thr Gln Leu Gln Val Ala Asn Ala Leu Met Gln Gly
    850                 855                 860

Val Thr Leu Ser Ser Asn Leu Asn Thr Asn Leu His Ser Asp Val Asp
865                 870                 875                 880

Asn Ile Asp Phe Lys Ser Leu Leu Gly Cys Leu Gly Ser Gln Cys Gly
                885                 890                 895

Ser Ser Ser Arg Ser Leu Leu Glu Asp Leu Leu Phe Asn Lys Val Lys
                900                 905                 910

Leu Ser Asp Val Gly Phe Val Glu Ala Tyr Asn Asn Cys Thr Gly Gly
            915                 920                 925

Ser Glu Ile Arg Asp Leu Leu Cys Val Gln Ser Phe Asn Gly Ile Lys
    930                 935                 940

Val Leu Pro Pro Ile Leu Ser Glu Thr Gln Ile Ser Gly Tyr Thr Thr
945                 950                 955                 960

Ala Ala Thr Val Ala Ala Met Phe Pro Pro Trp Ser Ala Ala Ala Gly
                965                 970                 975

Val Pro Phe Ser Leu Asn Val Gln Tyr Arg Ile Asn Gly Leu Gly Val
            980                 985                 990

Thr Met Asp Val Leu Asn Lys Asn  Gln Lys Leu Ile Ala  Asn Ala Phe
        995                 1000                1005

Asn Lys  Ala Leu Leu Ser Ile  Gln Asn Gly Phe Thr  Ala Thr Asn
    1010                1015                1020

Ser Ala  Leu Ala Lys Ile Gln  Ser Val Val Asn Ala  Asn Ala Gln
    1025                1030                1035

Ala Leu  Asn Ser Leu Leu Gln  Gln Leu Phe Asn Lys  Phe Gly Ala
    1040                1045                1050

Ile Ser  Ser Ser Leu Gln Glu  Ile Leu Ser Arg Leu  Asp Asn Leu
    1055                1060                1065

Glu Ala  Gln Val Gln Ile Asp  Arg Leu Ile Asn Gly  Arg Leu Thr
    1070                1075                1080

Ala Leu  Asn Ala Tyr Val Ser  Gln Gln Leu Ser Asp  Ile Thr Leu
    1085                1090                1095

Ile Lys  Ala Gly Ala Ser Arg  Ala Ile Glu Lys Val  Asn Glu Cys
    1100                1105                1110

Val Lys  Ser Gln Ser Pro Arg  Ile Asn Phe Cys Gly  Asn Gly Asn
    1115                1120                1125

His Ile  Leu Ser Leu Val Gln  Asn Ala Pro Tyr Gly  Leu Leu Phe

1130                1135                1140

Ile His Phe Ser Tyr Lys Pro Thr Ser Phe Lys Thr Val Leu Val
    1145                1150                1155

Ser Pro Gly Leu Cys Leu Ser Gly Asp Arg Gly Ile Ala Pro Lys
    1160                1165                1170

Gln Gly Tyr Phe Ile Lys Gln Asn Asp Ser Trp Met Phe Thr Gly
    1175                1180                1185

Ser Ser Tyr Tyr Tyr Pro Glu Pro Ile Ser Asp Lys Asn Val Val
    1190                1195                1200

Phe Met Asn Ser Cys Ser Val Asn Phe Thr Lys Ala Pro Phe Ile
    1205                1210                1215

Tyr Leu Asn Asn Ser Ile Pro Asn Leu Ser Asp Phe Glu Ala Glu
    1220                1225                1230

Phe Ser Leu Trp Phe Lys Asn His Thr Ser Ile Ala Pro Asn Leu
    1235                1240                1245

Thr Phe Asn Ser His Ile Asn Ala Thr Phe Leu Asp Leu Tyr Tyr
    1250                1255                1260

Glu Met Asn Val Ile Gln Glu Ser Ile Lys Ser Leu Asn Ser Ser
    1265                1270                1275

Phe Ile Asn Leu Lys Glu Ile Gly Thr Tyr Glu Met Tyr Val Lys
    1280                1285                1290

Trp Pro Trp Tyr Ile Trp Leu Leu Ile Val Ile Leu Phe Ile Ile
    1295                1300                1305

Phe Leu Met Ile Leu Phe Phe Ile Cys Cys Cys Thr Gly Cys Gly
    1310                1315                1320

Ser Ala Cys Phe Ser Lys Cys His Asn Cys Cys Asp Glu Tyr Gly
    1325                1330                1335

Gly His Asn Asp Phe Val Ile Lys Ala Ser His Asp
    1340                1345                1350

<210>  7
<211>  1173
<212>  PRT
<213>  Human coronavirus 229E

<400>  7

Met Phe Val Leu Leu Val Ala Tyr Ala Leu Leu His Ile Ala Gly Cys
1               5                   10                  15

Gln Thr Thr Asn Gly Leu Asn Thr Ser Tyr Ser Val Cys Asn Gly Cys
            20                  25                  30

Val Gly Tyr Ser Glu Asn Val Phe Ala Val Glu Ser Gly Gly Tyr Ile
        35                  40                  45

Pro Ser Asp Phe Ala Phe Asn Asn Trp Phe Leu Leu Thr Asn Thr Ser
    50                  55                  60

Ser Val Val Asp Gly Val Val Arg Ser Phe Gln Pro Leu Leu Leu Asn
65                  70                  75                  80

Cys Leu Trp Ser Val Ser Gly Leu Arg Phe Thr Thr Gly Phe Val Tyr
                85                  90                  95

Phe Asn Gly Thr Gly Arg Gly Asp Cys Lys Gly Phe Ser Ser Asp Val
            100                 105                 110

Leu Ser Asp Val Ile Arg Tyr Asn Leu Asn Phe Glu Glu Asn Leu Arg
        115                 120                 125

Arg Gly Thr Ile Leu Phe Lys Thr Ser Tyr Gly Val Val Val Phe Tyr
    130                 135                 140

Cys Thr Asn Asn Thr Leu Val Ser Gly Asp Ala His Ile Pro Phe Gly
145                 150                 155                 160

Thr Val Leu Gly Asn Phe Tyr Cys Phe Val Asn Thr Thr Ile Gly Asn
                165                 170                 175

Glu Thr Thr Ser Ala Phe Val Gly Ala Leu Pro Lys Thr Val Arg Glu
            180                 185                 190

Phe Val Ile Ser Arg Thr Gly His Phe Tyr Ile Asn Gly Tyr Arg Tyr
        195                 200                 205

Phe Thr Leu Gly Asn Val Glu Ala Val Asn Phe Asn Val Thr Thr Ala
    210                 215                 220

Glu Thr Thr Asp Phe Cys Thr Val Ala Leu Ala Ser Tyr Ala Asp Val
225                 230                 235                 240

Leu Val Asn Val Ser Gln Thr Ser Ile Ala Asn Ile Ile Tyr Cys Asn
                245                 250                 255

Ser Val Ile Asn Arg Leu Arg Cys Asp Gln Leu Ser Phe Asp Val Pro
            260                 265                 270

Asp Gly Phe Tyr Ser Thr Ser Pro Ile Gln Ser Val Glu Leu Pro Val
        275                 280                 285

Ser Ile Val Ser Leu Pro Val Tyr His Lys His Thr Phe Ile Val Leu
    290                 295                 300

Tyr Val Asp Phe Lys Pro Gln Ser Gly Gly Gly Lys Cys Phe Asn Cys
305                 310                 315                 320

Tyr Pro Ala Gly Val Asn Ile Thr Leu Ala Asn Phe Asn Glu Thr Lys
                325                 330                 335

Gly Pro Leu Cys Val Asp Thr Ser His Phe Thr Thr Lys Tyr Val Ala
            340                 345                 350

Val Tyr Ala Asn Val Gly Arg Trp Ser Ala Ser Ile Asn Thr Gly Asn
        355                 360                 365

Cys Pro Phe Ser Phe Gly Lys Val Asn Asn Phe Val Lys Phe Gly Ser
    370                 375                 380

Val Cys Phe Ser Leu Lys Asp Ile Pro Gly Gly Cys Ala Met Pro Ile
385                 390                 395                 400

Val Ala Asn Trp Ala Tyr Ser Lys Tyr Tyr Thr Ile Gly Ser Leu Tyr
                405                 410                 415

Val Ser Trp Ser Asp Gly Asp Gly Ile Thr Gly Val Pro Gln Pro Val
            420                 425                 430

Glu Gly Val Ser Ser Phe Met Asn Val Thr Leu Asp Lys Cys Thr Lys
        435                 440                 445

Tyr Asn Ile Tyr Asp Val Ser Gly Val Gly Val Ile Arg Val Ser Asn
    450                 455                 460

Asp Thr Phe Leu Asn Gly Ile Thr Tyr Thr Ser Thr Ser Gly Asn Leu
465                 470                 475                 480

Leu Gly Phe Lys Asp Val Thr Lys Gly Thr Ile Tyr Ser Ile Thr Pro
                485                 490                 495

Cys Asn Pro Pro Asp Gln Leu Val Val Tyr Gln Gln Ala Val Val Gly
            500                 505                 510

Ala Met Leu Ser Glu Asn Phe Thr Ser Tyr Gly Phe Ser Asn Val Val
        515                 520                 525

Glu Leu Pro Lys Phe Phe Tyr Ala Ser Asn Gly Thr Tyr Asn Cys Thr
    530                 535                 540

Asp Ala Val Leu Thr Tyr Ser Ser Phe Gly Val Cys Ala Asp Gly Ser
545                 550                 555                 560

Ile Ile Ala Val Gln Pro Arg Asn Val Ser Tyr Asp Ser Val Ser Ala
                565                 570                 575

Ile Val Thr Ala Asn Leu Ser Ile Pro Ser Asn Trp Thr Thr Ser Val
            580                 585                 590

Gln Val Glu Tyr Leu Gln Ile Thr Ser Thr Pro Ile Val Val Asp Cys
        595                 600                 605

Ser Thr Tyr Val Cys Asn Gly Asn Val Arg Cys Val Glu Leu Leu Lys
    610                 615                 620

Gln Tyr Thr Ser Ala Cys Lys Thr Ile Glu Asp Ala Leu Arg Asn Ser
625                 630                 635                 640

Ala Arg Leu Glu Ser Ala Asp Val Ser Glu Met Leu Thr Phe Asp Lys
                645                 650                 655

Lys Ala Phe Thr Leu Ala Asn Val Ser Ser Phe Gly Asp Tyr Asn Leu
            660                 665                 670

Ser Ser Val Ile Pro Ser Leu Pro Thr Ser Gly Ser Arg Val Ala Gly
        675                 680                 685

Arg Ser Ala Ile Glu Asp Ile Leu Phe Ser Lys Leu Val Thr Ser Gly
    690                 695                 700

Leu Gly Thr Val Asp Ala Asp Tyr Lys Lys Cys Thr Lys Gly Leu Ser
705                 710                 715                 720

Ile Ala Asp Leu Ala Cys Ala Gln Tyr Tyr Asn Gly Ile Met Val Leu
                725                 730                 735

Pro Gly Val Ala Asp Ala Glu Arg Met Ala Met Tyr Thr Gly Ser Leu
            740                 745                 750

Ile Gly Gly Ile Ala Leu Gly Gly Leu Thr Ser Ala Val Ser Ile Pro
        755                 760                 765

Phe Ser Leu Ala Ile Gln Ala Arg Leu Asn Tyr Val Ala Leu Gln Thr
    770                 775                 780

Asp Val Leu Gln Glu Asn Gln Lys Ile Leu Ala Ala Ser Phe Asn Lys
785                 790                 795                 800

Ala Met Thr Asn Ile Val Asp Ala Phe Thr Gly Val Asn Asp Ala Ile
                805                 810                 815

Thr Gln Thr Ser Gln Ala Leu Gln Thr Val Ala Thr Ala Leu Asn Lys
            820                 825                 830

Ile Gln Asp Val Val Asn Gln Gln Gly Asn Ser Leu Asn His Leu Thr
        835                 840                 845

Ser Gln Leu Arg Gln Asn Phe Gln Ala Ile Ser Ser Ser Ile Gln Ala
    850                 855                 860

Ile Tyr Asp Arg Leu Asp Thr Ile Gln Ala Asp Gln Gln Val Asp Arg
865                 870                 875                 880

Leu Ile Thr Gly Arg Leu Ala Ala Leu Asn Val Phe Val Ser His Thr
                885                 890                 895

Leu Thr Lys Tyr Thr Glu Val Arg Ala Ser Arg Gln Leu Ala Gln Gln
            900                 905                 910

Lys Val Asn Glu Cys Val Lys Ser Gln Ser Lys Arg Tyr Gly Phe Cys
        915                 920                 925

Gly Asn Gly Thr His Ile Phe Ser Ile Val Asn Ala Ala Pro Glu Gly
    930                 935                 940

Leu Val Phe Leu His Thr Val Leu Leu Pro Thr Gln Tyr Lys Asp Val
945                 950                 955                 960

Glu Ala Trp Ser Gly Leu Cys Val Asp Gly Thr Asn Gly Tyr Val Leu
                965                 970                 975

Arg Gln Pro Asn Leu Ala Leu Tyr Lys Glu Gly Asn Tyr Tyr Arg Ile
            980                 985                 990

Thr Ser Arg Ile Met Phe Glu Pro  Arg Ile Pro Thr Met  Ala Asp Phe

995                 1000                1005

Val Gln  Ile Glu Asn Cys Asn  Val Thr Phe Val Asn  Ile Ser Arg
    1010                 1015                 1020

Ser Glu  Leu Gln Thr Ile Val  Pro Glu Tyr Ile Asp  Val Asn Lys
    1025                 1030                 1035

Thr Leu  Gln Glu Leu Ser Tyr  Lys Leu Pro Asn Tyr  Thr Val Pro
    1040                 1045                 1050

Asp Leu  Val Val Glu Gln Tyr  Asn Gln Thr Ile Leu  Asn Leu Thr
    1055                 1060                 1065

Ser Glu  Ile Ser Thr Leu Glu  Asn Lys Ser Ala Glu  Leu Asn Tyr
    1070                 1075                 1080

Thr Val  Gln Lys Leu Gln Thr  Leu Ile Asp Asn Ile  Asn Ser Thr
    1085                 1090                 1095

Leu Val  Asp Leu Lys Trp Leu  Asn Arg Val Glu Thr  Tyr Ile Lys
    1100                 1105                 1110

Trp Pro  Trp Trp Val Trp Leu  Cys Ile Ser Val Val  Leu Ile Phe
    1115                 1120                 1125

Val Val  Ser Met Leu Leu Leu  Cys Cys Cys Ser Thr  Gly Cys Cys
    1130                 1135                 1140

Gly Phe  Phe Ser Cys Phe Ala  Ser Ser Ile Arg Gly  Cys Cys Glu
    1145                 1150                 1155

Ser Thr  Lys Leu Pro Tyr Tyr  Asp Val Glu Lys Ile  His Ile Gln
    1160                 1165                 1170

<210>  8
<211>  30
<212>  PRT
<213>  Severe acute respiratory syndrome coronavirus 1

<400>  8

Arg Gly Val Tyr Tyr Pro Asp Glu Ile Phe Arg Ser Asp Thr Leu Tyr
1               5                   10                  15

Leu Thr Gln Asp Leu Phe Leu Pro Phe Tyr Ser Asn Val Thr
            20                  25                  30

<210>  9

<211>  42
<212>  PRT
<213>  Severe acute respiratory syndrome coronavirus 1

<400>  9

Asp Gly Ile Tyr Phe Ala Ala Thr Glu Lys Ser Asn Val Val Arg Gly
1               5                   10                  15

Trp Val Phe Gly Ser Thr Met Asn Asn Lys Ser Gln Ser Val Ile Ile
            20                  25                  30

Ile Asn Asn Ser Thr Asn Val Val Ile Arg
        35                  40

<210>  10
<211>  11
<212>  PRT
<213>  Severe acute respiratory syndrome coronavirus 1

<400>  10

Asn Ala Phe Asn Cys Thr Phe Glu Tyr Ile Ser
1               5                   10

<210>  11
<211>  13
<212>  PRT
<213>  Severe acute respiratory syndrome coronavirus 1

<400>  11

Gly Asn Phe Lys His Leu Arg Glu Phe Val Phe Lys Asn
1               5                   10

<210>  12
<211>  62
<212>  PRT
<213>  Severe acute respiratory syndrome coronavirus 1

<400>  12

Ala Ala Ala Tyr Phe Val Gly Tyr Leu Lys Pro Thr Thr Phe Met Leu
1               5                   10                  15

Lys Tyr Asp Glu Asn Gly Thr Ile Thr Asp Ala Val Asp Cys Ser Gln
            20                  25                  30

Asn Pro Leu Ala Glu Leu Lys Cys Ser Val Lys Ser Phe Glu Ile Asp
        35                  40                  45

Lys Gly Ile Tyr Gln Thr Ser Asn Phe Arg Val Val Pro Ser
    50                  55                  60

<210>  13
<211>  46
<212>  PRT
<213>  Severe acute respiratory syndrome coronavirus 1

<400>  13

Val Val Arg Phe Pro Asn Ile Thr Asn Leu Cys Pro Phe Gly Glu Val
1               5                   10                  15

Phe Asn Ala Thr Lys Phe Pro Ser Val Tyr Ala Trp Glu Arg Lys Lys
            20                  25                  30

Ile Ser Asn Cys Val Ala Asp Tyr Ser Val Leu Tyr Asn Ser
        35                  40                  45

<210>  14
<211>  70
<212>  PRT
<213>  Severe acute respiratory syndrome coronavirus 1

<400>  14

Phe Ser Thr Phe Lys Cys Tyr Gly Val Ser Ala Thr Lys Leu Asn Asp
1               5                   10                  15

Leu Cys Phe Ser Asn Val Tyr Ala Asp Ser Phe Val Val Lys Gly Asp
            20                  25                  30

Asp Val Arg Gln Ile Ala Pro Gly Gln Thr Gly Val Ile Ala Asp Tyr
        35                  40                  45

Asn Tyr Lys Leu Pro Asp Asp Phe Met Gly Cys Val Leu Ala Trp Asn
    50                  55                  60

Thr Arg Asn Ile Asp Ala
65                  70

<210>  15
<211>  9
<212>  PRT
<213>  Severe acute respiratory syndrome coronavirus 1

<400>  15

Leu Arg Pro Phe Glu Arg Asp Ile Ser
1               5

<210>  16
<211>  119
<212>  PRT
<213>  Severe acute respiratory syndrome coronavirus 1

<400>  16

Gly Ile Gly Tyr Gln Pro Tyr Arg Val Val Val Leu Ser Phe Glu Leu
1               5                   10                  15

Leu Asn Ala Pro Ala Thr Val Cys Gly Pro Lys Leu Ser Thr Asp Leu
            20                  25                  30

Ile Lys Asn Gln Cys Val Asn Phe Asn Phe Asn Gly Leu Thr Gly Thr
        35                  40                  45

Gly Val Leu Thr Pro Ser Ser Lys Arg Phe Gln Pro Phe Gln Gln Phe
    50                  55                  60

Gly Arg Asp Val Ser Asp Phe Thr Asp Ser Val Arg Asp Pro Lys Thr
65                  70                  75                  80

Ser Glu Ile Leu Asp Ile Ser Pro Cys Ser Phe Gly Gly Val Ser Val
                85                  90                  95

Ile Thr Pro Gly Thr Asn Ala Ser Ser Glu Val Ala Val Leu Tyr Gln
            100                 105                 110

Asp Val Asn Cys Thr Asp Val
        115

<210>  17
<211>  54
<212>  PRT
<213>  Severe acute respiratory syndrome coronavirus 1

<400>  17

Ala Ile His Ala Asp Gln Leu Thr Pro Ala Trp Arg Ile Tyr Ser Thr
1               5                   10                  15

Gly Asn Asn Val Phe Gln Thr Gln Ala Gly Cys Leu Ile Gly Ala Glu
            20                  25                  30

His Val Asp Thr Ser Tyr Glu Cys Asp Ile Pro Ile Gly Ala Gly Ile
        35                  40                  45

Cys Ala Ser Tyr His Thr
    50

<210>  18
<211>  247
<212>  PRT
<213>  Severe acute respiratory syndrome coronavirus 1

<400>  18

Arg Ser Thr Ser Gln Lys Ser Ile Val Ala Tyr Thr Met Ser Leu Gly
1               5                   10                  15

Ala Asp Ser Ser Ile Ala Tyr Ser Asn Asn Thr Ile Ala Ile Pro Thr
            20                  25                  30

Asn Phe Ser Ile Ser Ile Thr Thr Glu Val Met Pro Val Ser Met Ala
        35                  40                  45

Lys Thr Ser Val Asp Cys Asn Met Tyr Ile Cys Gly Asp Ser Thr Glu
    50                  55                  60

Cys Ala Asn Leu Leu Leu Gln Tyr Gly Ser Phe Cys Thr Gln Leu Asn
65                  70                  75                  80

Arg Ala Leu Ser Gly Ile Ala Ala Glu Gln Asp Arg Asn Thr Arg Glu
                85                  90                  95

Val Phe Ala Gln Val Lys Gln Met Tyr Lys Thr Pro Thr Leu Lys Tyr
            100                 105                 110

Phe Gly Gly Phe Asn Phe Ser Gln Ile Leu Pro Asp Pro Leu Lys Pro
        115                 120                 125

Thr Lys Arg Ser Phe Ile Glu Asp Leu Leu Phe Asn Lys Val Thr Leu
    130                 135                 140

Ala Asp Ala Gly Phe Met Lys Gln Tyr Gly Glu Cys Leu Gly Asp Ile
145                 150                 155                 160

Asn Ala Arg Asp Leu Ile Cys Ala Gln Lys Phe Asn Gly Leu Thr Val
                165                 170                 175

Leu Pro Pro Leu Leu Thr Asp Asp Met Ile Ala Ala Tyr Thr Ala Ala
            180                 185                 190

Leu Val Ser Gly Thr Ala Thr Ala Gly Trp Thr Phe Gly Ala Gly Ala
        195                 200                 205

Ala Leu Gln Ile Pro Phe Ala Met Gln Met Ala Tyr Arg Phe Asn Gly
    210                 215                 220

Ile Gly Val Thr Gln Asn Val Leu Tyr Glu Asn Gln Lys Gln Ile Ala
225                 230                 235                 240

Asn Gln Phe Asn Lys Ala Ile
                245

<210>  19
<211>  281
<212>  PRT
<213>  Severe acute respiratory syndrome coronavirus 1

<400>  19

Gln Ile Gln Glu Ser Leu Thr Thr Thr Ser Thr Ala Leu Gly Lys Leu
1               5                   10                  15

Gln Asp Val Val Asn Gln Asn Ala Gln Ala Leu Asn Thr Leu Val Lys
            20                  25                  30

Gln Leu Ser Ser Asn Phe Gly Ala Ile Ser Ser Val Leu Asn Asp Ile
        35                  40                  45

Leu Ser Arg Leu Asp Lys Val Glu Ala Glu Val Gln Ile Asp Arg Leu
    50                  55                  60

Ile Thr Gly Arg Leu Gln Ser Leu Gln Thr Tyr Val Thr Gln Gln Leu
65                  70                  75                  80

Ile Arg Ala Ala Glu Ile Arg Ala Ser Ala Asn Leu Ala Ala Thr Lys
                85                  90                  95

Met Ser Glu Cys Val Leu Gly Gln Ser Lys Arg Val Asp Phe Cys Gly
            100                 105                 110

Lys Gly Tyr His Leu Met Ser Phe Pro Gln Ala Ala Pro His Gly Val
        115                 120                 125

Val Phe Leu His Val Thr Tyr Val Pro Ser Gln Glu Arg Asn Phe Thr
    130                 135                 140

Thr Ala Pro Ala Ile Cys His Glu Gly Lys Ala Tyr Phe Pro Arg Glu
145                 150                 155                 160

Gly Val Phe Val Phe Asn Gly Thr Ser Trp Phe Ile Thr Gln Arg Asn
                165                 170                 175

Phe Phe Ser Pro Gln Ile Ile Thr Thr Asp Asn Thr Phe Val Ser Gly
            180                 185                 190

Asn Cys Asp Val Val Ile Gly Ile Ile Asn Asn Thr Val Tyr Asp Pro
        195                 200                 205

Leu Gln Pro Glu Leu Asp Ser Phe Lys Glu Glu Leu Asp Lys Tyr Phe
    210                 215                 220

Lys Asn His Thr Ser Pro Asp Val Asp Leu Gly Asp Ile Ser Gly Ile
225                 230                 235                 240

Asn Ala Ser Val Val Asn Ile Gln Lys Glu Ile Asp Arg Leu Asn Glu
                245                 250                 255

Val Ala Lys Asn Leu Asn Glu Ser Leu Ile Asp Leu Gln Glu Leu Gly
            260                 265                 270

Lys Tyr Glu Gln Tyr Ile Lys Trp Pro
        275                 280

<210>  20
<211>  10
<212>  PRT
<213>  Severe acute respiratory syndrome coronavirus 1

<400>  20

Lys Ser Phe Glu Ile Asp Lys Gly Ile Tyr
1               5                   10

<210>  21
<211>  13
<212>  PRT
<213>  Severe acute respiratory syndrome coronavirus 1

<400>  21

Gln Tyr Gly Ser Phe Cys Thr Gln Leu Asn Arg Ala Leu
1               5                   10

<210>  22
<211>  13
<212>  PRT
<213>  Severe acute respiratory syndrome coronavirus 1

<400>  22

Ile Glu Asp Leu Leu Phe Asn Lys Val Thr Leu Ala Asp
1               5                   10

<210>  23
<211>  9
<212>  PRT
<213>  Severe acute respiratory syndrome coronavirus 1

<400>  23

Asn Ala Arg Asp Leu Ile Cys Ala Gln
1               5

<210>  24
<211>  11

<212>  PRT
<213>  Severe acute respiratory syndrome coronavirus 1

<400>  24

Asp Met Ile Ala Ala Tyr Thr Ala Ala Leu Val
1               5                   10


<210>  25
<211>  28
<212>  PRT
<213>  Severe acute respiratory syndrome coronavirus 1

<400>  25

Tyr Arg Phe Asn Gly Ile Gly Val Thr Gln Asn Val Leu Tyr Glu Asn
1               5                   10                  15


Gln Lys Gln Ile Ala Asn Gln Phe Asn Lys Ala Ile
            20                  25


<210>  26
<211>  11
<212>  PRT
<213>  Severe acute respiratory syndrome coronavirus 1

<400>  26

Gln Leu Ser Ser Asn Phe Gly Ala Ile Ser Ser
1               5                   10


<210>  27
<211>  10
<212>  PRT
<213>  Severe acute respiratory syndrome coronavirus 1

<400>  27

Gln Ile Asp Arg Leu Ile Thr Gly Arg Leu
1               5                   10


<210>  28
<211>  11
<212>  PRT
<213>  Severe acute respiratory syndrome coronavirus 1

<400>  28

Phe Cys Gly Lys Gly Tyr His Leu Met Ser Phe
1               5                   10


<210>  29
<211>  10
<212>  PRT
<213>  Severe acute respiratory syndrome coronavirus 1

<400>  29

Ala Pro His Gly Val Val Phe Leu His Val
1               5                   10


<210>  30
<211>  11
<212>  PRT
<213>  Severe acute respiratory syndrome coronavirus 1

<400>  30

Phe Lys Glu Glu Leu Asp Lys Tyr Phe Lys Asn
1               5                   10


<210>  31
<211>  12
<212>  PRT
<213>  Severe acute respiratory syndrome coronavirus 1

<400>  31

Leu Asn Glu Ser Leu Ile Asp Leu Gln Glu Leu Gly
1               5                   10


<210>  32
<211>  11
<212>  PRT
<213>  Severe acute respiratory syndrome coronavirus 1

<400>  32

Ile Ala Ile Pro Thr Asn Phe Ser Ile Ser Ile
1               5                   10


<210>  33
<211>  12
<212>  PRT
<213>  Severe acute respiratory syndrome coronavirus 1

<400>  33

Arg Ser Phe Ile Glu Asp Leu Leu Phe Asn Lys Val
1               5                   10


<210>  34
<211>  11
<212>  PRT
<213>  Severe acute respiratory syndrome coronavirus 1

<400>  34

Ala Leu Gly Lys Leu Gln Asp Val Val Asn Gln
1               5                   10


<210>  35
<211>  15
<212>  PRT

<213>  Severe acute respiratory syndrome coronavirus 1

<400>  35

Val Glu Ala Glu Val Gln Ile Asp Arg Leu Ile Thr Gly Arg Leu
1               5                   10                  15


<210>  36
<211>  10
<212>  PRT
<213>  Severe acute respiratory syndrome coronavirus 1

<400>  36

Ala Ala Pro His Gly Val Val Phe Leu His
1               5                   10


<210>  37
<211>  11
<212>  PRT
<213>  Severe acute respiratory syndrome coronavirus 1

<400>  37

Ser Ser Glu Val Ala Val Leu Tyr Gln Asp Val
1               5                   10


<210>  38
<211>  16
<212>  PRT
<213>  Severe acute respiratory syndrome coronavirus 1

<400>  38

Arg Ser Phe Ile Glu Asp Leu Leu Phe Asn Lys Val Thr Leu Ala Asp
1               5                   10                  15


<210>  39
<211>  12
<212>  PRT
<213>  Severe acute respiratory syndrome coronavirus 1

<400>  39

Val Leu Pro Pro Leu Leu Thr Asp Asp Met Ile Ala
1               5                   10


<210>  40
<211>  10
<212>  PRT
<213>  Severe acute respiratory syndrome coronavirus 1

<400>  40

Asn Gly Ile Gly Val Thr Gln Asn Val Leu
1               5                   10

<210>  41
<211>  35
<212>  PRT
<213>  Severe acute respiratory syndrome coronavirus 1

<400>  41

Thr Ser Thr Ala Leu Gly Lys Leu Gln Asp Val Val Asn Gln Asn Ala
1               5                   10                  15

Gln Ala Leu Asn Thr Leu Val Lys Gln Leu Ser Ser Asn Phe Gly Ala
            20                  25                  30

Ile Ser Ser
        35

<210>  42
<211>  11
<212>  PRT
<213>  Severe acute respiratory syndrome coronavirus 1

<400>  42

Asp Ile Leu Ser Arg Leu Asp Lys Val Glu Ala
1               5                   10

<210>  43
<211>  10
<212>  PRT
<213>  Severe acute respiratory syndrome coronavirus 1

<400>  43

Gln Ile Asp Arg Leu Ile Thr Gly Arg Leu
1               5                   10

<210>  44
<211>  11
<212>  PRT
<213>  Severe acute respiratory syndrome coronavirus 1

<400>  44

Phe Lys Glu Glu Leu Asp Lys Tyr Phe Lys Asn
1               5                   10

<210>  45
<211>  10
<212>  PRT
<213>  Severe acute respiratory syndrome coronavirus 1

<400>  45

Val Asn Ile Gln Lys Glu Ile Asp Arg Leu
1               5                   10

<210>  46
<211>  10
<212>  PRT
<213>  Severe acute respiratory syndrome coronavirus 1

<400>  46

Ile Asp Leu Gln Glu Leu Gly Lys Tyr Glu
1               5                   10


<210>  47
<211>  10
<212>  PRT
<213>  Severe acute respiratory syndrome coronavirus 1

<400>  47

Val Asn Ile Gln Lys Glu Ile Asp Arg Leu
1               5                   10


<210>  48
<211>  10
<212>  PRT
<213>  Severe acute respiratory syndrome coronavirus 1

<400>  48

Ile Asp Leu Gln Glu Leu Gly Lys Tyr Glu
1               5                   10


<210>  49
<211>  13
<212>  PRT
<213>  Severe acute respiratory syndrome coronavirus 1

<400>  49

Tyr Asp Glu Asn Gly Thr Ile Thr Asp Ala Val Asp Cys
1               5                   10


<210>  50
<211>  11
<212>  PRT
<213>  Severe acute respiratory syndrome coronavirus 1

<400>  50

Ser Ser Glu Val Ala Val Leu Tyr Gln Asp Val
1               5                   10


<210>  51
<211>  10
<212>  PRT
<213>  Severe acute respiratory syndrome coronavirus 1

<400>  51

Ile Ala Ile Pro Thr Asn Phe Ser Ile Ser

1                   5                   10


<210>  52
<211>  12
<212>  PRT
<213>  Severe acute respiratory syndrome coronavirus 1

<400>  52

Arg Ser Phe Ile Glu Asp Leu Leu Phe Asn Lys Val
1               5                   10


<210>  53
<211>  10
<212>  PRT
<213>  Severe acute respiratory syndrome coronavirus 1

<400>  53

Asn Gly Ile Gly Val Thr Gln Asn Val Leu
1               5                   10


<210>  54
<211>  16
<212>  PRT
<213>  Severe acute respiratory syndrome coronavirus 1

<400>  54

Val Val Asn Gln Asn Ala Gln Ala Leu Asn Thr Leu Val Lys Gln Leu
1               5                   10                  15


<210>  55
<211>  23
<212>  PRT
<213>  Severe acute respiratory syndrome coronavirus 1

<400>  55

Ile Asp Leu Gln Glu Leu Gly Lys Tyr Glu Ala Glu Val Gln Ile Asp
1               5                   10                  15


Arg Leu Ile Thr Gly Arg Leu
            20


<210>  56
<211>  14
<212>  PRT
<213>  Severe acute respiratory syndrome coronavirus 1

<400>  56

Tyr Asp Glu Asn Gly Thr Ile Thr Asp Ala Val Asp Cys Ser
1               5                   10


<210>  57

<211>  11
<212>  PRT
<213>  Severe acute respiratory syndrome coronavirus 1

<400>  57

Ile Ala Ile Pro Thr Asn Phe Ser Ile Ser Ile
1               5                   10


<210>  58
<211>  12
<212>  PRT
<213>  Severe acute respiratory syndrome coronavirus 1

<400>  58

Arg Ser Phe Ile Glu Asp Leu Leu Phe Asn Lys Val
1               5                   10


<210>  59
<211>  13
<212>  PRT
<213>  Severe acute respiratory syndrome coronavirus 1

<400>  59

Ser Thr Ala Leu Gly Lys Leu Gln Asp Val Val Asn Gln
1               5                   10


<210>  60
<211>  15
<212>  PRT
<213>  Severe acute respiratory syndrome coronavirus 1

<400>  60

Val Glu Ala Glu Val Gln Ile Asp Arg Leu Ile Thr Gly Arg Leu
1               5                   10                  15


<210>  61
<211>  10
<212>  PRT
<213>  Severe acute respiratory syndrome coronavirus 1

<400>  61

Ala Ala Pro His Gly Val Val Phe Leu His
1               5                   10


<210>  62
<211>  10
<212>  PRT
<213>  Severe acute respiratory syndrome coronavirus 1

<400>  62

Asn Leu Asn Glu Ser Leu Ile Asp Leu Gln
1               5                   10

## Description

The present invention relates to polypeptides comprising epitopes having a sequence homology to a sequence section of subunit S1 and/or subunit S2 of coronavirus spike protein from severe acute respiratory syndrome coronavirus 2 (SARS-CoV-2). Moreover, the invention refers to a vaccine comprising such polypeptide. The invention further relates to an antibody binding to subunit S1 and/or subunit S2 and to methods for isolating and detecting such antibody and to uses of the polypeptides and antibodies. The invention also relates to a polypeptide-based vaccine against coronaviruses that is prepared analogue to a classical vaccine against tuberculosis. Moreover, the invention refers to a hyperimmune composition obtained from immunized individual's donation blood or plasma as a source for specific antibodies against coronaviruses. The invention also refers to a vaccine against coronaviruses, which may comprise *Bacillus Calmette-Guérin* (BCG, a primary tuberculosis vaccination) which may be coated with one or more partly oxidized polysaccharides, which may be coated with one or more polypeptides of the present invention.

Coronaviruses have caused large-scale pandemics in the past decades, SARS and Middle East respiratory syndrome (MERS) (Drosten, C. et al.: Identification of a novel coronavirus in patients with severe acute respiratory syndrome. N. Engl. J. Med. 348, 1967-1976 (2003); Zaki, A. M. et al.: Isolation of a novel coronavirus from a man with pneumonia in Saudi Arabia. N. Engl. J. Med. 367, 1814-1820 (2012)).

It has generally been thought that severe acute respiratory syndrome coronavirus (1) (SARS-CoV(-1)), which is mainly found in bats, could cause a future disease outbreak (Cui, J., Li, F. & Shi, Z. L. Origin and evolution of pathogenic coronaviruses. Nat. Rev. Microbiol. 17, 181-192 (2019); Fan, Y., Zhao, K., Shi, Z.-L. & Zhou, P. Bat coronaviruses in China. Viruses 11, 210 (2019)).

According to a recent publication in Nature, the spread of coronavirus disease 2019 (COVID-19) is becoming unstoppable and has already reached the necessary epidemiological criteria for it to be declared a pandemic, having infected far more than 100,000 persons in 100 countries (Callaway E. Time to use the p-word? Coronavirus enters dangerous new phase. Nature 2020; 579:12). Therefore, a coordinated global response is desperately needed to prepare health systems to meet this unprecedented challenge. Countries that have been unfortunate enough to have been exposed to this disease already have, paradoxically, very valuable lessons to pass on (Remuzzi A et al.: COVID-19 and Italy: what next? The Lancet March 12th, 2020).

Patients had underlying health conditions and acute respiratory distress syndrome (ARDS) caused by severe acute respiratory syndrome coronavirus 2 (SARS-CoV-2) pneumonia needed respiratory support. Of the patients who died, 42.2% were aged 80-89 years, 32.4% were aged 70-79 years, 8.4% were aged 60-69 years, and 2.8% were aged 50-59 years (those aged >90 years made up 14.1%). The male to female ratio is 80% to 20% with an older median age for women (83.4 years for women vs 79.9 years for men) (Remuzzi A et al.: COVID-19 and Italy: what next? The Lancet March 12th, 2020).

Owing to a shortage of specific treatments and considering the relatedness of 2019-nCoV to SARS-CoV-1, some drugs and pre-clinical vaccines against SARS-CoV-1 could probably be used to treat SARS-CoV-2 as well. Finally, considering the wide spread of SARS-CoV (e.g., SARS-CoV-1 and SARS-CoV-2) in their natural reservoirs, future research should be focused on active surveillance of these viruses for broader geographical regions. In the long term, broad-spectrum antiviral drugs and vaccines should be prepared for emerging infectious diseases that are caused by this cluster of viruses in the future (Zhou P et al.: A pneumonia outbreak associated with a new coronavirus Nature 579 2020, 270).

Aggravating, the corona-viruses show a high variability during their evolution in humans. This could become a major threat for vaccinations with commonly used vaccination strategies. Finally, both an active and a passive vaccine should be present either for prophylaxis or treatment of the infection with corona-viruses.

An active vaccine should be designed in a way, that the high variability of the virus is captured presenting the existing natural variants as well as potential variations at the same time.

If a so designed vaccine is used for the immunization of potential patients of blood and plasma donors, the plasma drawn can be used also as a passive vaccine, covering the broad variability by a wide range of specific antibodies. Immunoglobulins like IgG and IgA have already been proven in their efficacy against a number of infections, either caused by bacteria or viruses.

The polypeptide composition according to DE-A 4141970 and DE-A 4122906, synthesized variable epitopes based on *Mycobacterium tuberculosis* bacterial surface allows the induction of a protective polyspecific immune response against a broad variety of epitopes, including viral epitopes. Due to the use of *M. tuberculosis,* a decisive part of the immune response may be induced specific T cell response. Especially this response is important for a successful fight against viruses.

Also, the induced antibodies, show such a high variability, so that variants of viruses can be neutralized. The unspecific immune stimulation and therefore also the increased defense against infectious diseases also against tumors is already described (Howard, J. G. et al.: Brit. J. Exp. Path. 40 281-190 (1959); Adolps, H. D. und Bastian, H. P.: Urologe A 25 51-55 (1986); Kubica, G. P. und Wayne, L. G.: The Mycobacteria Part B New York und Basel pp 863-902 (1984). The vaccine strains (BCG) (preferably in vital stage) are INH-sensible (INH = iso-nicotinic acid hydrazide), so that even an infection by the vaccine strain can be treated by the respective antibiotics. The divestation of the BCG using partially oxidized polysaccharides which bind viral, bacterial and parasitic pathogens was described by DE-A 4141970.

There is, however, still an unmet need for effective and efficient vaccines against SARS-CoV-2 viral proteins.

Surprisingly, it was found that epitopes that have a considerable sequence homology to SARS-CoV-2 as well as to other coronaviruses are potent epitopes to prevent COVID-19 and to induce antibody production. In particular those epitopes located in the subunit S1 and/or subunit S2 of coronavirus spike protein from SARS-CoV-2 and other coronaviruses are particularly beneficial.

In a first aspect, the present invention relates to a polypeptide or an immunogenic peptidomimetic or retro-inverso polypeptide thereof, wherein said polypeptide comprises (or consists of) at least one epitope of severe acute respiratory syndrome coronavirus 2 (SARS-CoV-2), whereby:
said epitope has a length of 8 to 100 consecutive amino acids;
said epitope has a sequence homology of at least 75% to a sequence section of subunit S1 and/or subunit S2 of coronavirus spike protein from SARS-CoV-2; and said epitope has a sequence homology of at least 75% to a sequence section of subunit S1 and/or subunit S2 of a coronavirus spike protein from at least one other type of coronavirus.

In a preferred embodiment, the epitope has a sequence homology of at least 80%, of at least 85%, of at least 90%, of at least 95%, of at least 97%, of at least 98%, of at least 99%, or sequence identity to a sequence section of subunit S1 and/or subunit S2 of coronavirus spike protein from SARS-CoV-2.

In a preferred embodiment, the epitope has a sequence homology of at least 80%, of at least 85%, of at least 90%, of at least 95%, of at least 97%, of at least 98%, of at least 99%, or sequence identity to a sequence section of subunit S1 and/or subunit S2 of a coronavirus spike protein from at least one other type of coronavirus.

In a preferred embodiment, the epitope has a sequence homology of at least 80%, of at least 85%, of at least 90%, of at least 95%, of at least 97%, of at least 98%, of at least 99%, or sequence identity to a sequence section of subunit S1 and/or subunit S2 of coronavirus spike protein from SARS-CoV-2 and
has a sequence homology of at least 80%, of at least 85%, of at least 90%, of at least 95%, of at least 97%, of at least 98%, of at least 99%, or sequence identity to a sequence section of subunit S1 and/or subunit S2 of a coronavirus spike protein from at least one other type of coronavirus.

In a preferred embodiment, the epitope has a sequence homology of at least 80%, to a sequence section of subunit S1 and/or subunit S2 of coronavirus spike protein from SARS-CoV-2 and
has a sequence homology of at least 80%, of at least 85%, of at least 90%, of at least 95%, of at least 97%, of at least 98%, of at least 99%, or sequence identity to a sequence section of subunit S1 and/or subunit S2 of a coronavirus spike protein from at least one other type of coronavirus.

In a preferred embodiment, the epitope has a sequence homology of at least 90%, to a sequence section of subunit S1 and/or subunit S2 of coronavirus spike protein from SARS-CoV-2 and
has a sequence homology of at least 80%, of at least 85%, of at least 90%, of at least 95%, of at least 97%, of at least 98%, of at least 99%, or sequence identity to a sequence section of subunit S1 and/or subunit S2 of a coronavirus spike protein from at least one other type of coronavirus.

In a preferred embodiment, the epitope has a sequence homology of at least 95%, to a sequence section of subunit S1 and/or subunit S2 of coronavirus spike protein from SARS-CoV-2 and
has a sequence homology of at least 80%, of at least 85%, of at least 90%, of at least 95%, of at least 97%, of at least 98%, of at least 99%, or sequence identity to a sequence section of subunit S1 and/or subunit S2 of a coronavirus spike protein from at least one other type of coronavirus.

In a preferred embodiment, the epitope has a sequence identity to a sequence section of subunit S1 and/or subunit S2 of coronavirus spike protein from SARS-CoV-2 and
has a sequence homology of at least 80%, of at least 85%, of at least 90%, of at least 95%, of at least 97%, of at least 98%, of at least 99%, or sequence identity to a sequence section of subunit S1 and/or subunit S2 of a coronavirus spike protein from at least one other type of coronavirus.

As used herein, the at least one other type of coronavirus may any type of coronavirus that is not SARS-CoV-2.

Accordingly, the term "at least one other type of coronavirus" may be understood interchangeably with "at least one type of coronavirus other than SARS-CoV-2" or "at least one type of coronavirus that is not SARS-CoV-2".

In a preferred embodiment, the at least one other type of coronavirus is selected from the group consisting of severe acute respiratory syndrome coronavirus 1 (SARS-CoV-1), Middle East respiratory syndrome coronavirus (MERS-CoV), human coronavirus NL63 (HCoV-NL63), human coronavirus OC43 (HCoV-OC43), human coronavirus HLU1 (HCoV-HKU1), human coronavirus 229E (HCoV-229E), and combinations of two or more thereof.

Such other types of coronavirus are described in the literature, such as in L, Annu. Rev. Virol. 2016, 3(1):237-261.

It will be understood that there may be the same or different homology to the corresponding sequence section of subunit S1 and/or subunit S2 of two different coronavirus spike proteins from two different types of coronaviruses when compared to the section of subunit S1 and/or subunit S2 of coronavirus spike protein from SARS-CoV-2.
The coronavirus spike protein may be a spike glycoprotein. Each coronavirus spike protein may be the unmodified polypeptide or an immunogenic peptidomimetic or retro-inverso polypeptide thereof or may be modified such as, e.g, glycosylated. As used in the context of the present invention, the common abbreviations for amino acid moieties, in particular in one letter code, are used. The coronavirus spike protein from SARS-CoV-1 in the context of the present invention is SARS-CoV-1 spike 1 protein. This protein preferably has a sequence homology of at least 95%, preferably a sequence homology of at least 98%, more preferably a sequence homology of at least 99%, in particular sequence identity to a polypeptide sequence of SEQ ID NO: 1:

MFIFLLFLTL TSGSDLDRCT TFDDVQAPNY TQHTSSMRGV YYPDEIFRSD TLYLTQDLFL
PFYSNVTGFH TINHTFGNPV IPFKDGIYFA ATEKSNVVRG WVFGSTMNNK SQSVIIINNS
TNVVIRACNF ELCDNPFFAV SKPMGTQTHT MIFDNAFNCT FEYISDAFSL DVSEKSGNFK
HLREFVFKNK DGFLYVYKGY QPIDVVRDLP SGFNTLKPIF KLPLGINITN FRAILTAFSP
AQDIWGTSAA AYFVGYLKPT TFMLKYDENG TITDAVDCSQ NPLAELKCSV KSFEIDKGIY
QTSNFRVVPS GDVVRFPNIT NLCPFGEVFN ATKFPSVYAW ERKKISNCVA DYSVLYNSTF
FSTFKCYGVS ATKLNDLCFS NVYADSFVVK GDDVRQIAPG QTGVIADYNY KLPDDFMGCV
LAWNTRNIDA TSTGNYNYKY RYLRHGKLRP FERDISNVPF SPDGKPCTPP ALNCYWPLND
YGFYTTTGIG YQPYRVVVLS FELLNAPATV CGPKLSTDLI KNQCVNFNFN GLTGTGVLTP
SSKRFQPFQQ FGRDVSDFTD SVRDPKTSEI LDISPCSFGG VSVITPGTNA SSEVAVLYQD
VNCTDVSTAI HADQLTPAWR IYSTGNNVFQ TQAGCLIGAE HVDTSYECDI PIGAGICASY
HTVSLLRSTS QKSIVAYTMS LGADSSIAYS NNTIAIPTNF SISITTEVMP VSMAKTSVDC
NMYICGDSTE CANLLLQYGS FCTQLNRALS GIAAEQDRNT REVFAQVKQM YKTPTLKYFG
GFNFSQILPD PLKPTKRSFI EDLLFNKVTL ADAGFMKQYG ECLGDINARD LICAQKFNGL
TVLPPLLTDD MIAAYTAALV SGTATAGWTF GAGAALQIPF AMQMAYRFNG IGVTQNVLYE
NQKQIANQFN KAISQIQESL TTTSTALGKL QDVVNQNAQA LNTLVKQLSS NFGAISSVLN
DILSRLDKVE AEVQIDRLIT GRLQSLQTYV TQQLIRAAEI RASANLAATK MSECVLGQSK
RVDFCGKGYH LMSFPQAAPH GVVFLHVTYV PSQERNFTTA PAICHEGKAY FPREGVFVFN
GTSWFITQRN FFSPQIITTD NTFVSGNCDV VIGIINNTVY DPLQPELDSF KEELDKYFKN
HTSPDVDLGD ISGINASVVN IQKEIDRLNE VAKNLNESLI DLQELGKYEQ YIKWPWYVWL
GFIAGLIAIV MVTILLCCMT SCCSCLKGAC SCGSCCKFDE DDSEPVLKGV

This protein is a spike protein (virion surface; position 14-1195).
As used herein, the subunit S1 of the SARS-CoV-1 preferably comprises or consists of a sequence having sequence homology of at least 95%, preferably a sequence homology of at least 98%, more preferably a sequence homology of at least 99%, in particular sequence identity to a polypeptide sequence of amino acid positions 14 to 657 of SEQ ID NO: 1.
As used herein, the subunit S2 of the SARS-CoV-1 preferably comprises or consists of a sequence having sequence homology of at least 95%, preferably a sequence homology of at least 98%, more preferably a sequence homology of at least 99%, in particular sequence identity to a polypeptide sequence of amino acid positions 668 to 1250 or 798 to 1250 of SEQ ID NO: 1.
The coronavirus spike protein from SARS-CoV-2 in the context of the present invention preferably has a sequence homology of at least 95%, preferably a sequence homology of at least 98%, more preferably a sequence homology of at least 99%, in particular sequence identity to a polypeptide sequence of SEQ ID NO: 2:

The coronavirus spike protein from MERS-CoV in the context of the present invention preferably has a sequence homology of at least 95%, preferably a sequence homology of at least 98%, more preferably a sequence homology of at least 99%, in particular sequence identity to a polypeptide sequence of SEQ ID NO: 3:

MIHSVFLLMF LLTPTESYVD VGPDSAKSAC IEVDIQQTFF DKTWPRPIDV SKADGIIYPQ
GRTYSNITIT YQGLFPYQGD HGDMYVYSAG HATGTTPQKL FVANYSQDVK QFANGFVVRI
GAAANSTGTV IISPSTSATI RKIYPAFMLG SSVGNFSDGK MGRFFNYTLV LLPDGCGTLL
RAFYCILEPR SGNYCPAGNS YTSFATYHTP ATDCSDGNYN RNASLNSFKE YFNLRNCTFM
YTYNITEDEI LEWFGITQTA QGVHLFSSRY VDLYGGNMFQ FATLPVYDTI KYYSIIPHSI
RSIQSDRKAW AAFYVYKLQP LTFLLDFSVD GYIRRAIDCG FNDLSQLHCS YESFDVESGV
YSVSSFEAKP SGSVVEQAEG VECDFSPLLS GTPPQVYNFK RLVFTNCNYN LTKLLSLFSV
NDFTCSQISP AAIASNCYSS LILDYFSYPL SMKSDLSVSS AGPISQFNYK QSFSNPTCLI
LATVPHNLTT ITKPFKYSYI NKCSRLLSDD RTEVPQLVNA NQYSPCVSIV PSTVWEDGDY
YRKQLSPLEG GGWLVASGST VAMTEQLQMG FGITVQYGTD TNSVCPKFEF ANDTKIASQL
GNCVEYSLYG VSGRGVFQNC TAVGVRQQRF VYDAYQNLVG YYSDDGNYYC LRACVSVPVS
VIYDKETKTH ATLFGSVACE HISSTMSQYS RSTRSMLKRR DSTYGPLQTP VGCVLGLVNS
SLFVEDCKLP LGQSLCALPD TPSTLTPRSV RSVPGEMRLA SIAFNHPIQV DQLNSSYFKL
SIPTNFSFGV TQEYIQTTIQ KVTVDCKQYV CNGFQKCEQL LREYGQFCSK INQALHGANL
RQDDSVRNLF ASVKSYQSSP IIPGFGGDFN LTLLEPVSIS TGSRSARSAI EDLLFDKVTI
ADPGYMQGYD DCMQQGPASA RDLICAQYVA GYKVLPPLMD VNMEAAYTSS LLGSIAGVGW
TAGLSSFAAI PFAQSIFYRL NGVGITQQVL SENQKLIANK FNQALGAMQT GFTTTNEAFQ
KVQDAVNNNA QALSKLASEL SNTFGAISAS IGDIIQRLDV LEQDAQIDRL INGRLTTLNA
FVAQQLVRSE SAALSAQLAK DKVNECVKAQ SKRSGFCGQG THIVSFVVNA PNGLYFMHVG
YYPSNHIEVV SAYGLCDLAN PTNCIAPVNG YFIKTNNTRI VDEWSYTGSS FYAPEPITSF
NTKYVAPQVT YQNISTNLPP PLLGNSTGID FQDELDEFFK NVSTSIPNFG SLTQINTTLL
DLTYEMLSLQ QVVKALNESY IDLKELGNYT YYNKWPWYIW LGFIAGLVAL ALCVFFILCC
TGCGTNCMGK LKCNRCCDRY EEYDLEPHKV

The coronavirus spike protein from HCoV-NL63 in the context of the present invention preferably has a sequence homology of at least 95%, preferably a sequence homology of at least 98%, more preferably a sequence homology of at least 99%, in particular sequence identity to a polypeptide sequence of SEQ ID NO: 4:

MKLFLILLVL PLASCFFTCN SNANLSMLQL GVPDNSSTIV TGLLPTHWFC ANQSTSVYSA
NGFFYIDVGN HRSAFALHTG YYDVNQYYIY VTNEIGLNAS VTLKICKFGI NTTFDFLSNS
SSSFDCIVNL LFTEQLGAPL GITISGETVR LHLYNVTRTF YVPAAYKLTK LSVKCYFNYS
CVFSVVNATV TVNVTTHNGR VVNYTVCDDC NGYTDNIFSV QQDGRIPNGF PFNNWFLLTN

GSTLVDGVSR LYQPLRLTCL WPVPGLKSST GFVYFNATGS DVNCNGYQHN SVADVMRYNL
NFSANSVDNL KSGVIVFKTL QYDVLFYCSN SSSGVLDTTI PFGPSSQPYY CFINSTINTT
HVSTFVGILP PTVREIVVAR TGQFYINGFK YFDLGFIEAV NFNVTTASAT DFWTVAFATF
VDVLVNVSAT KIQNLLYCDS PFEKLQCEHL QFGLQDGFYS ANFLDDNVLP ETYVALPIYY
QHTDINFTAT ASFGGSCYVC KPHQVNISLN GNTSVCVRTS HFSIRYIYNR VKSGSPGDSS
WHIYLKSGTC PFSFSKLNNF QKFKTICFST VEVPGSCNFP LEATWHYTSY TIVGALYVTW
SEGNSITGVP YPVSGIREFS NLVLNNCTKY NIYDYVGTGI IRSSNQSLAG GITYVSNSGN
LLGFKNVSTG NIFIVTPCNQ PDQVAVYQQS IIGAMTAVNE SRYGLQNLLQ LPNFYYVSNG
GNNCTTAVMT YSNFGICADG SLIPVRPRNS SDNGISAIIT ANLSIPSNWT TSVQVEYLQI
TSTPIVVDCA TYVCNGNPRC KNLLKQYTSA CKTIEDALRL SAHLETNDVS SMLTFDSNAF
SLANVTSFGD YNLSSVLPQR NIRSSRIAGR SALEDLLFSK VVTSGLGTVD VDYKSCTKGL
SIADLACAQY YNGIMVLPGV ADAERMAMYT GSLIGGMVLG GLTSAAAIPF SLALQARLNY
VALQTDVLQE NQKILAASFN KAINNIVASF SSVNDAITQT AEAIHTVTIA LNKIQDVVNQ
QGSALNHLTS QLRHNFQAIS NSIQAIYDRL DSIQADQQVD RLITGRLAAL NAFVSQVLNK
YTEVRSSRRL AQQKVNECVK SQSNRYGFCG NGTHIFSIVN SAPDGLLFLH TVLLPTDYKN
VKAWSGICVD GIYGYVLRQP NLVLYSDNGV FRVTSRIMFQ PRLPVLSDFV QIYNCNVTFV
NISRVELHTV IPDYVDVNKT LQEFAQNLPK YVKPNFDLTP FNLTYLNLSS ELKQLEAKTA
SLFQTTVELQ GLIDQINSTY VDLKLLNRFE NYIKWPWWVW LIISVVFVVL LSLLVFCCLS
TGCCGCCNCL TSSMRGCCDC GSTKLPYYEF

The coronavirus spike protein from HCoV-OC43 in the context of the present invention preferably has a sequence homology of at least 95%, preferably a sequence homology of at least 98%, more preferably a sequence homology of at least 99%, in particular sequence identity to a polypeptide sequence of SEQ ID NO: 5:

MFLILLISLP TAFAVIGDLN CPLDPKLKGS FNNRDTGSPS ISTDTVDVTN GLGTYYVLDR
VYLNTTLFLN GYYPTSGSTY RNMALKGTDL LSTLWFKPPF LSDFINGIFA KVKNTKVFKD
GVMYSEFPAI TIGSTFVNTS YSVVVQPRTI NSTQDGVNKL QGLLEVSVCQ YNMCEHPHTI
CHPNLGNHFK ELWHLDTGVV SCLYKRNFTY DVNATYLYFH FYQEGGTFYA YFTDTGFVTK
FLFNVYLGMA LSHYYVMPLT CISRRDIGFT LEYWVTPLTP RQYLLAFNQD GIIFNAVDCM
SDFMSEIKCK TQSIAPPTGV YELNGYTVQP IADVYRRKPD LPNCNIEAWL NDKSVPSPLN
WERKTFSNCN FNMSSLMSFI QADSFTCNNI DAAKIYGMCF SSITIDKFAI PNRRKVDLQL
GNLGYLQSSN YRIDTTATSC QLYYNLPAAN VSVSRFNPST WNKRFGFIED SVFVPQPTGV
FTNHSVVYAQ HCFKAPKNFC PCSSCPGKNN GIGTCPAGTN SLTCDNLCTL DPITFKAPDT
YKCPQTKSLV GIGEHCSGLA VKSDYCGNNS CTCQPQAFLG WSADSCLQGD KCNIFANFIL
HDVNNGLTCS TDLQKANTEI ELGVCVNYDL YGISGQGIFV EVNATYYNSW QNLLYDSNGN
LYGFRDYITN RTFMIHSCYS GRVSAAYHAN SSEPALLFRN IKCNYVFNNS LTRQLQPINY
SFDSYLGCVV NAYNSTAISV QTCDLTVGSG YCVDYSKNRR SRRAITTGYR FTNFEPFTVN
SVNDSLEPVG GLYEIQIPSE FTIGNMEEFI QTSSPKVTID CAAFVCGDYA ACKLQLVEYG
SFCDNINAIL TEVNELLDTT QLQVANSLMN GVTLSTKLKD GVNFNVDDIN FSPVLGCLGS

ECSKASSRSA IEDLLFDKVK LSDVGFVEAY NNCTGGAEIR DLICVQSYKG IKVLPPLLSE
NQISGYTLAA TSASLFPPWT AAAGVPFYLN VQYRINGLGV TMDVLSQNQK LIANAFNNAL
HAIQQGFDAT NSALVKIQAV VNANSEALNN LLQQLSNRFG AISASLQEIL SRLDALEAEA
QIDRLINGRL TALNAYVSQQ LSDSTLVKFS AAQAMEKVNE CVKSQSSRIN FCGNGNHIIS
LVQNAPYGLY FIHFNYVPTK YVTAKVSPGL CIAGNRGIAP KSGYFVNVNN TWMYTGSGYY
YPEPITENNV VVMSTCAVNY TKAPYVMLNT SIPNLPDFKE ELGQWFKNQT SVAPDLSLDY
INVTFLDLQV EMNRLQEAIK VLNHSYINLK DIGTYEYYVK WPWYVWLLIC LAGVAMLVLL
FFICCCTGCG TSCFKKCGGC CDDYTGYQEL

The coronavirus spike protein from HCoV-HKU1 in the context of the present invention preferably has a sequence homology of at least 95%, preferably a sequence homology of at least 98%, more preferably a sequence homology of at least 99%, in particular sequence identity to a polypeptide sequence of SEQ ID NO: 6:

MFLIIFILPT TLAVIGDFNC TNSFINDYNK TIPRISEDVV DVSLGLGTYY VLNRVYLNTT
LLFTGYFPKS GANFRDLALK GSKYLSTLWY KPPFLSDFNN GIFSKVKNTK LYVNNTLYSE
FSTIVIGSVF VNTSYTIVVQ PHNGILEITA CQYTMCEYPH TVCKSKGSIR NESWHIDSSE
PLCLFKKNFT YNVSADWLYF HFYQERGVFY AYYADVGMPT TFLFSLYLGT ILSHYYVMPL
TCKAISSNTD NETLEYWVTP LSRRQYLLNF DEHGVITNAV DCSSSFLSEI QCKTQSFAPN
TGVYDLSGFT VKPVATVYRR IPNLPDCDID NWLNNVSVPS PLNWERRIFS NCNFNLSTLL
RLVHVDSFSC NNLDKSKIFG SCFNSITVDK FAIPNRRRDD LQLGSSGFLQ SSNYKIDISS
SSCQLYYSLP LVNVTINNFN PSSWNRRYGF GSFNVSSYDV VYSDHCFSVN SDFCPCADPS
VVNSCVKSKP LSAICPAGTK YRHCDLDTTL YVNNWCRCSC LPDPISTYSP NTCPQKKVVV
GIGEHCPGLG INEEKCGTQL NHSSCSCSPD AFLGWSFDSC ISNNRCNIFS NFIFNGINSG
TTCSNDLLYS NTEVSTGVCV NYDLYGITGQ GIFKEVSAAY YNNWQNLLYD SNGNIIGFKD
FLTNKTYTIL PCYSGRVSAA FYQNSSSPAL LYRNLKCSYV LNNISFISQP FYFDSYLGCV
LNAVNLTSYS VSSCDLRMGS GFCIDYALPS SRRKRRGISS PYRFVTFEPF NVSFVNDSVE
TVGGLFEIQI PTNFTIAGHE EFIQTSSPKV TIDCSAFVCS NYAACHDLLS EYGTFCDNIN
SILNEVNDLL DITQLQVANA LMQGVTLSSN LNTNLHSDVD NIDFKSLLGC LGSQCGSSSR
SLLEDLLFNK VKLSDVGFVE AYNNCTGGSE IRDLLCVQSF NGIKVLPPIL SETQISGYTT
AATVAAMFPP WSAAAGVPFS LNVQYRINGL GVTMDVLNKN QKLIANAFNK ALLSIQNGFT
ATNSALAKIQ SVVNANAQAL NSLLQQLFNK FGAISSSLQE ILSRLDNLEA QVQIDRLING
RLTALNAYVS QQLSDITLIK AGASRAIEKV NECVKSQSPR INFCGNGNHI LSLVQNAPYG
LLFIHFSYKP TSFKTVLVSP GLCLSGDRGI APKQGYFIKQ NDSWMFTGSS YYYPEPISDK
NVVFMNSCSV NFTKAPFIYL NNSIPNLSDF EAEFSLWFKN HTSIAPNLTF NSHINATFLD
LYYEMNVIQE SIKSLNSSFI NLKEIGTYEM YVKWPWYIWL LIVILFIIFL MILFFICCCT
GCGSACFSKC HNCCDEYGGH NDFVIKASHD

As used herein, the subunit S1 of the HCoV-HKU1 preferably comprises or consists of a sequence having sequence homology of at least 95%, preferably a sequence homology of at least 98%, more preferably a sequence homology of at least 99%, in particular sequence identity to a polypeptide sequence of amino acid positions 1 to 752 of SEQ ID NO: 6.

As used herein, the subunit S2 of the HCoV-HKU1 preferably comprises or consists of a sequence having sequence homology of at least 95%, preferably a sequence homology of at least 98%, more preferably a sequence homology of at least 99%, in particular sequence identity to a polypeptide sequence of amino acid positions 753to 1350 or 901 to 1350 of SEQ ID NO: 1.

The coronavirus spike protein from HCoV-229E in the context of the present invention preferably has a sequence homology of at least 95%, preferably a sequence homology of at least 98%, more preferably a sequence homology of at least 99%, in particular sequence identity to a polypeptide sequence of SEQ ID NO: 7:

MFVLLVAYAL LHIAGCQTTN GLNTSYSVCN GCVGYSENVF AVESGGYIPS DFAFNNWFLL
TNTSSVVDGV VRSFQPLLLN CLWSVSGLRF TTGFVYFNGT GRGDCKGFSS DVLSDVIRYN
LNFEENLRRG TILFKTSYGV VVFYCTNNTL VSGDAHIPFG TVLGNFYCFV NTTIGNETTS
AFVGALPKTV REFVISRTGH FYINGYRYFT LGNVEAVNFN VTTAETTDFC TVALASYADV
LVNVSQTSIA NIIYCNSVIN RLRCDQLSFD VPDGFYSTSP IQSVELPVSI VSLPVYHKHT
FIVLYVDFKP QSGGGKCFNC YPAGVNITLA NFNETKGPLC VDTSHFTTKY VAVYANVGRW
SASINTGNCP FSFGKVNNFV KFGSVCFSLK DIPGGCAMPI VANWAYSKYY TIGSLYVSWS
DGDGITGVPQ PVEGVSSFMN VTLDKCTKYN IYDVSGVGVI RVSNDTFLNG ITYTSTSGNL
LGFKDVTKGT IYSITPCNPP DQLVVYQQAV VGAMLSENFT SYGFSNVVEL PKFFYASNGT
YNCTDAVLTY SSFGVCADGS IIAVQPRNVS YDSVSAIVTA NLSIPSNWTT SVQVEYLQIT
STPIVVDCST YVCNGNVRCV ELLKQYTSAC KTIEDALRNS ARLESADVSE MLTFDKKAFT
LANVSSFGDY NLSSVIPSLP TSGSRVAGRS AIEDILFSKL VTSGLGTVDA DYKKCTKGLS
IADLACAQYY NGIMVLPGVA DAERMAMYTG SLIGGIALGG LTSAVSIPFS LAIQARLNYV
ALQTDVLQEN QKILAASFNK AMTNIVDAFT GVNDAITQTS QALQTVATAL NKIQDVVNQQ
GNSLNHLTSQ LRQNFQAISS SIQAIYDRLD TIQADQQVDR LITGRLAALN VFVSHTLTKY
TEVRASRQLA QQKVNECVKS QSKRYGFCGN GTHIFSIVNA APEGLVFLHT VLLPTQYKDV
EAWSGLCVDG TNGYVLRQPN LALYKEGNYY RITSRIMFEP RIPTMADFVQ IENCNVTFVN
ISRSELQTIV PEYIDVNKTL QELSYKLPNY TVPDLVVEQY NQTILNLTSE ISTLENKSAE
LNYTVQKLQT LIDNINSTLV DLKWLNRVET YIKWPWWVWL CISVVLIFVV SMLLLCCCST
GCCGFFSCFA SSIRGCCEST KLPYYDVEKI HIQ

This protein is a spike protein (virion surface; position 16-1115).

As used herein, the subunit S1 of the HCoV-229E preferably comprises or consists of a sequence having sequence homology of at least 95%, preferably a sequence homology of at least 98%, more preferably a sequence homology of at least 99%, in particular sequence identity to a polypeptide sequence of amino acid positions 16 to 536 of SEQ ID NO: 7.

As used herein, the subunit S2 of the HCoV-229E preferably comprises or consists of a sequence having sequence homology of at least 95%, preferably a sequence homology of at least 98%, more preferably a sequence homology of at least 99%, in particular sequence identity to a polypeptide sequence of amino acid positions 537 to 1173 of SEQ ID NO: 7.

The polypeptides (i.e., the peptidic structures, peptidic) may be each independently from another be obtained by any means know for this purpose in the art. The polypeptide of the present invention may be obtained by any means known in the art. In a preferred embodiment, the polypeptide is obtained by a method comprising one or more procedural steps selected from the group consisting of polypeptide synthesis, gene technological means, and isolation of subunit S1 and/or subunit S2 from SARS-CoV-2 and subsequent protein digestion.

In one embodiment of the present invention, the polypeptide is obtained by solid phase peptide synthesis (SPPS) such as, e.g., of Fmoc- or Boc-based SPPS. Alternatively, the polypeptides may also be obtained by liquid phase peptide synthesis (LPPS) or, in the case of consisting of L-amino acid moieties.

In another embodiment of the present invention, the polypeptide is obtained by biotechnology means such as heterologous expression in a genetically modified hosts (e.g., organism excluding human bodies, tissue cultures or cells) such as, e.g., bacteria (e.g. *E. coli*), fungi (e.g., yeast), mammalian cells or mammalians excluding humans, insect cells or insects, plant cells or plants, etc. Accordingly, genetic manipulation of a host organism with the sequence comprising an epitope of the present invention being genetically fused to a gene encoding for the rest of the polypeptide may be used. Gene technological means includes, for instance, recombinant expression (e.g., heterologous expression and/or overexpression).

In a preferred embodiment, the epitope has a sequence homology of at least 50% to a sequence section located at the outer surface of subunit S1 and/or subunit S2 of the coronavirus spike protein from SARS-CoV-2.

In a preferred embodiment, the epitope has a sequence homology of at least 50% to a sequence section of subunit S1 and/or subunit S2 of at least one coronavirus spike protein from at least one other type of coronavirus located at the outer surface of the virus particle of said at least one other type of coronavirus.

In a preferred embodiment, the epitope is characterized in that it has a sequence homology of at least 50%, of at least 75%, of at least 80%, of at least 85%, of at least 90%, of at least 95%, of at least 97%, of at least 99% or identity to a sequence section of subunit S1 and/or subunit S2 of coronavirus spike protein from SARS-CoV-2 located at the outer surface of the SARS-CoV-2 virus particle.

In a preferred embodiment, the epitope is characterized in that it has a sequence homology of at least 50%, of at least 75%, of at least 80%, of at least 85%, of at least 90%, of at least 95%, of at least 97%, of at least 99% or identity to a sequence section of subunit S1 and/or subunit S2 of at least one coronavirus spike protein from at least one other type of coronavirus located at the outer surface of the virus particle of said at least one other type of coronavirus.

In a preferred embodiment, the epitope is characterized in that:
it has a sequence homology of at least 75% to a sequence section of subunit S1 and/or subunit S2 of coronavirus spike protein from SARS-CoV-2 located at the outer surface of the SARS-CoV-2 virus particle, and
it has a sequence homology of at least 75% to a sequence section of subunit S1 and/or subunit S2 of at least one coronavirus spike protein from at least one other type of coronavirus located at the outer surface of the virus particle of said at least one other type of coronavirus.

In a preferred embodiment, the epitope is characterized in that:
it has a sequence homology of at least 90% to a sequence section of subunit S1 and/or subunit S2 of coronavirus spike protein from SARS-CoV-2 located at the outer surface of the SARS-CoV-2 virus particle, and
it has a sequence homology of at least 75% to a sequence section of subunit S1 and/or subunit S2 of at least one coronavirus spike protein from at least one other type of coronavirus located at the outer surface of the virus particle of said at least one other type of coronavirus.

In a preferred embodiment, the epitope is characterized in that:
it has a sequence identity to a sequence section of subunit S1 and/or subunit S2 of coronavirus spike protein from SARS-CoV-2 located at the outer surface of the SARS-CoV-2 virus particle, and
it has a sequence homology of at least 75% to a sequence section of subunit S1 and/or subunit S2 of at least one coronavirus spike protein from at least one other type of coronavirus located at the outer surface of the virus particle of said at least one other type of coronavirus.

Optionally, variability may be included in a peptide sequence. This may be obtained by any means.

The variability in the peptide sequences may be obtained by one or more of the following:
1. Amino acid exchange: different amino acid precursors may be added during peptide synthesis. By using such method, two different peptide intermediates may be synthesized at the same time depending on the stochiometric ratios of the precursors. When the next amino acid precursor is added, the two already synthesized peptides may be prolonged and at this time, two different peptides are in the same synthesis product. Optionally, this step may be repeated several times so that finally a high variability can be achieved.
2. Amino acid deletion: For this approach, the peptide synthesis approach may be splitted. For the deletion part, no amino acid precursor is added, in the second part, the next precursor in the sequence may be added so that after the reunification of both approaches, two peptides are in one synthesis batch, one with and the second without the deletion. Such step may be repeated several times so that finally a high variability can be achieved.
3. Insertion of an amino acid: In this method, the same approach as described above for amino acid deletion may be used. The sample including the insertion may be supplemented with an additional precursor.

In a preferred embodiment, the epitope comprises (or consist of) a polypeptide sequence of at least eight consecutive amino acid moieties in length having a sequence homology of at least 25%, of at least of at least 50%, of at least 75%, of at least 80%, of at least 85%, of at least 90%, of at least 95%, of at least 97%, of at least 99% or identity to one or more of SEQ ID NOs: 1 to 7 or the epitope may be an immunogenic peptidomimetic or retro-inverso polypeptide thereof.

In a preferred embodiment, the epitope comprises (or consist of) a polypeptide sequence of at least eight consecutive amino acid moieties in length having a sequence homology of at least 25%, of at least of at least 50%, of at least 75%, of at least 80%, of at least 85%, of at least 90%, of at least 95%, of at least 97%, of at least 99% or identity to SEQ ID NO: 1 or the epitope may be an immunogenic peptidomimetic or retro-inverso polypeptide thereof.

In a preferred embodiment, the epitope comprises (or consist of) a polypeptide sequence of at least eight consecutive amino acid moieties in length having a sequence homology of at least 25%, of at least of at least 50%, of at least 75%, of at least 80%, of at least 85%, of at least 90%, of at least 95%, of at least 97%, of at least 99% or identity to SEQ ID NO: 2 or the epitope may be an immunogenic peptidomimetic or retro-inverso polypeptide thereof.

In a preferred embodiment, the epitope comprises (or consist of) a polypeptide sequence of at least eight consecutive amino acid moieties in length having a sequence homology of at least 25%, of at least of at least 50%, of at least 75%, of at least 80%, of at least 85%, of at least 90%, of at least 95%, of at least 97%, of at least 99% or identity to SEQ ID NO: 3 or the epitope may be an immunogenic peptidomimetic or retro-inverso polypeptide thereof.

In a preferred embodiment, the epitope comprises (or consist of) a polypeptide sequence of at least eight consecutive amino acid moieties in length having a sequence homology of at least 25%, of at least of at least 50%, of at least 75%, of at least 80%, of at least 85%, of at least 90%, of at least 95%, of at least 97%, of at least 99% or identity to SEQ ID NO: 4 or the epitope may be an immunogenic peptidomimetic or retro-inverso polypeptide thereof.

In a preferred embodiment, the epitope comprises (or consist of) a polypeptide sequence of at least eight consecutive amino acid moieties in length having a sequence homology of at least 25%, of at least of at least 50%, of at least 75%, of at least 80%, of at least 85%, of at least 90%, of at least 95%, of at least 97%, of at least 99% or identity to SEQ ID NO: 5 or the epitope may be an immunogenic peptidomimetic or retro-inverso polypeptide thereof.

In a preferred embodiment, the epitope comprises (or consist of) a polypeptide sequence of at least eight consecutive amino acid moieties in length having a sequence homology of at least 25%, of at least of at least 50%, of at least 75%, of at least 80%, of at least 85%, of at least 90%, of at least 95%, of at least 97%, of at least 99% or identity to SEQ ID NO: 6 or the epitope may be an immunogenic peptidomimetic or retro-inverso polypeptide thereof.
In a preferred embodiment, the epitope comprises (or consist of) a polypeptide sequence of at least eight consecutive amino acid moieties in length having a sequence homology of at least 25%, of at least of at least 50%, of at least 75%, of at least 80%, of at least 85%, of at least 90%, of at least 95%, of at least 97%, of at least 99% or identity to SEQ ID NO: 7 or the epitope may be an immunogenic peptidomimetic or retro-inverso polypeptide thereof.

In a preferred embodiment, the epitope comprises (or consist of) a polypeptide sequence of at least eight consecutive amino acid moieties in length having a sequence identity to one or more of SEQ ID NOs: 1 to 7 or the epitope may be an immunogenic peptidomimetic or retro-inverso polypeptide thereof.

In a preferred embodiment, the epitope comprises (or consist of) a polypeptide sequence of at least eight consecutive amino acid moieties in length of one or more of SEQ ID NOs: 1 to 7, wherein not more than two amino acid moieties have been deleted or replaced, in particular, if replaced, each has been replaced by an analogue amino acid moiety, or an immunogenic peptidomimetic or retro-inverso polypeptide thereof.

In a preferred embodiment, the epitope comprises (or consist of) a polypeptide sequence of at least eight consecutive amino acid moieties in length of one or more of SEQ ID NOs: 1 to 7, wherein (exactly) one amino acid moiety has been deleted or replaced, in particular has been replaced by an analogue amino acid moiety, or an immunogenic peptidomimetic or retro-inverso polypeptide thereof.

As used throughout the present invention, the term "replaced by an analogue amino acid moiety" may be understood in the broadest sense as being substituted by an amino acid moiety of similar physicochemical properties.

For example, "replaced by an analogue amino acid moiety" may have the meaning that a non-polar amino acid moiety may be substituted by another non-polar amino acid moiety, in particular an aliphatic non-polar amino acid moiety may be substituted by another aliphatic non-polar amino acid moiety and an aromatic non-polar amino acid moiety may be substituted by another aromatic non-polar amino acid moiety.

In this context, a non-polar amino acid moiety may be selected from the group consisting of G (Gly), A (Ala), V (Val), P (Pro), L (Leu), I (Ile), M (Met), W (Trp) and F (Phe). An aliphatic non-polar amino acid moiety may be selected from the group consisting of G (Gly), A (Ala), V (Val), P (Pro), L (Leu), I (Ile), and M (Met). An aromatic non-polar amino acid moiety may be selected from the group consisting of W (Trp) and F (Phe). In a preferred embodiment, in particular G (Gly), A (Ala), V (Val), L (Leu) or I (Ile) may be replaced by one another.

For example, "replaced by an analogue amino acid moiety" may also have the meaning that a polar (uncharged) amino acid moiety may be substituted by another polar (uncharged) amino acid moiety, in particular an aliphatic polar (uncharged) amino acid moiety may be substituted by another aliphatic polar (uncharged) amino acid moiety. In this context, a polar (uncharged) amino acid moiety may be selected from the group consisting of S (Ser), T (Thr), Y (Tyr), C (Cys), N (Asn), U (Sec, selenocysteinyl), O (Pyl, pyrrolysinyl) and Q (Gln). An aliphatic polar (uncharged) amino acid moiety may be selected from the group consisting of S (Ser), T (Thr), C (Cys), N (Asn), U (Sec), O (Pyl) and Q (Gln). In a preferred embodiment, in particular S (Ser) may be replaced by T (Thr) and vice versa and Q (Gln) may be replaced by N (Asn) and vice versa.

For example, "replaced by an analogue amino acid moiety" may also have the meaning that a basic amino acid moiety may be substituted by another basic amino acid moiety. In this context, a basic amino acid moiety may be selected from the group consisting of K (Lys), R (Arg) and H (His). In a preferred embodiment in particular K (Lys) may be replaced by R (Arg) and vice versa.

For example, "replaced by an analogue amino acid moiety" may also have the meaning that an acidic amino acid moiety may be substituted by another acidic amino acid moiety. In this context, an acidic amino acid moiety may be selected from the group consisting of D (Asp) and E (Glu).

For example, "replaced by an analogue amino acid moiety" may also have the meaning that a polar amino acid moiety including interactions of the opposite charge may be substituted comparable amino acid moieties. Such amino acid moieties which are exchangeable by one another may be selected from the group consisting of S (Ser), T (Thr), Y (Tyr), C (Cys), N (Asn), Q (Gln), K (Lys), R (Arg), H (His), U (Sec), O (Pyl), D (Asp) and E (Glu).

For example, "replaced by an analogue amino acid moiety" may also have the meaning that a small-sized amino acid moiety may be replaced by another small-sized amino acid moiety. In this context, a small-sized amino acid moiety may be selected from the group consisting of A (Ala), G (Gly) and S (Ser).

For example, "replaced by an analogue amino acid moiety" may also have the meaning that an at least partly polar amino acid moiety may be substituted by another at least partly polar amino acid moiety, in particular an aliphatic at least partly polar amino acid moiety may be substituted by another aliphatic at least partly polar amino acid moiety and an aromatic at least partly polar amino acid moiety may be substituted by another aromatic at least partly polar amino acid moiety. In this context, an at least partly polar amino acid moiety may be selected from the group consisting of Y (Tyr), G (Gly), A (Ala), V (Val), P (Pro), L (Leu), I (Ile), M (Met), W (Trp) and F (Phe). An aliphatic at least partly polar amino acid moiety may be selected from the group consisting of G (Gly), A (Ala), V (Val), P (Pro), L (Leu), I (Ile) and M (Met). An aromatic at least partly polar amino acid moiety may be selected from the group consisting of Y (Tyr), W (Trp) and F (Phe).

In a preferred embodiment, the epitope comprises (or consist of) a polypeptide sequence of at least eight consecutive amino acid moieties in length of one or both of SEQ ID NO: 1 and/or 2, wherein (exactly) two amino acid moieties have been deleted or replaced, in particular have been replaced by analogue amino acid moieties, or an immunogenic peptidomimetic or retro-inverso polypeptide thereof.

In a preferred embodiment, the polypeptide of the present invention or immunogenic peptidomimetic or retro-inverso thereof is between 8 and 100, between 9 and 90, between 10 and 80, between 11 and 70, between 18 and 50, between 9 and 40, between 8 and 20, between 12 and 25, between 13 and 20, between 14 and 18 consecutive amino acid moieties (or analogues thereof) in length.

In a preferred embodiment, the epitope comprises (or consist of) a polypeptide sequence having a sequence homology of at least 50%, at least 85%, at least 90%, at least 95%, or at least 98%, or identity to one or more of SEQ ID NOs: 1 to 7. In a preferred embodiment, the epitope is between 8 and 100, between 8 and 62, between 8 and 50, between 8 and 40, between 8 and 30, between 8 and 20, between 8 and 17, between 8 and 11, consecutive amino acids in length.

For example, an epitope is 8, 9, 10, 11, 12, 13, 14, 15, 16, 17, 18, 19 or 20 consecutive amino acids in length.

In a preferred embodiment, the polypeptide is between 8 and 100, between 8 and 62, between 8 and 50, between 8 and 40, between 8 and 30, between 8 and 20, between 8 and 17, between 8 and 11, consecutive amino acids in length. For example, an epitope is 8, 9, 10, 11, 12, 13, 14, 15, 16, 17, 18, 19 or 20 consecutive amino acids in length.
In a preferred embodiment, the polypeptide of the present invention or an immunogenic peptidomimetic or retro-inverso polypeptide thereof consists of one or more epitopes of the present invention.

In a preferred embodiment, the epitope has a sequence homology of at least 80%, at least 85%, at least 90%, at least 95%, at least 98%, at least 99%, or sequence identity to a sequence section of subunit S1 of coronavirus spike protein from SARS-CoV-2.

In a preferred embodiment, the epitope has a sequence homology of at least 80%, at least 85%, at least 90%, at least 95%, at least 98%, at least 99%, or sequence identity to a sequence section of subunit S2 of coronavirus spike protein from SARS-CoV-2.
In a preferred embodiment, the epitope has a sequence homology of at least 80%, at least 85%, at least 90%, at least 95%, at least 98%, at least 99%, or sequence identity to a sequence section of both subunit S1 and subunit S2 of coronavirus spike protein from SARS-CoV-2.

The epitope sequence may be any sequence that has a sequence homology of at least 75% to a sequence section of subunit S1 and/or subunit S2 of coronavirus spike protein from SARS-CoV-2; and has a sequence homology of at least 75% to a sequence section of subunit S1 and/or subunit S2 of a coronavirus spike protein from at least one other type of coronavirus.

Specific sequences are identified by means of BLAST analysis that are particularly beneficial in the context of the present invention.

BLAST analyses were performed to identify overlapping sequence sections between sections of the coronavirus spike proteins:
(A) comparison of SARS-CoV-2 with SARS-CoV-1 (amino acid position range 26-1213, resulting in score: 1906 bits (4938), expect: 0.0, method: compositional matrix adjust., identities: 906/1190 (76%), positives: 1038/1190 (87%), gaps: 24/1190 (2%));
(B) comparison of SARS-CoV-2 with MERS-CoV (amino acid position range 311-1296 resulting in score: 499 bits (1284), expect: 2e-158, method: compositional matrix adjust., identities: 344/1001 (34%), positives: 514/1001 (51%), 70/1001 (6%); and
   amino acid position range: 189-274 resulting in score: 20.4 bits (41), expect: 5.1, method: compositional matrix adjust., identities: 21/88 (24%), positives: 33/88 (37%),19/88 (6%));
(C) comparison of SARS-CoV-2 with HCoV-NL63 (amino acid position range 577-1296, resulting in score: 301 bits (770), expect: 1e-87, method: compositional matrix adjust., identities: 231/775 (30%), positives: 356/775 (45%), gaps: 108/775 (13%);
   amino acid position range 1110-1137, resulting in score: 20.8 bits (42), expect: 4.0, method: compositional matrix adjust., identities: 9/28 (32%), positives: 13/28 (46%), gaps: 0/28 (0%); and
   amino acid position range 254-274, resulting in score: 20.0 bits (40), expect: 6.7, method: compositional matrix adjust., identities: 2/23 (39%), positives: 12/23 (52%), gaps: 2/23 (8%));
(D) comparison of SARS-CoV-2 with HCoV-OC43 (amino acid position range 615-1302, resulting in score: 427 bits (1097), expect: 7e-132, method: compositional matrix adjust., identities: 257/705 (36%), positives: 378/705 (53%), gaps: 40/705 (5%);
   amino acid position range 118-445, resulting in score: 107 bits (267), , expect: 1e-26, method: compositional matrix adjust., identities: 94/357 (26%), positives: 159/357 (44%), gaps: 44/357 (12%); and
   amino acid position range 165-185, resulting in score: 20.4 bits (41), expect: 5.6, method: compositional matrix adjust., identities: 7/21 (33%), positives: 10/21 (47%), gaps: 0/21 (0%));
(E) comparison of SARS-CoV-2 with HCoV-HKU1 (amino acid position range 600-1295, resulting in score: 422 bits (1086), expect: 2e-130, method:
   compositional matrix adjust., identities: 257/719 (36%), positives: 388/719 (53%), gaps: 54/719 (7%);
   amino acid position range 125-481, resulting in score: 108 bits (270), expect: 7e-27, method: compositional matrix adjust., identities: 96/379 (25%), positives: 163/379 (43%), gaps: 30/379 (7%); and
   amino acid position range 1125-1150, resulting in score: 20.8 bits (42), expect: 3.7, method: compositional matrix adjust., identities: 8/26 (31%), positives: 13/26 (50%), gaps: 0/26 (0%)); and
(F) comparison of SARS-CoV-2 with HCoV-229E (amino acid position range 557 to 1100, resulting in score: 320 bits (819), expect: 3e-95, method:
   compositional matrix adjust., identities: 190/551 (34%), positives: 289/551 (52%), gaps: 46/551 (8%);
   amino acid position range 246 to 269, resulting in score: 21.2 bits (43), expect: 2.6, method: compositional matrix adjust., identities: 9/24 (38%), positives: 13/24 (54%), gaps: 0/24 (0%);
   amino acid position range 255-278, resulting in score: 20.8 bits (42), expect: 3.9, method: compositional matrix adjust., identities: 8/24(33%), positives: 14/24 (58%), gaps: 0/24 (0%);
   amino acid position range 61-81, resulting in score: 20.4 bits(41), expect: 4.5, method: compositional matrix adjust., identities: 10/23 (43%), positives: 12/23 (52%), gaps: 2/23 (8%);
   amino acid position range 275-365, resulting in score: 20.4 bits(41), expect: 4.7, method: compositional matrix adjust., identities: 22/91 (24%), positives: 35/91 (38%), gaps: 2/91(2%)).

The following sequences were identified as being particularly beneficial due to their sequence homology between coronavirus spike proteins of SARS-CoV-2 and of one or more other coronaviruses:
The following matching epitopes of SARS-Cov-2 and SARS-Cov-1 of SEQ ID NOs: 8 to 19 were identified:

SEQ ID NO: 8:    RGVYYPDEIFRSDTLYLTQDLFLPFYSNVT
Consensus:       RGVYYPD++FRS  L+ TQDLFLPF+SNVT

SEQ ID NO: 9:    DGIYFAATEKSNVVRGWVFGSTMNNKSQSVIIINNSTNVVIR
Consensus:       DG+YFA+TEKSN++RGW+FG+T+++K+QS++I+NN+TNVVI+

SEQ ID NO: 10:  NAFNCTFEYIS
Consensus:      +A NCTFEY+S
Homology to HCoV-OC43

SEQ ID NO: 11:  GNFKHLREFVFKN
Consensus:      GNFK+LREFVFKN
Homology to SARS-CoV and SARS-CoV2

SEQ ID NO: 12:
AAAYFVGYLKPTTFMLKYDENGTITDAVDCSQNPLAELKCSVKSFEIDKGIYQTSNFRVVPS
Consensus:
AAAY+VGYL+P TF+LKY+ENGTITDAVDC+ +PL+E KC++KSF ++KGIYQTSNFRV P+
Homology to HCoV-HKU1

SEQ ID NO: 13:  VVRFPNITNLCPFGEVFNATKFPSVYAWERKKISNCVADYSVLYNS
Consensus:      +VRFPNITNLCPFGEVFNAT+F SVYAW RK+ISNCVADYSVLYNS
Homology to MERS-CoV

SEQ ID NO: 14:
FSTFKCYGVSATKLNDLCFSNVYADSFVVKGDDVRQIAPGQTGVIADYNYKLPDDFMGCVLAWNTR
NIDA
Consensus:
FSTFKCYGVS TKLNDLCF+NVYADSFV++GD+VRQIAPGQTG IADYNYKLPDDF GCV+AWN+
N+D+

SEQ ID NO: 15:  LRPFERDIS
Consensus:      L+PFERDIS
Homology to MERS-CoV

SEQ ID NO: 16:
GIGYQPYRVVVLSFELLNAPATVCGPKLSTDLIKNQCVNFNFNGLTGTGVLTPSSKRFQPFQQFGR
DVSDFTDSVRDPKTSEILDISPCSFGGVSVITPGTNASSEVAVLYQDVNCTDV
Consensus:
G+GYQPYRVVVLSFELL+APATVCGPK ST+L+KN+CVNFNFNGLTGTGVLT S+K+F PFQQFGR
D++D TD+VRDP+T EILDI+PCSFGGVSVITPGTN S++VAVLYQDVNCT+V

SEQ ID NO: 17:
AIHADQLTPAWRIYSTGNNVFQTQAGCLIGAEHVDTSYECDIPIGAGICASYHT
Consensus:
AIHADQLTP WR+YSTG+NVFQT+AGCLIGAEHV+ SYECDIPIGAGICASY T
Partial homology to HCoV-OC43

SEQ ID NO: 18:
RSTSQKSIVAYTMSLGADSSIAYSNNTIAIPTNFSISITTEVMPVSMAKTSVDCNMYICGDSTECA
NLLLQYGSFCTQLNRALSGIAAEQDRNTREVFAQVKQMYKTPTLKYFGGFNFSQILPDPLKPTKRS
FIEDLLFNKVTLADAGFMKQYGECLGDINARDLICAQKFNGLTVLPPLLTDDMIAAYTAALVSGTA
TAGWTFGAGAALQIPFAMQMAYRFNGIGVTQNVLYENQKQIANQFNKAI
Consensus:
RS + +SI+AYTMSLGA++S+AYSNN+IAIPTNF+IS+TTE++PVSM KTSVDC MYICGDSTEC+
NLLLQYGSFCTQLNRAL+GIA EQD+NT+EVFAQVKQ+YKTP +K FGGFNFSQILPDP KP+KRS
FIEDLLFNKVTLADAGF+KQYG+CLGDI ARDLICAQKFNGLTVLPPLLTD+MIA YT+AL++GT
T+GWTFGAGAALQIPFAMQMAYRFNGIGVTQNVLYENQK IANQFN AI
Homology to MERS-CoV and HCoV-HKU1

SEQ ID NO: 19:
QIQESLTTTSTALGKLQDVVNQNAQALNTLVKQLSSNFGAISSVLNDILSRLDKVEAEVQIDRLIT
GRLQSLQTYVTQQLIRAAEIRASANLAATKMSECVLGQSKRVDFCGKGYHLMSFPQAAPHGVVFLH
VTYVPSQERNFTTAPAICHEGKAYFPREGVFVFNGTSWFITQRNFFSPQIITTDNTFVSGNCDVVI
GIINNTVYDPLQPELDSFKEELDKYFKNHTSPDVDLGDISGINASVVNIQKEIDRLNEVAKNLNES
LIDLQELGKYEQYIKWP
Consensus:
+IQ+SL++T++ALGKLQDVVNQNAQALNTLVKQLSSNFGAISSVLNDILSRLDKVEAEVQIDRLIT
GRLQSLQTYVTQQLIRAAEIRASANLAATKMSECVLGQSKRVDFCGKGYHLMSFPQ+APHGVVFLH
VTYVP+QE+NFTTAPAICH+GKA+FPREGVFV NGT WF+TQRNF+ PQIITTDNTFVSGNCDVVI
GI+NNTVYDPLQPELDSFKEELDKYFKNHTSPDVDLGDISGINASVVNIQKEIDRLNEVAKNLNES
LIDLQELGKYEQYIKWP
Homology to HCoV-NL63, HCoV-OC43 and HCoV-229E

The following matching epitopes of MERS-CoV and SARS-Cov-1 of SEQ ID NOs: 20 to 31 were identified:

SEQ ID NO: 20:  KSFEIDKGIY
Consensus:      +SF+++ G+Y
Homology to SARS-Cov-2, HCoV-OC43, HCoV-HKU1, HCoV-229E

SEQ ID NO: 21:  QYGSFCTQLNRAL
Consensus:      +YG FC+++N+AL
Homology to SARS-CoV-2, MERS-CoV, HCoV-NL63, HCoV-HKU1

SEQ ID NO: 22:  IEDLLFNKVTLAD
Consensus:      IEDLLF+KVT+AD

Homology to SARS-Cov-2, HCoV-NL63, HCoV-OC43, HCoV-HKU1, HCoV-229E

SEQ ID NO: 23:  NARDLICAQ
Consensus:      +ARDLICAQ
Homology to SARS-CoV-2, MERS-CoV, HCoV-NL63, HCoV-OC43, HCoV-
HKU1, HCoV-229E

SEQ ID NO: 24:  DMIAAYTAALV
Consensus:      +M AAYT++L+
Homology to SARS-CoV-2, MERS-CoV, HCoV-NL63, HCoV-HKU1

SEQ ID NO: 25:  YRFNGIGVTQNVLYENQKQIANQFNKAI
Consensus:      YR NG+G+TQ VL ENQK IAN+FN+A+
Homology to MERS-CoV, HCoV-NL63

SEQ ID NO: 26:  QLSSNFGAISS
Consensus:      +LS+ FGAIS+
Homology to MERS-CoV, HCoV-NL63, HCoV-OC43, HCoV-229E

SEQ ID NO: 27:  QIDRLITGRL
Consensus:      QIDRLI GRL
Homology to MERS-CoV, HCoV-NL63, HCoV-OC43, HCoV-HKU1, HCoV-229E

SEQ ID NO: 28:  FCGKGYHLMSF
Consensus:      FCG+G H++SF
Homology to SARS-CoV-2, HCoV-NL63, HCoV-HKU1

SEQ ID NO: 29:  APHGVVFLHV
Consensus:      AP+G+ F+HV
Homology to MERS-CoV, HCoV-OC43, HCoV-HKU1, HCoV-229E

SEQ ID NO: 30:  FKEELDKYFKN
Consensus:      F++ELD++FKN
Homology to SARS-CoV-2, MERS-CoV, HCoV-NL63

SEQ ID NO: 31:  LNESLIDLQELG
Consensus:      LNES IDL+ELG
Homology to SARS-CoV-2, MERS-CoV, HCoV-OC43, HCoV-HKU1

The following matching epitopes of HCoV-NL63 and SARS-Cov-1 of SEQ ID NOs: 32 to 36 were identified:

SEQ ID NO: 32:  IAIPTNFSISI
Consensus:      ++IP+N++ S+
Homology to SARS-CoV-2, MERS-CoV, HCoV-NL63, HCoV-OC43, HCoV-HKU1,
HCoV229E

SEQ ID NO: 33:  RSFIEDLLFNKV
Consensus:      RS +EDLLF+KV
Homology to SARS-CoV-2, MERS-CoV, HCoV-NL63, HCoV-OC43, HCoV-HKU1

SEQ ID NO: 34:  ALGKLQDVVNQ
Consensus:      AL K+QDVVNQ
Homology to SARS-CoV-2, HCoV-NL63, HCoV-OC43, HCoV-HKU1, HCoV-229E

SEQ ID NO: 35:  VEAEVQIDRLITGRL
Consensus:      ++A+ Q+DRLITGRL
Homology to SARS-CoV-2, MERS-CoV, HCoV-NL63, HCoV-HKU1, HCoV-229E

SEQ ID NO: 36:  AAPHGVVFLH
Consensus:      +AP G++FLH
Homology to SARS-CoV-2, MERS-CoV, HCoV-OC43, HCoV-HKU1, HCoV-229E

The following matching epitopes of HCoV-OC43 and SARS-Cov-1 of SEQ ID NOs: 37 to 49 were identified:

SEQ ID NO: 37:  SSEVAVLYQDV
Consensus:      SSE A+L++++
Homology to SARS-CoV-2, MERS-CoV, HCoV-OC43, HCoV-HKU1

SEQ ID NO: 38:  RSFIEDLLFNKVTLAD
Consensus:      RS IEDLLF+KV L+D
Homology to SARS-CoV-2, HCoV-NL63, HCoV-OC43, HCoV-HKU1

SEQ ID NO: 39:  VLPPLLTDDMIA
Consensus:      VLPPLL+++ I+
Homology to MERS-CoV, HCoV-NL63, HCoV-229E

SEQ ID NO: 40:  NGIGVTQNVL
Consensus:      NG+GVT +VL

Homology to SARS-CoV-2, MERS-CoV, HCoV-NL63, HCoV-OC43, HCoV-HKU1,
HCoV-229E

SEQ ID NO: 41:  TSTALGKLQDVVNQNAQALNTLVKQLSSNFGAISS
Consensus:      T++AL K+Q VVN N++ALN L++QLS+ FGAIS+
Homology to SARS-CoV-2, HCoV-OC43, HCoV-HKU1, HCoV-229E

SEQ ID NO: 42:  DILSRLDKVEA
Consensus:      +ILSRLD +EA
Homology to MERS-CoV, HCoV-NL63, HCoV-OC43

SEQ ID NO: 43:  QIDRLITGRL
Consensus:      QIDRLI GRL
Homology to SARS-CoV-2, MERS-CoV, HCoV-NL63, HCoV-OC43, HCoV-HKU1,
HCoV-229E

SEQ ID NO: 44:  FKEELDKYFKN
Consensus:      FKEEL ++FKN
Homology to SARS-CoV-2, MERS-CoV, HCoV-NL63, HCoV-OC43

SEQ ID NO: 45:  VNIQKEIDRL
Consensus:      +++Q E++RL
Homology to SARS-CoV-2, MERS-CoV, HCoV-NL63, HCoV-OC43, HCoV-HKU1

SEQ ID NO: 46:  IDLQELGKYE
Consensus:      I+L+++G YE
Homology to SARS-CoV-2, MERS-CoV, HCoV-NL63, HCoV-OC43, HCoV-HKU1

SEQ ID NO: 47:  VNIQKEIDRL
Consensus:      +N+  E+ +L
Homology to SARS-CoV-2, MERS-CoV, HCoV-OC43, HCoV-HKU1

SEQ ID NO: 48:  IDLQELGKYE
Consensus:      I+L+++G YE
Homology to SARS-CoV-2, MERS-CoV, HCoV-NL63, HCoV-OC43, HCoV-HKU1

SEQ ID NO: 49:  YDENGTITDAVDC
Consensus:      ++++G I +AVDC
Homology to SARS-CoV-2, MERS-CoV, HCoV-OC43, HCoV-HKU1

The following matching epitopes of HCoV-HKU1 and SARS-Cov-1 of SEQ ID NOs: 50 to 56 were identified:

SEQ ID NO: 50:  SSEVAVLYQDV
Consensus:      SS  A+LY+++
Homology to SARS-CoV-2, MERS-CoV, HCoV-OC43, HCoV-HKU1

SEQ ID NO: 51:  IAIPTNFSIS
Consensus:      I IPTNF+I+
Homology to SARS-CoV-2, MERS-CoV, HCoV-NL63, HCoV-OC43, HCoV-HKU1,
HCoV-229E

SEQ ID NO: 52:  RSFIEDLLFNKV
Consensus:      RS +EDLLFNKV
Homology to SARS-CoV-2, MERS-CoV, HCoV-NL63, HCoV-OC43, HCoV-HKU1

SEQ ID NO: 53:  NGIGVTQNVL
Consensus:      NG+GVT +VL
Homology to SARS-CoV-2, MERS-CoV, HCoV-NL63, HCoV-OC43, HCoV-HKU1,
HCoV-229E

SEQ ID NO: 54:  VVNQNAQALNTLVKQL
Consensus:      VVN NAQALN+L++QL
Homology to SARS-CoV-2, MERS-CoV, HCoV-NL63, HCoV-OC43, HCoV-HKU1,
HCoV-229E

SEQ ID NO: 55:  IDLQELGKYEAEVQIDRLITGRL
Consensus:      +ILSRLD +EA+VQIDRLI GRL
Homology to SARS-CoV-2, MERS-CoV, HCoV-NL63, HCoV-OC43, HCoV-HKU1

SEQ ID NO: 56:  YDENGTITDAVDCS
Consensus:      +DE+G IT+AVDCS
Homology to SARS-CoV-2, MERS-CoV, HCoV-OC43, HCoV-HKU1

The following matching epitopes of HCoV-HKU1 and SARS-Cov-1 of SEQ ID NOs: 57 to 62 were identified:

SEQ ID NO: 57:  IAIPTNFSISI
Consensus:      ++IP+N++ S+
Homology to SARS-CoV-2, MERS-CoV, HCoV-NL63, HCoV-OC43, HCoV-HKU1,
HCoV-229E

SEQ ID NO: 58:  RSFIEDLLFNKV
Consensus:      RS IED+LF+K+
Homology to SARS-CoV-2, MERS-CoV, HCoV-NL63, HCoV-OC43, HCoV-HKU1,
HCoV-229E

SEQ ID NO: 59:  STALGKLQDVVNQ
Consensus:      +TAL K+QDVVNQ
Homology to SARS-CoV-2, HCoV-OC43, HCoV-HKU1, HCoV-229E

SEQ ID NO: 60:  VEAEVQIDRLITGRL
Consensus:      ++A+ Q+DRLITGRL
Homology to SARS-CoV-2, MERS-CoV, HCoV-NL63, HCoV-HKU1, HCoV-229E

SEQ ID NO: 61:  AAPHGVVFLH
Consensus:      AAP G+VFLH
Homology to SARS-CoV-2, MERS-CoV, HCoV-OC43, HCoV-HKU1, HCoV-229E

SEQ ID NO: 62:  NLNESLIDLQ
Consensus:      N+N +L+DL+
Homology to SARS-CoV-2, MERS-CoV, HCoV-OC43, HCoV-HKU1

In a preferred embodiment, the epitope comprises or consists of an amino acid sequence that has a sequence homology of at least 75% to at least one of the sequences selected from the group consisting of sequences of SEQ ID NOs: 8 to 62 or in an immunogenic peptidomimetic or retro-inverso polypeptide thereof.

In a preferred embodiment, the epitope comprises or consists of an amino acid sequence that has a sequence homology of at least 80%, of at least 85%, of at least 90%, of at least 95%, of at least 97%, of at least 98%, or of at least 99 to at least one of the sequences selected from the group consisting of sequences of SEQ ID NOs: 8 to 62 or in an immunogenic peptidomimetic or retro-inverso polypeptide thereof.

In a preferred embodiment, the epitope comprises or consists of an amino acid sequence selected from the group consisting of sequences of SEQ ID NOs: 8 to 62 or in an immunogenic peptidomimetic or retro-inverso polypeptide thereof.

In other words, the present invention also relates to a polypeptide or an immunogenic peptidomimetic or retro-inverso polypeptide thereof, wherein said polypeptide comprises (or consists of) at least one epitope of severe acute respiratory syndrome coronavirus 2 (SARS-CoV-2), characterized in that the epitope comprises or consists of an amino acid sequence that has a sequence homology of at least 75%, of at least 80%, of at least 85%, of at least 90%, of at least 95%, of at least 97%, of at least 98%, of at least 99, or identity to at least one of the sequences selected from the group consisting of sequences of SEQ ID NOs: 8 to 62 or in an immunogenic peptidomimetic or retro-inverso polypeptide thereof.

In a preferred embodiment, the epitope comprises or consists of an amino acid sequence that has a sequence homology of at least 80%, of at least 85%, of at least 90%, of at least 95%, of at least 97%, of at least 98%, or of at least 99 to at least one of the sequences selected from the group consisting of sequences of SEQ ID NOs: 8 to 19 or in an immunogenic peptidomimetic or retro-inverso polypeptide thereof.

In another preferred embodiment, the epitope comprises or consists of an amino acid sequence that has a sequence homology of at least 80%, of at least 85%, of at least 90%, of at least 95%, of at least 97%, of at least 98%, or of at least 99 to at least one of the sequences selected from the group consisting of sequences of SEQ ID NOs: 20 to 31 or in an immunogenic peptidomimetic or retro-inverso polypeptide thereof.

In another preferred embodiment, the epitope comprises or consists of an amino acid sequence that has a sequence homology of at least 80%, of at least 85%, of at least 90%, of at least 95%, of at least 97%, of at least 98%, or of at least 99 to at least one of the sequences selected from the group consisting of sequences of SEQ ID NOs: 32 to 36 or in an immunogenic peptidomimetic or retro-inverso polypeptide thereof.

In another preferred embodiment, the epitope comprises or consists of an amino acid sequence that has a sequence homology of at least 80%, of at least 85%, of at least 90%, of at least 95%, of at least 97%, of at least 98%, or of at least 99 to at least one of the sequences selected from the group consisting of sequences of SEQ ID NOs: 37 to 49 or in an immunogenic peptidomimetic or retro-inverso polypeptide thereof.

In another preferred embodiment, the epitope comprises or consists of an amino acid sequence that has a sequence homology of at least 80%, of at least 85%, of at least 90%, of at least 95%, of at least 97%, of at least 98%, or of at least 99 to at least one of the sequences selected from the group consisting of sequences of SEQ ID NOs: 50 to 56 or in an immunogenic peptidomimetic or retro-inverso polypeptide thereof.

In another preferred embodiment, the epitope comprises or consists of an amino acid sequence that has a sequence homology of at least 80%, of at least 85%, of at least 90%, of at least 95%, of at least 97%, of at least 98%, or of at least 99 to at least one of the sequences selected from the group consisting of sequences of SEQ ID NOs: 57 to 62 or in an immunogenic peptidomimetic or retro-inverso polypeptide thereof.

In a preferred embodiment, the epitope comprises (or consist of) a polypeptide sequence of at least eight consecutive amino acid moieties in length of any of SEQ ID NOs: 1 to 62 or an immunogenic peptidomimetic or retro-inverso polypeptide thereof. In a further preferred embodiment, the epitope comprises (or consist of) a polypeptide sequence of at least nine, at least ten, at least eleven, at least twelve, at least 13, or at least 14 consecutive amino acid moieties in length of any of SEQ ID NOs: 1 to 62 or an immunogenic peptidomimetic or retro-inverso polypeptide thereof.

In a further preferred embodiment, the epitope comprises (or consist of) a polypeptide sequence of at least eight consecutive amino acid moieties in length of any of SEQ ID NOs: 1 to 62, wherein not more than two amino acid moieties have been deleted or replaced in comparison to the sequence having the largest homology of SEQ ID NOs: 1 to 7 in particular, if replaced, each has been replaced by an analogue amino acid moiety, or an immunogenic peptidomimetic or retro-inverso polypeptide thereof.

In a further preferred embodiment, the epitope comprises (or consist of) a polypeptide sequence of at least eight consecutive amino acid moieties in length of any of SEQ ID NOs: 8 to 62, wherein not more than two amino acid moieties have been deleted or replaced in comparison to the sequence having the largest homology of SEQ ID NO: 1 and wherein not more than two amino acid moieties have been deleted or replaced in comparison to the sequence having the largest homology of SEQ ID NO: 2 to 7, in particular, if replaced, each has been replaced by an analogue amino acid moiety, or an immunogenic peptidomimetic or retro-inverso polypeptide thereof.

In a further preferred embodiment, the epitope comprises (or consist of) a polypeptide sequence of at least eight consecutive amino acid moieties in length of any of SEQ ID NO: 8 to 62, wherein (exactly) one amino acid moiety has been deleted or replaced in comparison to the sequence having the largest homology of SEQ ID NOs: 1 to 7, in particular, if replaced, each has been replaced by an analogue amino acid moiety, or an immunogenic peptidomimetic or retro-inverso polypeptide thereof.

In a further preferred embodiment, the epitope comprises (or consist of) a polypeptide sequence of at least eight consecutive amino acid moieties in length of any of SEQ ID NOs: 8 to 62, wherein the sequence has a sequence section of SEQ ID NO: 1 and wherein not more than two amino acid moieties have been deleted or replaced in comparison to the sequence having the largest homology of SEQ ID NOs: 2 to 7, in particular, if replaced, each have been replaced by an analogue amino acid moiety, or an immunogenic peptidomimetic or retro-inverso polypeptide thereof.

In a further preferred embodiment, the epitope comprises (or consist of) a polypeptide sequence of at least eight consecutive amino acid moieties in length of any of SEQ ID NOs: 8 to 62, wherein the sequence has a sequence section of SEQ ID NO: 2 and wherein not more than two amino acid moieties have been deleted or replaced in comparison to the sequence having the largest homology of SEQ ID NOs: 1 and 3 to 7, in particular, if replaced, each have been replaced by an analogue amino acid moiety, or an immunogenic peptidomimetic or retro-inverso polypeptide thereof.

In a further preferred embodiment, the epitope comprises (or consist of) a polypeptide sequence of at least eight consecutive amino acid moieties in length of any of SEQ ID NOs: 8 to 62, wherein (exactly) two amino acid moieties have been deleted or replaced in comparison to the sequence having the largest homology of SEQ ID NOs: 1 to 7, in particular, if replaced, each has been replaced by an analogue amino acid moiety, or an immunogenic peptidomimetic or retro-inverso polypeptide thereof.

In a further preferred embodiment, the epitope comprises (or consist of) a polypeptide sequence of at least eight consecutive amino acid moieties in length of any of SEQ ID NOs: 8 to 62, wherein no, one or two amino acid moieties have been depleted of replaced, in particular by a homologue amino acid moiety, or an immunogenic peptidomimetic or retro-inverso polypeptide thereof.

In a further preferred embodiment, the epitope comprises (or consist of) a polypeptide sequence of at least eight consecutive amino acid moieties in length having a sequence homology of at least 55% to a sequence to a sequence selected from the group consisting of:
The polypeptide of the present invention may be immobilized on a solid support by any means. It may be directly conjugated to the surface or may be conjugated via any linker.

In a preferred embodiment, the solid support is a solid phase of an affinity column removing at least parts of the unbound fluid also includes the removal of unbound antibodies and antibody fragments having no or low binding affinity to the polypeptide of the present invention including the at least one epitope.

The polypeptide of the present invention may be used for various purposes and in different contexts. The polypeptide of the present invention may be used as a vaccine.

Accordingly, a further aspect of the present invention relates to a vaccine comprising (or consisting of) at least one polypeptide of the present invention and at least one pharmaceutically acceptable carrier.

It will be understood that all definitions are preferred embodiments as indicated above *mutatis mutandis* also apply to the vaccine.

As used herein, the terms "pharmaceutically acceptable carrier", "pharmaceutically acceptable excipient", "carrier" and "excipient" may be understood interchangeably in the broadest sense as any substance that may support the pharmacological acceptance of the vaccine.

Preferably, such vaccine may be prepared and/or used analogously to that described in DE-A 4141970, in particular that described in the context of tuberculosis (e.g., directed against *Mycobacterium tuberculosis*) in this document.

In a preferred embodiment, the vaccine of the present invention comprises one or more excipients that enhance immune response.

In a preferred embodiment, the vaccine of the present invention comprises one or more excipients that enhance (also: boost or intensity) the generation of antibodies (humoral immune response) and/or T-cell-mediated immune response. In a preferred embodiment, the vaccine of the present invention comprises one or more excipients that enhance (also: boost or intensity) the generation of antibodies (humoral immune response) and T-cell-mediated immune response.

In a preferred embodiment, the vaccine comprises one or more inactivated, attenuated and/or vital bacteria, and/or bacterial fragments. These may enhance immune response. BCG was also found to generally enhance immune response, optionally including T-cell-mediated immune response and/or the generation of antibodies (humoral immune response).

Humoral immune response, i.e., the binding of antibodies to virus particles (virions) may prevent or impede the entrance of the virus (e.g., a coronavirus such as SAR-CoV-2) into their target cells. T-cells may attack infected cells and, thereby, prevent or impede generation of further virus particles (virions).

In a preferred embodiment, the vaccine comprises *Bacillus Calmette-Guérin* (BCG). BCG was originally used for tuberculosis vaccination, but was found to generally enhance immune response. BCG was also found to generally enhance T-cell-mediated immune response and the generation of antibodies (humoral immune response). In a preferred embodiment, the BCG is coated with one or more partly oxidized polysaccharides. In a preferred embodiment, the BCG is coated with one or more polypeptides of the present invention. In a preferred embodiment, the BCG is coated with one or more partly oxidized polysaccharides. and with one or more polypeptides of the present invention. For example, in this context, a partly oxidized polysaccharide may be partly oxidized dextrane.

Alternatively or additionally, a partly oxidized polysaccharide may be or contain Lactose, amylose, amylopectin, glycogen, pectin, glucomannan, chitosan, lipopolysaccharide, one or more other polysaccharides, or a combination of two or more thereof. The vaccine may optionally be prepared as described in DE-A 4141970, in that instead of or in addition to HIV or Schistosoma epitopes the epitope-containing polypeptides of the present invention are used for coating.

In a preferred embodiment, at least parts of the one or more polypeptides of the present invention comprised in the vaccine are coated on BCG. In a preferred embodiment, essentially the whole content of the one or more polypeptide of the present invention comprised in the vaccine is coated on BCG.

In a preferred embodiment, vaccine prepared for final administration enables routes of administration which circumvent the first pass effect. More preferably, the vaccine is prepared to be suitable for administration by injection into the patient (e.g., suitable for administration routes selected from the group consisting of intravenous (i.v.), intraarterial (i.a.), intraperitoneal (i.p.), intramuscular (i.m.), and subcutaneous (s.c.) injection). Alternatively, or additionally, the vaccine may also be suitable for other routes of administration such as, e.g., nasal or trans- or intradermal administration.

The vaccine ready to use preferably is a liquid formulation, in particular an injection portion. The storage form may also be liquid, but may also be a dried form (e.g. a powder such as a powder comprising dried or freeze-dried one or more polypeptides of the present invention) or may be a paste or syrup or the like. Optionally, a dried form, paste or syrup may be dissolved or emulsified prior to being administered to the patient.

A pharmaceutically acceptable carrier may exemplarily be selected from the list consisting of an aqueous buffer, saline, water, dimethyl sulfoxide (DMSO), ethanol, vegetable oil, paraffin oil or combinations of two or more thereof. Furthermore, the pharmaceutically acceptable carrier may optionally contain one or more detergent(s), one or more foaming agent(s) (e.g., sodium lauryl sulfate (SLS), sodium doceyl sulfate (SDS)), one or more coloring agent(s) (e.g., food coloring), one or more vitamin(s), one or more salt(s) (e.g., sodium, potassium, calcium, zinc salts), one or more humectant(s) (e.g., sorbitol, glycerol, mannitol, propylenglycol, polydextrose), one or more enzyme(s), one or more preserving agent(s) (e.g., benzoic acid, methylparaben, one or more antioxidant(s), one or more herbal and plant extract(s), one or more stabilizing agent(s), one or more chelating agents (e.g., ethylenediaminetetraacetic acid (EDTA), and/or one or more uptake mediator(s) (e.g., polyethylene imine (PEI), a cell-penetrating peptide (CPP), a protein transduction domain (PTD), an antimicrobial peptide, etc.).

In a preferred embodiment, the vaccine is apyrogenic. The vaccine may be provided in a ampoule.

The present invention also relates to a dosage unit of the vaccine of the present invention. Exemplarily, the present invention may refer to a single dose container or to a multiple dosage form.

The vaccine may contain one or more than one polypeptides of the present invention. The vaccine may contain one or more than one epitopes of the present invention.

Due to the homology of the epitopes, the vaccine may also bind to different types of coronaviruses. Then, it may be designated as "multi-corona vaccine". Alternatively, it may be a vaccine directed to a specific type of coronaviruses such as SARS-CoV-2.

The vaccine may be prepared by any means.

In one embodiment, one or more polypeptides of the present invention is dissolved or suspended in a pharmaceutically acceptable carrier. Optionally, one or more excipients may be added.

In another embodiment, *Bacillus Calmette-Guérin* (BCG) (optionally be coated with one or more partially oxidized polysaccharides as exemplified above) is coated with one or more polypeptides of the present invention. This may be obtained by means as described in DE-A 4141970.

The partial oxidation of the polysaccharide may be achieved by using a suitable oxidizing agent, such as periodic acid. The oxidation degree may be in any range such as, e.g., in the range of 1 to 50%, or of 5 to 40%, or of 7 to 30%, or of 10 to 20%, such as, e.g, 12% (based on aldehyde groups per monosaccharide moieties units). Oxidized polysaccharide may be purified by precipitation with ethanol and may be dried.

*Bacille de Calmette Guérin* (BCG) may be any BCG such as, e.g., Jena-S-4, which is also usable for the non-specific immunization of tumor patients. BCG may be optionally be coated with a solution of partly oxidized polysaccharide (e.g., at pH 7 to10, or at pH 7.5 to 9). Such solution of partly oxidized polysaccharide may contain 0.5 to 10% (w/v), or 1 to 5% (w/v), or 1.5 to 2.5% (w/v), or 2% (w/v), of one or more partly oxidized polysaccharides. Also contain one or more mono- or disaccharides may be additionally or alternatively used for coating.

The obtained suspension or solution may be further diluted in a pharmaceutically acceptable carrier, which is preferably liquid.

The vaccine may be used either as therapeutic or prophylactic vaccine. Due to the fact, that this vaccine may be prepared analogously the *Mycobacterium tuberculosis* vaccine taught in DE-A 4141970, the activation of T cells which are beneficial for the immune response against viruses may be achieved.

This vaccination can be also used for the immunization of blood and plasma donors to achieve an (hyper)immune plasma. Products derived from such a plasma may be suitable for the passive immunization in e.g. severe corona cases and can be therefore life-saving.

Derivatives of those hyperimmune plasma products may be used for therapeutic and prophylactic uses. Especially in prophylactic setting, the immunoglobulin (especially IgA) based products shall be applied directly on the mucosal surface by dropping or spraying or inhalation.

A still further aspect of the present invention refers to the vaccine of the present invention for use in a method for preventing an individual from developing a coronavirus infection.

It will be understood that all definitions are preferred embodiments as indicated above *mutatis mutandis* also apply to the vaccine for use.

In other words, the present invention also relates to a method for preventing an individual from developing a coronavirus infection in a patient, wherein said patient is administered with a sufficient amount of the vaccine of the present invention.

Accordingly, a sill further aspect of the present invention relates to the use of a vaccine of the present invention for vaccination.

In a preferred embodiment, the present invention refers to the vaccine of the present invention for use in a method for preventing an individual from developing a SARS-CoV-2 infection.

As used in the context of the present invention, the term "patient" may be understood in the broadest sense as any living being, which is preferably any animal, more preferably a mammal including human, in particular a human being.

Preferably, administration of the vaccine is systemic administration (e.g., intravenously (i.v.), intraarterially (i.a.), intraperitoneally (i.p.), intramuscularly (i.m.), subcutaneously (s.c.), transdermal, nasally). Alternatively, administration may also be local administration (e.g., intrathecally or intravitreally). Preferably, administration is systemic administration, in particular intravenous injection.

It will be understood that the vaccine may trigger an immune response, in particular a humoral immune response, i.e., the generation of antibodies.

Preferably, administration of the hyperimmune IgG is systemic administration (e.g., intravenously (i.v.), intraarterially (i.a.), intraperitoneally (i.p.), intramuscularly (i.m.), subcutaneously (s.c.), transdermal, nasally). Alternatively, administration may also be local administration (e.g., intrathecally or intravitreally, intranasal e.g. by inhalation or by dropping or spray). Preferably, administration is systemic administration, in particular intravenous injection.

It will be understood that the vaccine may trigger an immune response, in particular a humoral immune response, i.e., the generation of antibodies.

Preferably, administration of the hyperimmune IgA is systemic administration (e.g., intravenously (i.v.), intraperitoneally (i.p.), intramuscularly (i.m.), subcutaneously (s.c.), transdermal, nasally). Alternatively, administration may also be local administration (e.g., intrathecally or intravitreally, intranasal e.g. by inhalation or by dropping or spray). Preferably, administration is the local administration.

It will be understood that the vaccine may trigger an immune response, in particular a humoral immune response, i.e., the generation of antibodies.

Accordingly, a still further aspect of the present invention relates to an antibody or antibody fragment binding to subunit S1 of coronavirus spike protein from SARS-CoV-2 with a dissociation constant Kd of less than 100 nM and/or to subunit S2 of coronavirus spike protein from SARS-CoV-2 with a dissociation constant Kd of less than 100 nM.

It will be understood that all definitions are preferred embodiments as indicated above *mutatis mutandis* also apply to the antibody or antibody fragment.

An antibody may be an antibody of any antibody class such as, e.g., IgG, IgA, IgD, IgM or IgE. An antibody fragment is preferably a fragment antigen-binding (Fab). An antibody or antibody fragment may also be an antibody mimetic such as, e.g., a designed ankyrin repeat protein (DARPin), an affibody, an affilins, an affimer, an affitins, an alphabodies, an anticalins, an avimerm, a fynomer, a Kunitz domain peptide, a monobody or a nanoCLAMP.

In a preferred embodiment, the antibody or antibody fragment binds to an epitope as defined herein with a dissociation constant Kd of less than 100 nM. In a preferred embodiment, the antibody or antibody fragment binds to an epitope as defined herein with a dissociation constant Kd of less than 50 nM, less than 25 nM or less than 10 nM.

In a preferred embodiment, the antibody or antibody fragment binds to subunit S1 of coronavirus spike protein from SARS-CoV-2 with a dissociation constant Kd of less than 100 nM, of less than 50 nM, of less than 25 nM, or of less than 10 nM.

In a preferred embodiment, the antibody or antibody fragment binds to subunit S2 of coronavirus spike protein from SARS-CoV-2 with a dissociation constant Kd of less than 100 nM, of less than 50 nM, of less than 25 nM, or of less than 10 nM.

In a preferred embodiment, the antibody or antibody fragment binds to subunit S1 of coronavirus spike protein from SARS-CoV-2 with a dissociation constant Kd of less than 100 nM, of less than 50 nM, of less than 25 nM, or of less than 10 nM, and
binds to subunit S2 of coronavirus spike protein from SARS-CoV-2 with a dissociation constant Kd of less than 100 nM, of less than 50 nM, of less than 25 nM, or of less than 10 nM.

In a preferred embodiment, the antibody or antibody fragment binds to an epitope according to the present invention with a dissociation constant Kd of less than 100 nM.

In a preferred embodiment, the antibody or antibody fragment is a neutralizing antibody or antibody fragment which may decrease enzymatic activity of its target (i.e, subunit S1 and/or subunit S2 of coronavirus spike protein from SARS-CoV-2) and/or inhibiting its capability to bind onto the receptors.

In a preferred embodiment, antibody or antibody fragment of the present invention is isolated out of general antibody pools (e.g., IgA pools) using positive affinity chromatography. The ligands on the affinity resin may be immunogenic epitopes of the subunit S1 and/or subunit S2 of coronavirus spike protein from SARS-CoV-2, which may allow the binding of specific antibodies or antibody fragments (e.g., IgAs).

One or more antibodies and/or antibody fragments of the present invention may also be comprised in a composition in high concentration (e.g., a hyperimmune composition). The present invention refers to a hyperimmune composition obtained from immunized individual's donation blood or plasma as a source for specific antibodies against coronaviruses.

The antibody or antibody fragment of the present invention may be used for any purpose, including *in vivo* and *in vitro* uses.

Such antibody or antibody fragment may also be isolated from any fluid, in particular from body fluids such as blood or fractions thereof. This may be performed for preparative or analytical purposes. Further, such antibody or antibody fragment may also be detected in any fluid, in particular in body fluids such as blood or fractions thereof. This may be performed for diagnostic or scientific purposes.

A further aspect of the present invention refers to a method for isolating and/or detecting an antibody or antibody fragment binding to subunit S1 and/or subunit S2 of coronavirus spike protein from SARS-CoV-2, in particular an antibody or antibody fragment of the present invention, from a fluid containing the antibody or antibody fragment, wherein said method comprises the following steps:
(i) providing:
   the fluid containing the antibody or antibody fragment, and
   a polypeptide immobilized on a solid support according to the present invention;
(ii) contacting the fluid with the immobilized polypeptide and allowing the antibody or antibody fragment to bind to the immobilized polypeptide; and
(iii) removing at least parts of the unbound fluid and optionally washing the solid support with a fluid not containing the containing the antibody or antibody fragment.

It will be understood that all definitions are preferred embodiments as indicated above *mutatis mutandis* also apply to the method. This method preferably is an *in vitro* method.

In a preferred embodiment, the solid support is a solid phase of an affinity column and said method preferably further comprises a step of eluting the antibody or antibody fragment from the affinity column.

In a preferred embodiment, the antibody or antibody fragment binding to subunit S1 and/or subunit S2 of coronavirus spike protein from SARS-CoV-2 is an antibody or antibody fragment of the present invention. Therefore, in a preferred embodiment, the method is a method for isolating and/or detecting an antibody or antibody fragment of the present invention.

In a preferred embodiment, the fluid is a body fluid, preferably a body fluid selected from the group consisting of blood plasma and a fraction of blood plasma, in particular wherein said method further comprises preparing of a fraction of blood plasma by means of a Cohn or Kistler-Nitschmann process.

In a further preferred embodiment, the fluid is a supernatant or extracellular liquid from a cell culture such as, e.g., from a cell line expressing such antibody or antibody fragment. For example, such cell culture may be a hybridoma cell culture.

The polypeptide of the present invention may be immobilized on a solid support by any means. It may be directly conjugated to the surface or may be conjugated via any linker. In a preferred embodiment, the solid support is a solid phase of an affinity column and said method further comprises a step of eluting the antibody or antibody fragment from the affinity column.

In a preferred embodiment, the solid support is a solid phase of an affinity column removing at least parts of the unbound fluid also includes the removal of unbound antibodies and antibody fragments having no or low binding affinity to the polypeptide of the present invention including the at least one epitope.

The present invention also deals with the purification of immunoglobulins with varying degrees of affinity, including immunoglobulins with neutralizing capabilities, against subunit S1 and/or subunit S2 of coronavirus spike protein from SARS-CoV-2 and their affiliated proteins including their proteins and subtypes.

In a preferred embodiment, the present invention relates to a method for isolating an antibody or antibody fragment binding to an epitope of at least one of subunit S1 and/or subunit S2 of coronavirus spike protein from SARS-CoV-2, in particular an antibody or antibody fragment independently from the immunoglobulin class of the present invention, from a body fluid, wherein said method comprises the following consecutive steps:
(a-i) providing:
   the body fluid, and
   a polypeptide according to any of the present invention or a mixture of polypeptides of the present invention immobilized on the solid phase of an affinity column;
(a-ii) contacting the body fluid with the immobilized polypeptide;
(a-iii) eluting the unbound body fluid and optionally washing the solid support by a flow-through of a buffer through the column; and
(a-iv) eluting the bound antibody or antibody fragment from the affinity column.

In another preferred embodiment, the present invention relates to a method for detecting an antibody or antibody fragment binding to an epitope of at least one of subunit S1 and/or subunit S2 of coronavirus spike protein from SARS-CoV-2, in particular an antibody or antibody fragment of the present invention, in a body fluid, wherein said method comprises the following consecutive steps:
(b-i) providing:
   the body fluid, and
   a polypeptide of the present invention immobilized on a solid support;
(b-ii) contacting the body fluid with the immobilized polypeptide;
(b-iii) removing the unbound body fluid and optionally washing the solid support; and
(b-iv) detecting the bound antibody or antibody fragment.

In a preferred embodiment, the method is performed by means of affinity chromatography and said method comprises:
(i-c) providing:
   blood plasma or a fraction thereof, and
   a polypeptide or a mixture of the present invention immobilized on the solid phase of an affinity column;
(ii-c) contacting the blood plasma with the immobilized polypeptide;
(iii-c) eluting the unbound blood plasma or a fraction thereof and optionally washing the solid support by a flow-through of a buffer through the column; and
(iv-c) eluting the bound antibody or antibody fragment of the present invention from the affinity column and detecting said antibody.

In a preferred embodiment, the method is performed by means of an enzyme-linked immunosorbent assay (ELISA) and said method comprises:
(i-d) providing:
   blood plasma or a fraction thereof, and
   a polypeptide or a mixture of polypeptides of the present invention immobilized on a solid phase;
(ii-d) contacting the blood plasma with the immobilized polypeptide;
(iii-d) removing the unbound blood plasma or a fraction thereof and optionally washing the solid support with a buffer; and
(iv-d) detecting the bound antibody or antibody fragment with a secondary antibody selectively to the Fc part of the antibody or antibody fragment of the present invention and detecting said secondary antibody, wherein said secondary antibody is labeled with a detectable label or is conjugated to an enzyme that generates a detectable compound from a precursor.

In a preferred embodiment, the method of the present invention further comprises a step of detecting the bound antibody or antibody fragment.

In a preferred embodiment, the step of detecting the bound antibody or antibody fragment comprises the following steps.
(a) binding a secondary antibody selectively to the Fc part of the bound antibody or antibody fragment; and
(b) detecting said secondary antibody.

In a preferred embodiment, the method further comprises isolating or removing one or more antibody classes selected from the group consisting of IgG, IgM, IgD, IgE and IgA.

In a preferred embodiment, the method further comprises detecting the bound antibody or antibody fragment. In a preferred embodiment, the method further comprises detecting the bound antibody or antibody fragment, wherein said step of detecting the bound antibody or antibody fragment comprises the following steps:
(a) binding a secondary antibody selectively to the Fc part of the bound antibody or antibody fragment; and
(b) detecting said secondary antibody, preferably wherein said secondary antibody is labeled with a detectable label or is conjugated to an enzyme that generates a detectable compound from a precursor.

In a preferred embodiment, the method further comprises:
isolating or removing one or more antibody classes selected from the group consisting of IgG, IgM, IgD, IgE and IgA; and
detecting the bound antibody or antibody fragment, preferably wherein said step of detecting the bound antibody or antibody fragment comprises the following steps:
   (a) binding a secondary antibody selectively to the Fc part of the bound antibody or antibody fragment; and
   (b) detecting said secondary antibody, preferably wherein said secondary antibody is labeled with a detectable label or is conjugated to an enzyme that generates a detectable compound from a precursor.

In a preferred embodiment, the similarity between the both epitopes of subunits S1 S2 of coronavirus spike protein from SARS-CoV-2 allows the use of a single epitope for an affinity purification of specific neutralizing antibodies.

The antibody or antibody fragment obtained or obtainable from a method of the present invention may be monoclonal or polyclonal. This may also depend on the fluid used as source. In a preferred embodiment, the antibody or antibody fragment of the present invention is monoclonal. In another preferred embodiment, the antibody or antibody fragment of the present invention is polyclonal.

In a preferred embodiment, the method of the present invention further comprises a step of isolating or removing one or more antibody classes selected from the group consisting of IgG, IgM, IgD, IgE and IgA.

The immunoglobulin may be IgA, where IgA may be monomeric, dimeric, polymeric, contains an additional J-chain, recombinant, comprises a secretory component, is administered orally as tablet or capsule or via inhalation. The immunoglobulin may be IgM, where IgM may be monomeric, dimeric, polymeric, contains an additional J-chain, recombinant, comprises a secretory component, is administered orally as tablet or capsule or via inhalation. The immunoglobulin may be IgG, where IgG may be monomeric, dimeric, polymeric, contains an additional J-chain, recombinant, comprises a secretory component, is administered orally as tablet or capsule or via inhalation.

The immunoglobulin may be a hyper-immune antibody that may be directly isolated from plasma and/or all its derivatives, fractions and waste fractions during plasma fractionation.

In a preferred embodiment, the secondary antibody is labeled with a detectable label or is conjugated to an enzyme that generates a detectable compound from a precursor.

For instance, the secondary antibody may be labelled by a fluorescent dye (including small-molecule dyes, quantum dots, fluorescent proteins, etc.), a metal bead (e.g., gold beads) by an enzyme that generates a detectable signal (e.g., horseradish peroxidase (HRP)).

A still further aspect of the present invention relates to the antibody or antibody fragment of the present invention or an antibody obtained from a method of the present invention for use in a method for treating or preventing an individual suffering from a coronavirus infection or being of risk of developing a coronavirus infection.

It will be understood that all definitions are preferred embodiments as indicated above *mutatis mutandis* also apply to the antibody or antibody fragment for use.

In other words, the present invention also relates to a method for treating or preventing an individual suffering from a coronavirus infection or being of risk of developing a coronavirus infection in a patient, wherein said patient is administered with a sufficient amount of antibody or antibody fragment of the present invention or an antibody obtained from a method of the present invention.

In a preferred embodiment, coronavirus infection is a SARS-CoV-2 infection.

The term "suffering from" as used herein may be understood in the broadest sense in a way that the patient has developed a pathological condition associated with coronaviruses. The patient suffering from a disorder not necessarily but optionally bears medicinal symptoms.

The term "being at risk of" or "being at risk of developing" means that the patient has a certain risk of having a disorder associated with coronaviruses.

The present invention may also include the neutralization and treatment of acute/chronic gastrointestinal infections/inflammations caused by viruses, like subunit S1 and/or subunit S2 of the coronavirus spike protein from SARS-CoV-2. This may be achieved by neutralizing by the following mechanics:
a) the blocking of receptor bindings sites, hindering the entrance of viruses into the cells of the respiratory tract of the human body; and/or
b) by sterically blocking the one or more of the four subdomains of the subunit S1 and/or subunit S2 of the coronavirus spike protein from SARS-CoV-2.

Testing of efficacy of hyperimmune for antibodies for mode of action-studies in animals (mice, rats, hamsters etc.) who are challenged with the viruses.

The antibodies may also be used for further purposes, including an *in vitro* uses and methods.

A further aspect of the present invention relates to the use of an antibody or antibody fragment of the present invention or an antibody obtained from a method of the present invention for detecting subunit S1 and/or subunit S2 of coronavirus spike protein from SARS-CoV-2 in a fluid.

It will be understood that all definitions are preferred embodiments as indicated above *mutatis mutandis* also apply to the use. This use preferably is an *in vitro* use.

A further aspect of the present invention relates to a method for testing the ability of an antibody or antibody fragment for neutralizing the bioactivity of *coronaviridae,* in particular SARS-CoV-2, wherein said method comprises the following steps:
(A) providing:
   an adherent mammalian cells in a cell culture,
   *coronaviridae* virus particles, in particular SARS-CoV-2 virus particles, and the antibody or antibody fragment, in particular an antibody or antibody fragment according to the present invention;
(B) contacting the virus particles and the antibody or antibody fragment with the adherent mammalian cells;
(C) incubating the exposed mammalian cells for a time sufficient for detachment of cells of lower viability; and
(D) detecting the degree of cell rounding,
wherein the degree of cell rounding indicates the degree of remaining bioactivity of the *coronaviridae,* in particular SARS-CoV-2.

It will be understood that all definitions are preferred embodiments as indicated above *mutatis mutandis* also apply to the method. This method preferably is an *in vitro* method.

The invention is further explained by the following examples and claims, which are intended to illustrate the present invention.

### Example I: Manufacturing the Vaccine

The polypeptides of the present invention are obtained by solid phase peptide synthesis, by heterologous expression in host cells, or may be obtained from coronaviruses.

According to the invention, the manufacturing of the vaccine is obtained by binding partially oxidized polysaccharide (predominantly dextran) to the surface of *Bacillus Calmette-Guérin* (BCG) and coating the modified attenuated bacteria (BCG) loaded in this way with one or more polypeptides of the present invention containing defined epitopes or with the proteins of the subunit S1 and/or subunit S2 of the coronavirus spike protein from SARS-CoV-2. The thus modified attenuated bacteria (BCG) are provided with additives, e.g., 3 to 7% (w/v) dextran/lactose solution for stabilization. Suitable polysaccharides are dextran, amylose, amylopectin or glycogen, but also other polysaccharides such as pectin, glucomannan, chitosan or lipopolysaccharides.

BCG - both in a vital and dead state - are preferably considered as mycobacteria. The partially oxidized polysaccharides (preferably dextran) bind covalently through the active aldehyde groups to the bacteria surfaces by reacting with existing amino groups. If the partially oxidized polysaccharides are added in excess to the attenuated or killed bacteria, free aldehyde groups remain to coat the BCG with foreign antigenic epitopes.

The partial oxidation of the polysaccharide can be carried out with a suitable oxidizing agent, such as periodic acid. Special attention is paid to the stoichiometric conditions. In order to maintain the vitality of the attenuated bacteria, a 12% degree of oxidation should generally not be exceeded. After oxidation of the polysaccharides, the oxidizing agent is removed gently.

The bacteria (BCG) are suspended in the partially oxidized polysaccharide solution and carefully shaken overnight at room temperature under sterile conditions. After the binding reactions have finished, the excess of oxidized polysaccharides is removed by washing.

The non-oxidized polysaccharide solution is suitable as washing liquid. When carrying out the reaction, the pH value is adjusted slightly acidic or the binding reaction time is extended.

Antigen binding is checked immunochemically, e.g. by immunofluorescence microscopy, using specific antibodies. All solutions are sterilized before use. A vitality test is carried out for attenuated bacteria. A large number of common epitopes as a mixture can be advantageously concentrated on the attenuated bacterial surfaces (1 mg moist BCG surface corresponds to approximately 50 cm²).

The disguised (divested) bacteria can be ampouled and used as a vaccine without the need for adjuvants. The antigenic epitopes of viral origin can react separately with the partially oxidized polysaccharides in a solution and be used as secondary vaccine together or separately with the primary vaccine (epitope-coated BCG).

### Examples for the production of a combination vaccine against the common epitopes of coronaviruses

50 mL (20 g/L) dextran-35 solution are slowly oxidized with 40 mL of 45 mM NaJO₄ at pH 4.5 under cooling. An oxidation degree of 12% (2×12 aldehyde groups per 100 glucose units) is achieved.

The partially oxidized dextran solution is purified by double precipitation with 60% or 96% ethanol and dried at room temperature under vacuum, protected from light. Prior to use, the partially oxidized dextran is placed in an aqueous solution and then filtered under sterile conditions.

Bacille de Calmette Guerin (BCG) Jena-S-4, which is usable for the non-specific immunization of tumor patients, is coated with 2% (w/v) partially oxidized dextran solution in a weakly acidic environment: In 100 mL partially oxidized dextran solution, 6000 mg of BCG stabilized in 15% (w/v) lactose solution are suspended and lightly shaken overnight at room temperature.

At the end of the binding reaction the suspension is washed three times with an apyrogenic 2% dextran-35 solution. The suspension is taken up in a solution consisting of 100 mL of 2% dextran-35 and 100 mL of 15% lactose and portioned 60 mg/2 ml wise. The living bacterial count is determined and the ampoules are sealed with sterile piercing stoppers. This basic vaccine is coated with antigenic epitopes of various origins by reacting the free aldehyde groups with the free amino groups of the antigen.

100 mg synthetic epitope selected from SEQ ID NOs: 8 to 62 of subunit S1 and/or subunit S2 of coronavirus spike protein from SARS-CoV-2 is taken up in 1000 mL aqueous (slightly acidic or alkaline) buffer and carefully injected into the vial under sterile conditions through the piercing stopper. The ampoules are shaken gently for 60 min at room temperature. After the binding reaction the vaccine is available.

The antigen binding can be confirmed immunochemically.

### Example II: Vaccination

Individuals can be immunized by the administration of the vaccine by 0.1 to 1 mL intramuscularly. After 4 weeks a booster shot should be given at the same administration route. The induction of specific antibodies can be measured by ELISA.

### Example III: Positive affinity chromatography at an epitope

A polypeptide as described above, for instance a polypeptide of one or more of SEQ ID NOs: 8 to 62, is immobilized on an affinity chromatography matrix (affinity beads). This material is filled in an affinity chromatography column. The column is washed with a buffer (PBS). The column is contacted with the body fluid (blood serum or a fraction thereof).

The antibodies and antibody fragments specifically binding to the respective polypeptide comprising the epitope bind to their targets in the affinity column. The column is washed by a flow-through of buffer. Thereby unbound components including unbound antibodies and antibody fragments having no or merely a low affinity to the polypeptide are removed.

Subsequently, the specific antibodies and antibody fragments are eluted either by acetic buffers (e.g. 0.1 mol/L glycine/HCI pH 2.0) or by 3 mol/L KSCN, or by 8 mol/L urea. Thereby, the specifically binding antibodies are isolated.

### Example IV: IgA separation

IgA is isolated directly from plasma or from plasma fractions which are purified from IgG. When those fractions are used, the separation of the specific immunoglobulin classes is not needed. When the positive affinity chromatography is performed ahead of the separation of the immunoglobulin classes, the separation of them is performed in a second step.

An example for the separation of (hyperimmune) IgA includes subjecting a plasma pool to Cohn fractionation. This is followed by an IgG polishing step providing IgG and an IgA/IgM fraction. The IgA/IgM fraction is subjected to affinity chromatography as described above and provides (hyperimmune) IgA.

An alternative example for the separation of (hyperimmune) IgA includes subjecting an unfractioned plasma pool subjected to affinity chromatography as described above. This provides an immunoglobulin fraction (including IgG and IgA) and a residual blood fraction which is further subjected to Cohn fractionation. The immunoglobulin fraction (including IgG and IgA) is subjected to a second affinity chromatography separating IgG and IGA and provides (hyperimmune) IgA.

### Example V: Separation of plasma containing (neutralizing) antibodies against subunit S1 and/or subunit S2 of the coronavirus spike protein from SARS-CoV-2

The donations (plasma and or blood) are screened by commercially available ELISA. Here, the S1 and or S2 of the SARS-CoV 2 is insolubilized at a solid phase (e.g. microtiter plate) (Porstmann et al. Enzyme immunoassay techniques. An overview. Journal of Immunological Methods, 150 (1992) 5-21). The specific antibodies of different immunoglobulin classes are separated by using different class-specific antibodies in a labelled form (e.g. anti-IgG-HRP, anti-IgA-HRP).

Due to the fact, that the immune reaction shows stable antibody responses even month and years after an infection, a quarterly or half-year screening of donors can be performed. The selected donations are pooled and used in the plasma fractionation for the separation of all other plasma proteins. The IgA containing fraction is used separately.

### Example VI: Enzyme-linked immunosorbent assay (ELISA)

A polypeptide as described above, for instance a polypeptide of SEQ ID NO: 8 to 62, is immobilized on a bottom of a microtiter plate. The body fluid (blood fraction, blood plasma/serum) is contacted for several minutes. Then, the microtiter plate is washed with buffer (PBS) and contacted with an enzyme-labelled secondary antibody (e.g., conjugated with horseradish peroxidase (HRP)). A substrate suitable to be converted into a detectable moiety by the enzyme is added and the staining of the microtiter plate is performed in a plate reader. This assay provides an antibody titer of the body fluid.

### Example VII: Cell-based neutralization assay

The ability of antibodies to neutralize the viruses is tested via the exposal of mammalian cells to one of the viruses.

The CHO cells are grown in DMEM/Ham's F12 and HT29 in McCoys 5A in in cell culture dishes and supplemented 20% fetal calf serum and glutamine. The virus solutions and the mono- or polyclonal antibodies or antiserum are incubated for 60 min at 37 °C. On the first day, the cells are seeded in a 96 well cell culture plates and incubated overnight.

Dilutions of antibodies and LCTs are done in the cell culture medium. The virus concentrations for the assay are chosen in a way that just enough virus is administered to induce complete rounding overnight. On the second day, the diluted antibodies are mixed with subsequent dilutions of the LCTs. After incubation (60 min at 37 °C) of the antibody-virus mixes are added to the cells and cell rounding is observed after 20-24 hours. As control, virus is added to the cells without antibodies. The cell lysis is evaluated by microscopic analysis on the third day.

## Claims

1. A polypeptide or an immunogenic peptidomimetic or retro-inverso polypeptide thereof, wherein said polypeptide comprises at least one epitope of severe acute respiratory syndrome coronavirus 2 (SARS-CoV-2), **characterized in that**:
said epitope has a length of 8 to 100 consecutive amino acids;
said epitope has a sequence homology of at least 75% to a sequence section of subunit S1 and/or subunit S2 of coronavirus spike protein from SARS-CoV-2; and
said epitope has a sequence homology of at least 75% to a sequence section of subunit S1 and/or subunit S2 of a coronavirus spike protein from at least one other type of coronavirus.

2. The polypeptide of claim 1, wherein the at least one other type of coronavirus is selected from the group consisting of severe acute respiratory syndrome coronavirus 1 (SARS-CoV-1), Middle East respiratory syndrome coronavirus (MERS-CoV), human coronavirus NL63 (HCoV-NL63), human coronavirus OC43 (HCoV-OC43), human coronavirus HLU1 (HCoV-HKU1), human coronavirus 229E (HCoV-229E), and combinations of two or more thereof.

3. The polypeptide of any one of claims 1 or 2, wherein said polypeptide is obtained by a method comprising one or more procedural steps selected from the group consisting of polypeptide synthesis, gene technological means, and isolation of subunit S1 and/or subunit S2 from SARS-CoV-2 and subsequent protein digestion.

4. The polypeptide of any one of claims 1 to 3, wherein the epitope is **characterized in that**:
it has a sequence homology of at least 75% to a sequence section of subunit S1 and/or subunit S2 of coronavirus spike protein from SARS-CoV-2 located at the outer surface of the SARS-CoV-2 virus particle, and
it has a sequence homology of at least 75% to a sequence section of subunit S1 and/or subunit S2 of at least one coronavirus spike protein from at least one other type of coronavirus located at the outer surface of the virus particle of said at least one other type of coronavirus.

5. The polypeptide of any one of claims 1 to 4, wherein the epitope comprises or consists of an amino acid sequence that has a sequence homology of at least 75% to at least one of the sequences selected from the group consisting of sequences of SEQ ID NOs: 8 to 62 or in an immunogenic peptidomimetic or retro-inverso polypeptide thereof,
in particular wherein the epitope comprises or consists of an amino acid sequence selected from the group consisting of sequences of SEQ ID NOs: 8 to 62 or in an immunogenic peptidomimetic or retro-inverso polypeptide thereof.

6. The polypeptide of any one of claims 1 to 5, wherein said polypeptide is immobilized on a solid support.

7. A vaccine comprising at least one polypeptide of any one of claims 1 to 6 and at least one pharmaceutically acceptable carrier.

8. The vaccine of claim 7 for use in a method for preventing an individual from developing a coronavirus infection.

9. An antibody or antibody fragment binding to subunit S1 of coronavirus spike protein from SARS-CoV-2 with a dissociation constant Kd of less than 100 nM and/or to subunit S2 of coronavirus spike protein from SARS-CoV-2 with a dissociation constant Kd of less than 100 nM,
in particular wherein said antibody or antibody fragment binds to an epitope as defined as in any of claims 1 to 6 with a dissociation constant Kd of less than 100 nM.

10. A method for isolating and/or detecting an antibody or antibody fragment binding to subunit S1 and/or subunit S2 of coronavirus spike protein from SARS-CoV-2, in particular an antibody or antibody fragment of claim 9, from a fluid containing the antibody or antibody fragment, wherein said method comprises the following steps:
(i) providing:
the fluid containing the antibody or antibody fragment, and
a polypeptide immobilized on a solid support according to claim 6;
(ii) contacting the fluid with the immobilized polypeptide and allowing the antibody or antibody fragment to bind to the immobilized polypeptide; and
(iii) removing at least parts of the unbound fluid and optionally washing the solid support with a fluid not containing the containing the antibody or antibody fragment,
preferably wherein the solid support is a solid phase of an affinity column and said method preferably further comprises a step of eluting the antibody or antibody fragment from the affinity column.

11. The method of claim 10, wherein the fluid is a body fluid, preferably a body fluid selected from the group consisting of blood plasma and a fraction of blood plasma, in particular wherein said method further comprises preparing of a fraction of blood plasma by means of a Cohn or Kistler-Nitschmann process.

12. The method of any one of claims 10 or 11, wherein said method further comprises:
isolating or removing one or more antibody classes selected from the group consisting of IgG, IgM, IgD, IgE and IgA; and/or
detecting the bound antibody or antibody fragment, preferably wherein said step of detecting the bound antibody or antibody fragment comprises the following steps:
(a) binding a secondary antibody selectively to the Fc part of the bound antibody or antibody fragment; and
(b) detecting said secondary antibody, preferably wherein said secondary antibody is labeled with a detectable label or is conjugated to an enzyme that generates a detectable compound from a precursor.

13. The antibody or antibody fragment of claim 9 or an antibody obtained from a method of any one of claims 10 to 12 for use in a method for treating or preventing an individual suffering from a coronavirus infection or being of risk of developing a coronavirus infection.

14. Use of an antibody or antibody fragment of claim 9 or an antibody obtained from a method of any one of claims 10 to 12 for detecting subunit S1 and/or subunit S2 of coronavirus spike protein from SARS-CoV-2 in a fluid.

15. A method for testing the ability of an antibody or antibody fragment for neutralizing the bioactivity of *coronaviridae,* in particular SARS-CoV-2, wherein said method comprises the following steps:
(A) providing:
an adherent mammalian cells in a cell culture,
*coronaviridae* virus particles, in particular SARS-CoV-2 virus particles, and
the antibody or antibody fragment, in particular an antibody or antibody fragment according to claim 9;
(B) contacting the virus particles and the antibody or antibody fragment with the adherent mammalian cells;
(C) incubating the exposed mammalian cells for a time sufficient for detachment of cells of lower viability; and
(D) detecting the degree of cell rounding,
wherein the degree of cell rounding indicates the degree of remaining bioactivity of the *coronaviridae,* in particular SARS-CoV-2.
